Europäisches Patentamt

European Patent Office

Office européen des brevets

(19)

(11) Publication number: **0 311 469**
**A2**

# EUROPEAN PATENT APPLICATION

(12)

(21) Application number: **88402204.7**

(22) Date of filing: **01.09.88**

(51) Int. Cl.⁴: **C 12 N 15/00**
**A 23 K 3/02, A 23 L 1/03,**
**A 61 K 35/74, C 12 Q 1/40,**
**C 12 N 9/28, C 12 N 1/20**
**//(C12N1/20,C12R1:01,1:46,**
**1:225)**

(30) Priority: **02.09.87 EP 87401972**

(43) Date of publication of application:
**12.04.89 Bulletin 89/15**

(84) Designated Contracting States:
**AT BE CH DE ES FR GB GR IT LI LU NL SE**

(71) Applicant: **PLANT GENETIC SYSTEMS N.V.**
**Kunstlaan Avenue des Arts, 46**
**B-1040 Bruxelles (BE)**

**UNIVERSITE CATHOLIQUE DE LOUVAIN**
**Place de l'Université, 1**
**B-1348 Ottignies (Louvain la Neuve) (BE)**

(72) Inventor: **Michiels, Frank**
**Sint Jozefstraat 7**
**B-9220 Merelbeke (BE)**

**Delcour, Jean**
**Rue Chapelle St. Anne 1**
**B-5865 Walhain (BE)**

**Mahillon, Jacques**
**Lousbergkaai 27**
**B-9000 Gent (BE)**

**Joos, Henz**
**Oostmolen Zuid 5**
**B-9880 Aalter (BE)**

**Platteeuw, Christ**
**Tiendenbergstraat 3**
**B-8699 Staden (BE)**

**Josson, Kathy**
**Meersstraat 124**
**B-9000 Gent (BE)**

(74) Representative: **Gutmann, Ernest et al**
**S.C. Ernest Gutmann - Yves Plasseraud 67, boulevard Haussmann**
**F-75008 Paris (FR)**

(54) **Transformed lactic acid bacteria.**

(57) An inoculum for silage and a probiotic which include lactic acid bacteria transformed with at least one exogenous gene or DNA fragment thereof coding for an enzyme which breaks down an oligosaccharide and/or a polysaccharide into a monosaccharide, disaccharide or other fermentable carbohydrate. Also provided are methods for transforming the lactic acid bacteria by electroporation and by the use of new plasmids, vectors and other DNA sequences. A new amylase is also provided.

EP 0 311 469 A2

## Description

# TRANSFORMED LACTIC ACID BACTERIA

Background of the Invention

This invention relates to bacterial microorganisms, particularly Streptococcus, Lactobacillus, Enterococcus, Lactococcus, Pediococcus and Leuconostoc species, which carry out lactic acid fermentation. This invention particularly relates to the use of such lactic acid bacteria as inocula in silage and as probiotics. This invention more particularly relates to the use in silage and as probiotics of lactic acid bacteria which have been transformed with foreign genes or DNA fragments which code for enzymes that degrade oligosaccharides and/or polysaccharides into monosaccharides, and disaccharides and other carbohydrates fermentable into lactic acid.

Lactic acid bacteria are a non-taxonomic group of Gram-positive non-sporeforming bacteria which ferment simple water soluble carbohydrates ("WSC") to produce lactic acid as the predominant end product. Such bacteria are widely used in the feed, food and dairy industries. Traditionally, they have mainly included species from the genera Lactobacillus, Streptococcus, Leuconostoc and Pediococcus. The taxonomy of these microorganisms has been described in "Bergey's Manual of Systematic Bacteriology" (Sneath et al., 1986). However, the taxonomy of Streptococcus has recently been revised extensively (Schleifer and Kilpper-Bälz, 1987). On the basis of many biochemical and serological studies, the genus Streptococcus has been divided into three new genera: 1) Streptococcus sensu stricto that contains the majority of known streptococcus species, many of which can be pathogenic; 2) Enterococcus that contains the enteric streptococci; and 3) Lactococcus that contains all lactic streptococci. Of these three genera, it is the Lactococcus and Enterococcus which are extensively used in the feed, food and dairy industries.

## Silage

Silage is a process of controlled anaerobic fermentation of a crop (e.g., grass, cereals or legumes) in order to preserve it for use as a feed for animals (McDonald, 1981). Proper ensilage depends upon the presence of suitable lactic acid bacteria in order to produce enough lactic acid to cause a rapid and significant pH decline in the fermentation medium. The pH decline stops the growth of undesirable microorganisms such as Clostridia (Woolford & Sawczyk, 1984) in the silage and tends to inhibit post-harvest changes in the silage, particularly in its protein fraction (Woolford, 1984).

The lactic acid bacteria commonly used in ensilage can be divided into two major categories: 1) the homofermentative lactic acid bacteria which ferment predominantly hexoses to lactic acid; and 2) the heterofermentative lactic acid bacteria which ferment hexoses to lactic acid and other products, such as ethanol and acetic acid (Beck, 1978). Both categories of bacteria can be further subdivided into rod and coccus categories (Beck, 1978). The most important homofermentative rod bacteria are: Lactobacillus casei, Lactobacillus coryniformis, subsp. coryniformis, Lactobacillus curvatus, Lactobacillus salvarius, Lactobacillus acidophilus and Lactobacillus plantarum. The most important homofermentative coccus bacteria are: Pediococcus acidilactici, Pediococcus cerevisae, Pediococcus pentosaceus, Enterococcus faecalis, Lactococcus lactis and Enterococcus faecium. The most important heterofermentative rod bacteria are: Lactobacillus brevis, Lactobacillus buchneri, Lactobacillus fermentum and Lactobacillus viridescens. The most important heterofermentative coccus bacteria are: Leuconostoc cremoris, Leuconostoc citrovorum, Leuconostoc dextranicum and Leuconostoc mesenteroides.

Lactic acid bacteria in a silo normally come from the ensiled vegetation itself or from natural inoculation by farm equipment (McDonald, 1981). To ensure a rapid and reproducible pH decline in silage, cultured lactic acid bacteria, such as one or more of the aforementioned Lactobacillus, Enterococcus, Lactococcus, Pediococcus and/or Leuconostoc species, are often added to a crop prior to ensilage (Seale, 1986).

The majority of lactic acid bacteria can ferment only simple water soluble carbohydrates, such as the monosaccharides, disaccharides and trisaccharides (e.g., glucose, arabinose, maltose, cellobiose, fructose, galactose, lactose, mannose, melizitose, melibiose, raffinose, rhamnose, ribose, sucrose, trehalose and xylose) and related compounds such as mannitol, sorbitol, galacturonate and gluconate, which are part of the WSC of the crop.

However, the WSC content of a crop is variable and depends upon the species of crop, its stage of growth, and the weather and fertilizer to which it has been exposed (Woolford, 1984). In order to increase the WSC content and composition of silage, carbohydrates fermentable to lactic acid by lactic acid bacteria (the "fermentable carbohydrates") are often added to a crop (McDonald, 1981), either in the form of: WSC-rich vegetation such as maize, barley, oat, wheat or milo; other WSC-rich material such as molasses or whey; or directly in the form of more or less pure fermentable monosaccharides, disaccharides and trisaccharides as mentioned above. These fermentable carbohydrates can be added to the crop either alone or in combination.

Besides adding fermentable carbohydrates to a crop to increase its WSC content, silage has been improved by adding enzymes to the crop to break down polysaccharides and oligosaccharides, whether naturally present in the crop or added to the crop prior to ensilage, which could not otherwise be fermented by the lactic acid bacteria. In this regard, exogenous enzymes such as amylases, pectinases, cellulases and/or hemicellulases, which can degrade polysaccharides (e.g., starch and cellulose) and/or oligosaccharides into monosaccharides, disaccharides, trisaccharides and/or other fermentable carbohydrates, have been added to crops prior to ensilage. Sometimes, non-fermentable carbohydrates, such as starch, have also been added to a crop, together with a suitable enzyme, such as an amylase, to break down the starch.

The addition of lactic acid bacteria inocula, enzymes and/or sources of fermentable carbohydrates, as described above, has been found to provide better control of the ensiling process, with rapid fermentation to produce lactic acid, with minimal losses of silage during storage and with maximal feed quality of the stored product (Bolson, 1985).

The addition of so-called "cellulases", together with lactic acid bacteria, has been particularly useful (Seale, 1987). Such cellulase additives have been important in breaking down rigid plant cell walls, which consist of complexes of cellulose, hemicellulose, pectin and lignin, and derivatives thereof, and liberating from the cell walls fermentable carbohydrates that would otherwise not be part of the WSC content of the crop. In addition, such cellulase additives are likely to have locally degraded, and thereby perforated, the cell walls so that intracellular WSC of the plant cells has become available for the lactic acid bacteria.

However, these cellulase additives have contained more than just cellulases. Cellulases have classically been divided into three major classes: 1) the endoglucanases which break the beta-1,4-linkage in the amorphous region of cellulose; 2) the exoglucanases or cellobiohydrolases which liberate cellobiose to glucose units from the ends of the glucose-chains; and 3) the beta-glucosidases which degrade cellobiose to glucose. Genes coding for these different cellulases are known (Knowles et al, 1987). It is now believed that many of these cellulase additives have contained, besides cellulases, proteins with no known enzymatic activity (i.e., non-enzymatic proteins) which have promoted the action of the cellulases in the additives in degrading cellulose in the silage. In this regard, it has long been known that although cellulose is a chemically simple compound, it is a physically complex beta-1,4-linked glucose polysaccharide which consists of crystalline cellulose regions linked by amorphous cellulose regions (Coughlan, 1985). For this reason, natural organisms which degrade cellulose have generally been found to, possess a multiprotein system, including different enzymatic and non-enzymatic proteins that act synergistically and, at least in some bacteria, are physically aggregated in so-called cellulosomes. Recent studies also have demonstrated that, at least in some cases, only two cellulases are required to obtain a substantial breakdown of cellulose (Wong et al 1988) and the use of only one cellulase, plus a non-enzymatic protein which promotes the degradation of cellulose by the cellulase, has been found to break down substantially crystalline cellulose (Wu et al, 1988). Thus as used herein, the term "enzyme that degrades a polysaccharide and/or an oligosaccharide" means an enzyme in the classic sense (e.g., a cellulase) which degrades a polysaccharide and/or an oligosaccharide, as well as the non-enzymatic proteins which promote the degradation of polysaccharides and/or the oligosaccharide by the enzyme.

## Probiotics

Lactic acid bacteria, particularly Enterococcus and Lactobacillus, also serve a key role in establishing and maintaining optimal intestinal flora in animals. The intestinal tracts of animals constitute a very specific ecological system colonized by a mixed population of microorganisms whose balance plays an essential role in the animals' health and growth (Raibaud and Ducluzeau, 1984). The spectrum of species and genera of microorganisms in such systems varies greatly from one animal species to another. Potentially harmful germs, such as Escherichia and Salmonella, cohabit with beneficial bacteria such as Enterococcus and Lactobacillus (Vrignaud, 1982). Their equilibrium is controlled by both external factors (mainly food quality and hygiene) and internal parameters such as stress. Severe disruption of this equilibrium can lead to diarrhea which is a significant cause of death among young farm animals such as piglets and calves (Vrignaud, 1982).

Probiotics are food additives which help to establish and maintain an optimal intestinal flora in man and other animals. Lactic acid bacteria are considered to be preferred bacterial probiotics (Fernandes et al, 1987). The beneficial effects of lactic acid bacteria is primarily due to their positive role in natural intestinal flora. By producing lactic acid, such bacteria lower the pH in the intestine, thereby setting up unfavorable conditions for the proliferation of most gram-negative pathogens. In addition, many species of lactic acid bacteria secrete compounds, known as bacteriocins (e.g., nisin produced by many strains of L. lactis), which exhibit strong antibiotic properties. Lactic acid bacteria also produce vitamins (mainly groups B and K) and lower redox potential (Klaenhammer, 1982; Aumaitre, 1988). The use of lactic acid-bacteria probiotics as human food and animal feed supplements has become popular in recent years (Wolter and Henry, 1982; Aumaitre 1988).

It has been demonstrated that the addition of 3-4% of dried lactic acid bacteria to the powdered aliments, fed to piglets and calves, reduces their incidence of diarrhea significantly, lowers their consumption index, and increases their growth (Vrignaud, 1982). It has also been demonstrated that the growth performance of farm animals, such as piglets, calves and chickens, can be significantly enhanced by supplementing their food with enzyme preparations (Lyons, 1987; Chesson, 1987). Enzymes, such as α-amylase, β-glucanase, xylanase and pectinase, have been found to be most useful in this respect (Marquardt et al, 1987). The addition of amylases to the feed of young calves has also been found to facilitate the transition from weaning to solid food (Gilliland, 1988). Not only do such enzymes increase the nutritional value of the diet by improving digestibility (Broz, 1987), but they also decrease the antinutritional value of some feedstuffs (such as rye) by degrading viscous polysaccharide components (e.g., pentosanes) known to interfere with the proper functioning of the digestive tract in chickens (Fengler and Marquardt, 1986).

Summary of the Invention

In accordance with this invention, an inoculum for silage is provided, which comprises lactic acid bacteria transformed with at least one exogenous gene or DNA fragment of the gene (the "exogenous DNA") coding for an enzyme which breaks down an oligosaccharide and/or a polysaccharide into a fermentable carbohydrate. By the use of this inocula, an ensilage method is provided in which it is not necessary to add exogenous enzymes to the crop and it may not even be necessary to add fermentable carbohydrates to the silage while carrying out the ensilage process in an otherwise conventional manner. In this regard, the transformed lactic acid bacteria of this invention can breakdown polysaccharides and oligosaccharides in the crop itself to provide a complete source of the fermentable carbohydrates that the lactic acid bacteria needs for producing lactic acid.

Also in accordance with this invention, a probiotic is provided which comprises lactic acid bacteria transformed with at least one of the exogenous DNAs; provided that when the transformed lactic acid bacteria is a Streptococcus, it is transformed with at least two of the exogenous DNAs which code for different enzymes that can degrade a polysaccharide and/or an oligosaccharide into a fermentable carbohydrate. This probiotic can be used in an otherwise conventional manner in a method for establishing and maintaining optimal intestinal flora in animals, including humans.

Further in accordance with this invention are provided lactic acid bacteria transformed with at least one, preferably at least two, of the exogenous DNAs; provided that when the transformed lactic acid bacteria are Streptococcus, they are transformed with at least two of the exogenous DNAs which code for different enzymes that can degrade a polysaccharide and/or oligosaccharide into a fermentable carbohydrate.

Still further in accordance with this invention, an electroporation method is provided for transforming lactic acid bacteria, including the use of new plasmids, vectors and DNA sequences.

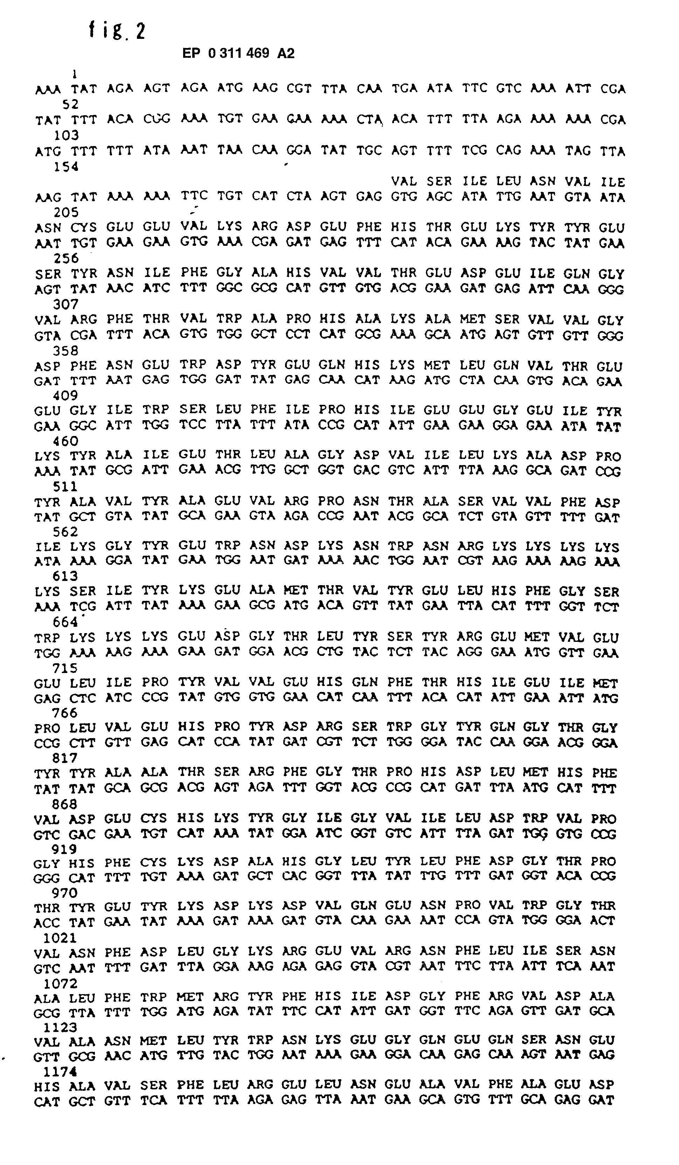

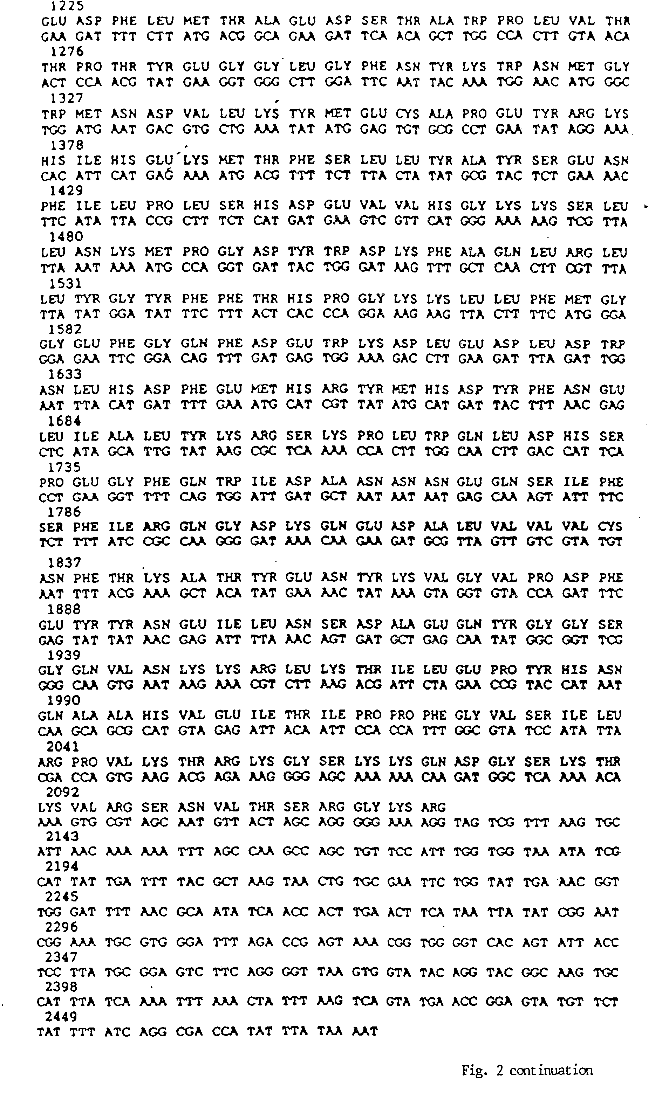

Yet further in accordance with this invention, a new amylase is provided, having an amino acid sequence as shown in Fig. 2. In addition, a DNA sequence, encoding the new amylase, is provided as also shown in Fig. 2, particularly from nucleotide 184 to nucleotide 2131.

Description of the Invention

In transforming lactic acid bacteria for use as an inoculum for silage or as a probiotic, particularly for silage, any lactic acid bacteria can be used in accordance with this invention. However, when the lactic acid bacteria is a Streptococcus that is to be used as a probiotic, the Streptococcus is transformed with at least two of the exogenous DNAs, and when the lactic acid bacteria is a Lactobacillus transformed with an exogenous DNA coding for an amylase, the Lactobacillus is preferably transformed also with at least one other exogenous DNA coding for a different enzyme, preferably a cellulase. The preferred species of lactic acid bacteria for silage are from the genera Lactobacillus, Enterococcus, Lactococcus, Pediococcus and Leuconostoc, all of which have been used previously in silage. The particularly preferred species for silage are from the genera Enterococcus, Lactobacillus and Pediococcus, such as the aforementioned species of such genera. The preferred species for probiotics are from the genera Enterococcus and Lactobacillus.

The lactic acid bacteria can be transformed with any foreign gene or DNA fragment thereof which codes for the expression and secretion of an enzyme that can degrade a polysaccharide and/or an oligosaccharide to a fermentable carbohydrate. In this regard, the foreign gene or DNA fragment can code for: an enzyme that can degrade starch, i.e., an amylase such as an $\alpha$-amylase, $\beta$-amylase, glucoamylase, isoamylase or pullulanase; an enzyme that can degrade cellulose, i.e., a cellulase such as an endoglucanase, exoglucanase or beta-glucosidase; an enzyme that can degrade pectin, i.e., a pectinase such as a pectic lyase, pectic glycosidase or a pectic methyl esterase; or an enzyme that can degrade hemicellulose, i.e., a hemicellulase such as a xylanase, mannanase, arabinanase, inulinase, levanase or fructanase.

For many uses as an inoculum for silage or as a probiotic, the lactic acid bacteria is preferably transformed with 2 or more (e.g., up to 5) of these exogenous DNAs coding for different enzymes. Each of the different enzymes, encoded by the plurality of exogenous DNAs, can degrade a polysaccharide or oligosaccharide into a fermentable carbohydrate. Alternatively, one or more of the different enzymes can degrade a polysaccharide or oligosaccharide into a simpler saccharide, and one or more of the other different enzymes can degrade the simpler saccharide into a fermentable carbohydrate, so long as the overall action of the different enzymes is to degrade a polysaccharide or oligosaccharide into a fermentable carbohydrate. In any event, a plurality of DNAs will preferably be selected to provide the transformed lactic acid bacteria with a suitable combination of enzymes for degrading the intended substrate (e.g., the crop or the animal feed) to obtain an increase in fermentable carbohydrates. Lactic acid bacteria for silage are preferably transformed with exogenous DNAs coding for at least one cellulase, especially two or more cellulases (e.g., an endoglucanase and an exoglucanase). Lactic acid bacteria for a probiotic are preferably transformed with at least one exogenous DNA coding for an amylase.

For any lactic acid bacteria, as well as any other microorganism, transformed with two or more exogenous DNAs, one of which codes for an enzyme, particularly an amylase, in accordance with this invention, the enzyme encoded by the exogenous DNA can serve as a marker for transformed microorganisms. Thereby, a method for detecting transformed microorganisms of this invention is provided, which involves detecting the enzyme produced by the transformed microorganisms.

Suitable exogenous DNAs of this invention, coding for enzymes that degrade cellulose, can be obtained from prokaryotic or eukaryotic microorganisms such as:

the eukaryotic fungi: Trichoderma, Aspergillus, Agaricus, Botryodiplodia, Chaetomium, Eupenicillium, Fusarium, Humicola, Macrophomina, Myrothecium, Myceliophthora, Pellicularia, Penicillium, Pestalotiopsis, Poluporus, Poria, Sporotrichum, Talaromyces, Thermoascus, Thielavia, Tranetes, Trichosporum, Mucor, Torula, Neurospora, Paecilomyces, Lipomyces, Schwanniomyces, Torulopsis, Endomycopsis, Rhizoctonia, Rhizopus, Coniothyrium, Corticium, Verticillum and Monilia;

the prokaryotic bacteria: Acetivibrio, Bacteroides, Bacillus, Cellulomonas, Cellvibrio, Clostridium, Microbispora, Plectridium, Erwinia, Pseudomonas, Selenomonas, Butyrivibrio, Eubacterium, Aerobacter, Micrococcus, Acinetobacter, Cytophaga, Escherichia, Myxobacter, Xanthomonas, Alternaria and Ruminococcus;

the prokaryotic actinomycetes: Streptomyces, Thermoactinomycete Thermomonospora and Caldocellum; and

the eukaryotic yeasts: Kluyveromyces, Candida, Saccharomyces and Schizosaccharomyces.

Suitable exogenous DNAs, coding for enzymes that degrade starch, can be obtained from a large variety of prokaryotes such as Bacillus, Streptomyces, Clostridium Klebsiella, Streptococcus and lactic acid bacteria such as Lactobacillus amylophilus, Lactobacillus amylovorus, Lactobacillus cellobiosus and Enterococcus, from fungi such as Candida japonica, Endomycopsis, Lipomyces, Schwanniomyces, Aspergillus niger and Rhizopus and from most higher plants and animals.

Suitable exogenous DNAs, coding for enzymes that degrade pectins, can be obtained from prokaryots such as Pseudomonas marginalis, Bacillus, Clostridium, Erwinia, Cytophaga, Xanthomonas, Aeromonas, Flavobacterium and Streptomyces, from various plant pathogenic fungi and saprophytic fungi, and from most higher plants.

Suitable exogenous DNAs, coding for enzymes that degrade hemicellulose, can be obtained from bacteria such as Streptomyces, Bacillus, Sporocytophaga myxococcoides, Clostridium, Butyrivibrio fibrisolvens, Ruminococcus albus and R. flavefaciens, from protozoa such as Epidinium ecaudatum, Entodinium and Eremoplastron bovis, and from a wide variety of plant pathogenic and saprophytic fungi.

Needless to say, other microorganisms can provide suitable exogenous DNAs for transforming lactic acid bacteria in accordance with this invention. Furthermore, one can transform lactic acid bacteria: with an exogenous DNA produced from a messenger RNA corresponding to a naturally produced gene or DNA fragment thereof; or when the sequence of a desired exogenous DNA has been established, with a synthetically produced exogenous DNA. Of course, exogenous DNA obtained by a combination of such methods can also be used. Thus, terms such as "gene", "foreign DNA fragment thereof" and "exogenous DNA", as used herein, are not limited to naturally occurring genes and DNA fragments thereof which encode natural enzymes that degrade polysaccharides and/or oligosaccharides. These terms also cover other nucleic acids, such as cDNAs, chimeric genes and synthetic nucleic acids, encoding the same or similar enzymes.

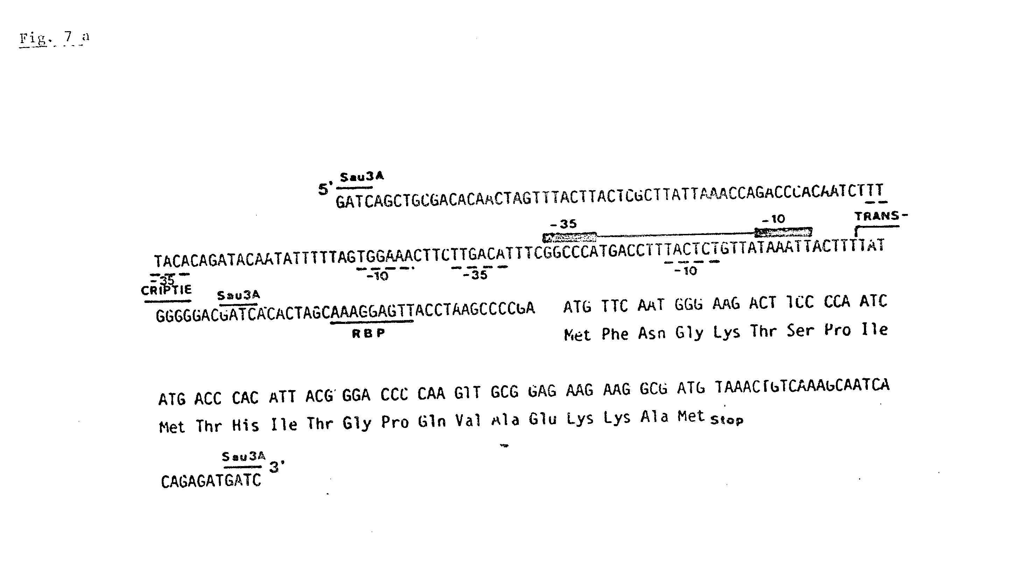

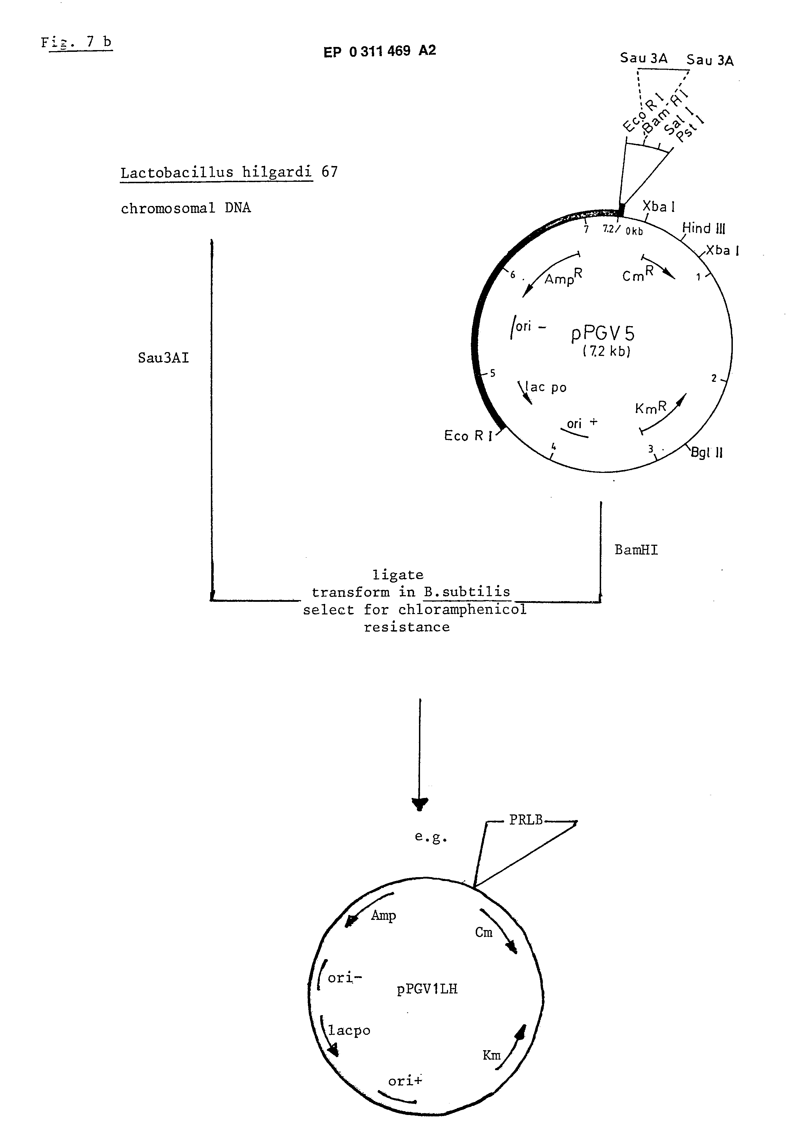

Further, it goes without saying that each exogenous DNA used for the transformation of lactic acid bacteria must cause the bacteria to express and secrete the enzyme encoded by the exogenous DNA. This means that the exogeneous DNA has to contain regulatory sequences such as a suitable promoter, secretion signal, translation initiation signal and termination signal operable in the lactic acid bacteria. These regulatory sequences may be homologous to the enzyme encoding sequences, i.e., naturally associated with such a coding sequence. However, this invention also extends to so called chimeric exogenous DNAs, i.e., DNAs in which one or more regulatory sequences are not normally associated with the enzyme encoding sequence. In this regard, the "PRLB" promoter of Fig. 7a is a preferred heterologous promoter for controlling an exogenous DNA of this invention in lactic acid bacteria.

For transforming lactic acid bacteria, each exogenous DNA can be provided in an episomal vector, e.g., a plasmid. Well known process techniques can then be used to carry out such a transformation with the vector to achieve high levels of expression of the exogeneous DNA of this invention. Cultivating cultures of such transformed lactic acid bacteria also can be carried out in a conventional manner. Of course, segregation of vectors during cell division occurs at random, and cells will appear which do not carry a vector with one or more of the exogenous DNAs of this invention cloned on them. As a result, cells without such a vector could eventually predominate in cultures of the bacteria in which there is no selective pressure for carrying cells with the vector. Nevertheless, a population of bacteria in which some bacteria carry the vector will be at an advantage under silage conditions. Indeed, since the growth of bacteria is limited by the supply of WSC in silage, bacteria carrying the vector, enabling them to degrade plant oligosaccharides and/or polysaccharides, will be assured of an increased supply of WSC. Thus, vectors carrying exogenous DNAs for degrading plant polysaccharides and/or oligosaccharides may be maintained in a silage environment, even though outside the silage environment, such vectors would probably be lost at a high rate.

However, it is preferred that, after transformation of the lactic acid bacteria, each exogenous DNA of this invention be inserted in the bacterial chromosome. This provides more stable transformants and can be suitably accomplished as follows Each exogenous DNA can be first inserted in a bacterial transposon. The transposon also carries a selectable or screenable marker gene and is cloned into a plasmid which cannot replicate in the lactic acid bacteria or whose replication can be inhibited in the bacteria. The plasmid is then introduced in the lactic acid bacteria, and the transformants are screened for the presence of the marker gene so that only bacteria, in which the transposon has moved to the chromosome, will be selected. Alternatively, a plasmid can be constructed which itself contains a marker gene and a part of the bacterial chromosome and which also cannot replicate in the lactic acid bacteria. After introduction of the exogenous DNA in the plasmid, transformation of the lactic acid bacteria and screening for the marker gene, only those bacteria are selected in which the plasmid has been inserted in the bacterial chromosome through a single recombination event between the homologous DNA regions of the plasmid and the bacterial chromosome. If the exogenous DNA and the marker gene are introduced within the bacterial chromosomal region of the vector, the plasmid can also be contructed so that, after transformation of the lactic acid bacteria, the exogenous DNA is inserted in the bacterial chromosome through a double homologous recombination event. Of course, the choice of a suitable region of the bacterial chromosome, which is to be cloned in the plasmid, must be such that the later integration of the exogenous DNA in the chromosome does not hinder the bacteria in their growth or other essential activities.

The transformed lactic acid bacteria of this invention can be used in silage in a conventional manner and in conventional amounts. For example, about $10^5$ to $10^6$ viable bacteria per gram of silage will generally be a minimum, and about $10^7$ bacteria per gram of silage will generally be a maximum. Preferably, the expression of the foreign genes incorporated in the lactic acid bacteria will: 1) increase the amount of fermentable carbohydrates to about 10 to 35 grams per kilogram of silage, preferably to an amount of 15 to 25 grams per kilogram of silage; and 2) lower the pH in the silage to a value of about 3 to 5, preferably to a value of 3.5 to 4.5, within a period of about 24 to 120 hours, preferably 24 to 72 hours.

For probiotics of this invention, the lactic acid bacteria is preferably transformed with an exogenous DNA fragment that encodes an amylase. This is because starch is included in most formulations of food for humans and industrial feed for farm animals and constitutes the major part of some natural aliments (e.g., grain for chicken). Thus, an amylase, produced by a lactic acid bacteria probiotic in the intestinal tract of the human or animal, will hydrolyse residual starch remaining undigested after the action of amylases from the human's or animal's saliva, thereby increasing the energy value of most food and feed formulations and/or allowing the use of less expensive formulations.

Also in probiotics, the lactic acid bacteria preferably is derived from a bacteria found in the natural intestinal flora of animals, such as Enterococcus and Lactobacillus species (Vrignaud, 1982). Such bacteria can be transformed in a conventional manner with an exogenous DNA which directs the expression and secretion of an enzyme, such as one of the aforementioned enzymes for degrading oligosaccharides and/or polysaccharides into fermentable carbohydrates.

The transformed lactic acid bacteria of this invention can be used as a probiotic in a conventional manner and in conventional amounts. For example, about $10^6$ cells per liter (or kg) of food stuff will generally be a minimum, and about $10^{10}$ to $10^{12}$ cells per liter of food stuff will generally be a maximum.

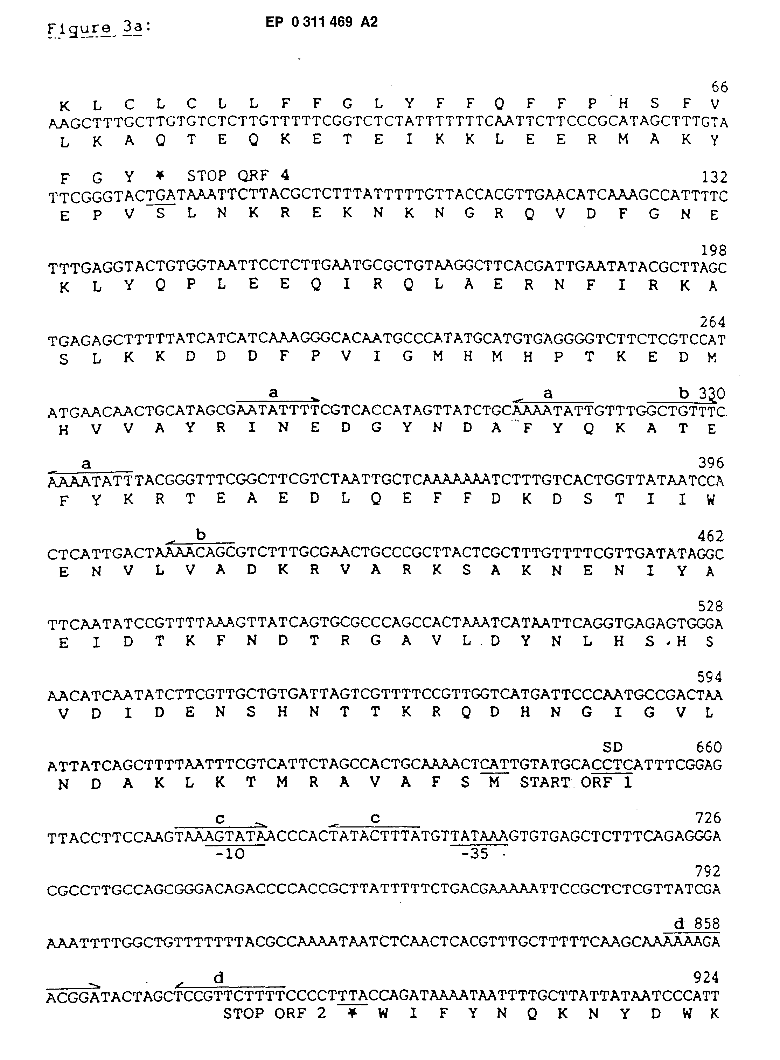

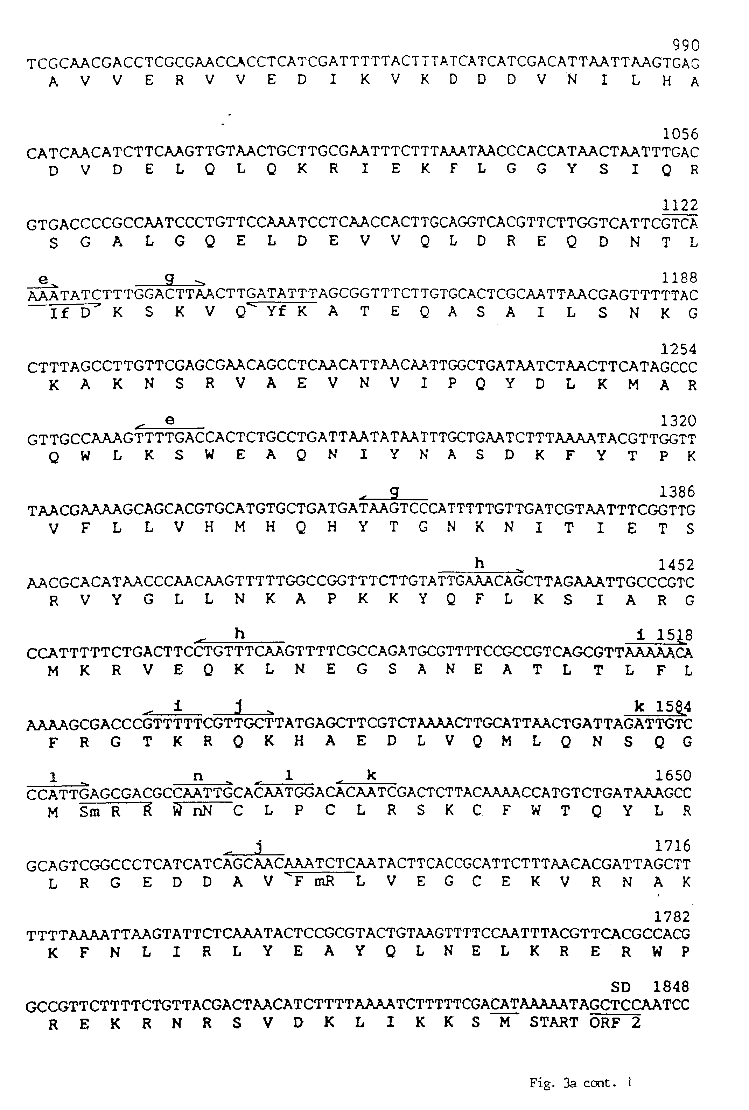

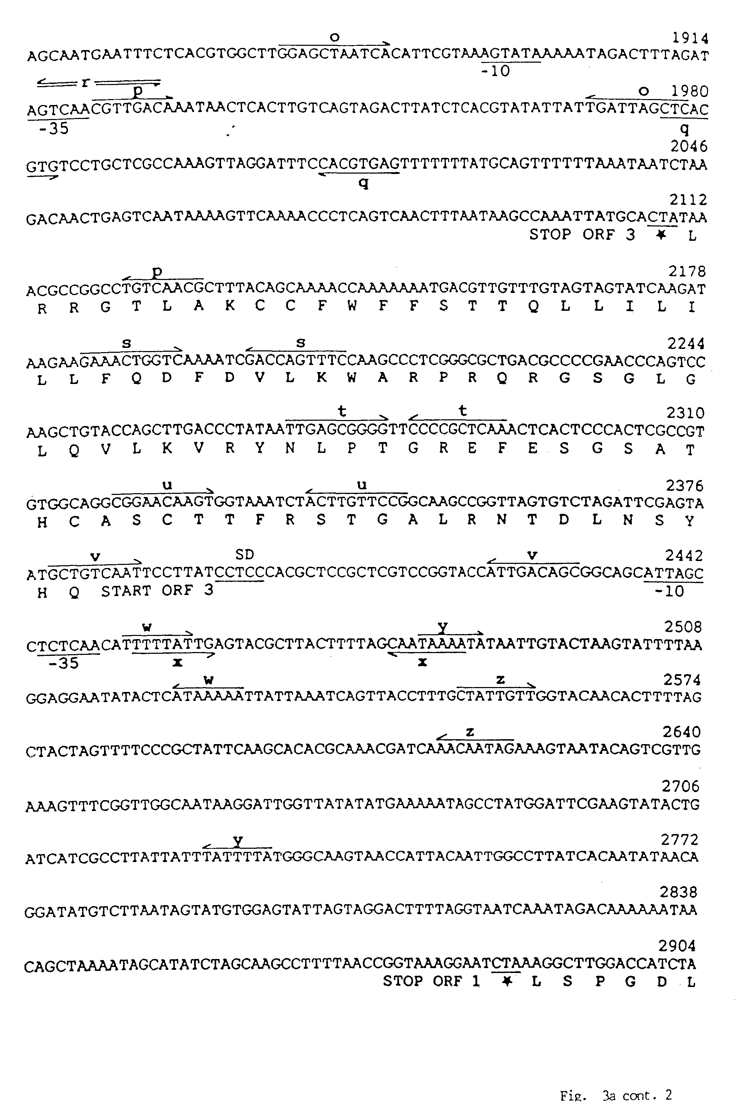

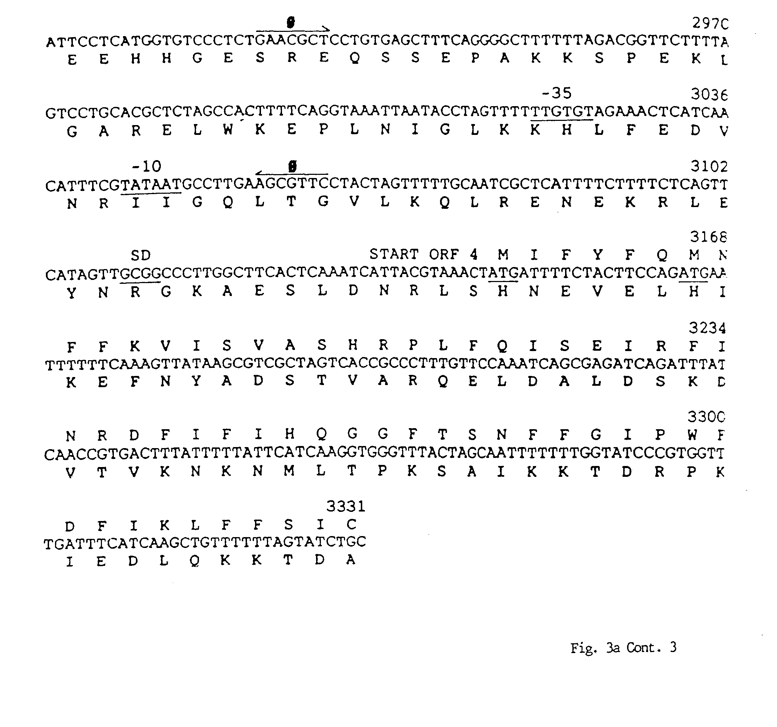

The invention further relates to the plasmid designated hereinafter as "pLH1" or "pLAB1000", the nucleotide sequence of which appears in Fig. 3a, and to cloning vectors for lactic acid bacteria which are derived from pHL1 and contain at least the regions of this plasmid necessary for replication in lactic acid bacteria. While this plasmid and its derivatives find preferred use for the production of lactic acid bacteria which produce enzymes which degrade polysaccharides and/or oligosaccharides into fermentable carbohydrates, they can also be used as vectors for the production of transformed lactic acid bacteria which, in cell culture media, produce other proteins. In this regard, the invention concerns plasmids derived from pLH1 in which other nucleotide sequences, preferably coding for determined polypeptides that are to be produced in lactic acid bacteria, are cloned.

The invention also extends to modified plasmids derived from pLH1 and containing a nucleotide sequence foreign to that plasmid and coding for a determined polypeptide inserted either within an exogenous DNA of the present invention, preferably an amylase, or in another part of the plasmid under the control of its own regulation sequences. In the latter case, a sequence encoding amylase can then be used as a screenable transformation marker. In other words, bacteria transformed with the so-modified plasmid and expressing the foreign nucleotide sequence can be detected by their amylase producing capability.

The invention also relates to plasmids derived from pLH1 which comprise a promoter operable in lactic acid bacteria, such as the PRLB promoter of Lactobacillus hilgardii, the DNA sequence of which is shown in Fig. 7a (Platteeuw, 1986). DNA fragments, which are normally not expressible in lactic acid bacteria due to the absence of a suitable promoter, can be easily cloned in these pLH1-derived expression vectors to achieve expression of the DNA fragments in these bacteria.

The invention still further relates to a process for transforming lactic acid bacteria with one or more exogenous DNAs by electroporation. Preferably, electroporation is carried out with rapidly growing, exponential phase, bacterial cells, particularly as measured at an O.D.$_{600}$ equal to 0.2 to 0.8, preferably 0.4 to 0.6. It is also preferred that the cells be washed, immediately before electroporation, with water or conventional buffers of low ionic strength. It is further preferred that the electric field be applied to a concentrated suspension of the cells of $10^{10}$-$10^{11}$ Colony Forming Units ("CFU")/ml in a low ionic strength buffer. This process avoids the need for forming protoplasts of the lactic acid bacteria before transformation.

EP 0 311 469 A2

The processes for transforming lactic acid bacteria (e.g., Lactobacillus plantarum and Enterococcus faecalis) with one or more exogenous DNAs by inserting such exogenous DNAs in the bacterial chromosomes by means of transposon vectors or by means of homologous recombination will be further described in the Examples which follow and which illustrate the invention. The figures, referred to in the Examples, are as follows:

Figure 1 Restriction map of the Bacillus thuringiensis amylase gene.

Figure 2 DNA sequence of the B. thuringiensis amylase gene and the amino acid sequence of the amylase encoded by the DNA sequence.

Figure 3 a. Nucleotide sequence of the pLH1 or pLAB1000 plasmid.

b. Physical map of the pLAB1000 plasmid (with different numbering from Fig. 3a).

c.

A. homology of ORF 1 with other site-specific recombinases.

B. homology of $RS_A$ with target size of other site-specific recombinases.

d.

A. homology of ORF 2 with replication proteins of other organisms.

B. homology of nicking site with those of other organisms.

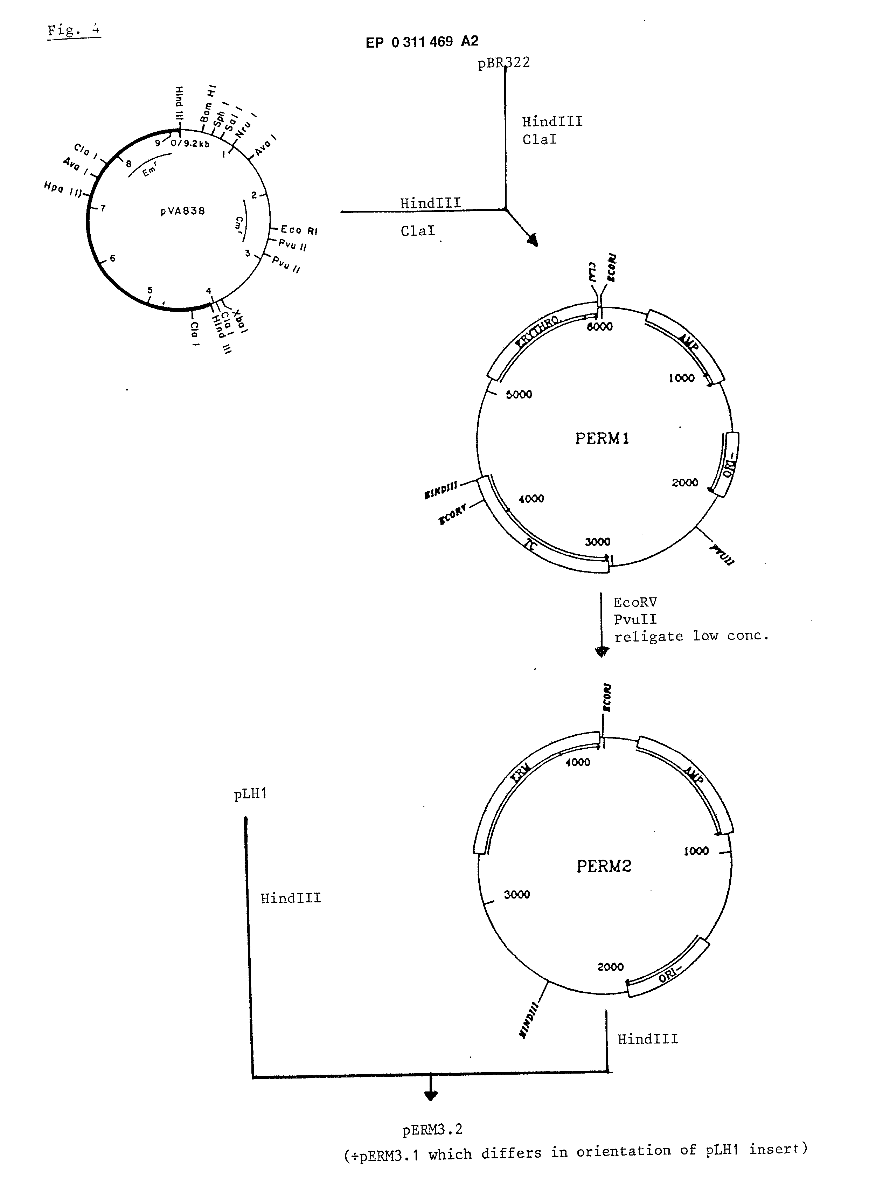

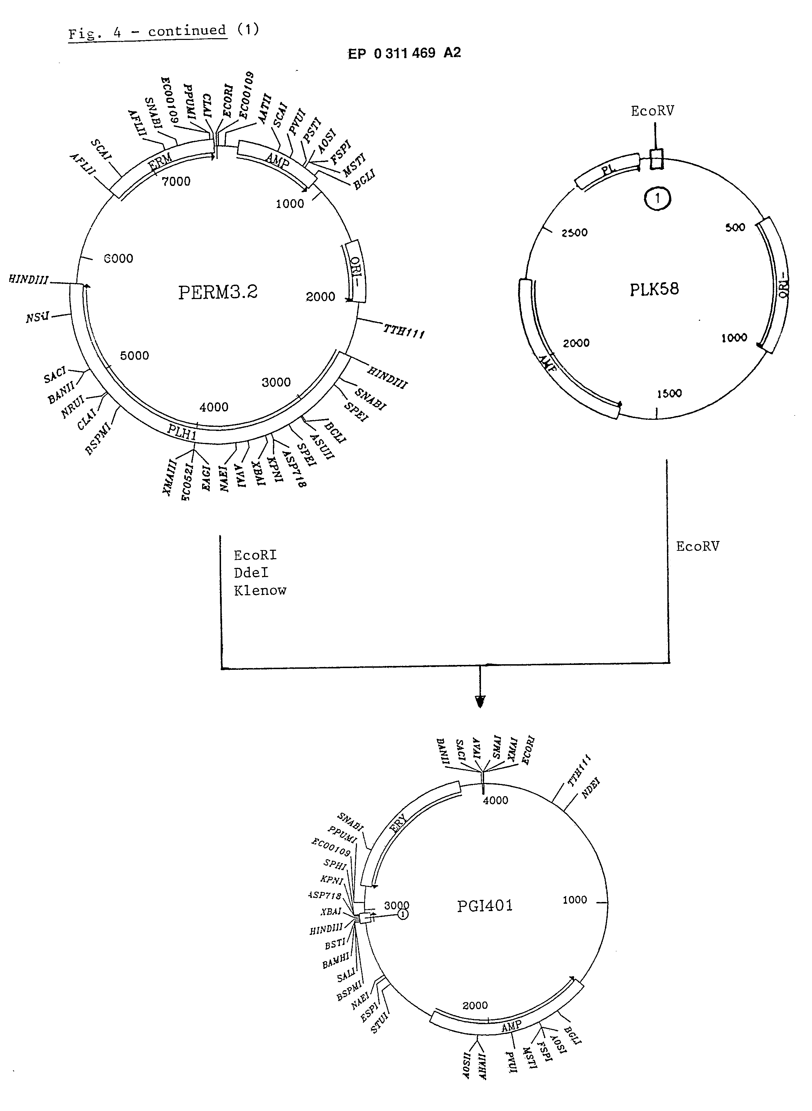

Figure 4 Construction of pGI4010, pGI4011 and pERM 3.2 derived from pLAB1000.

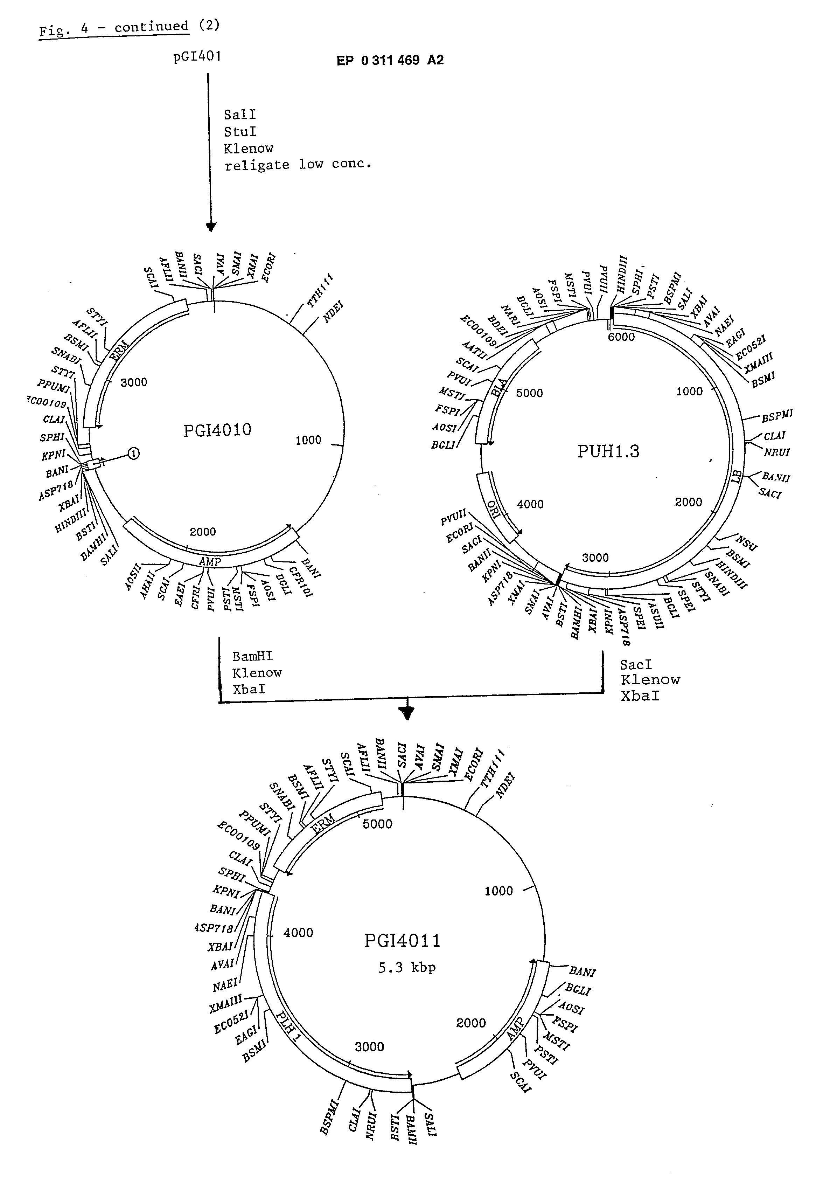

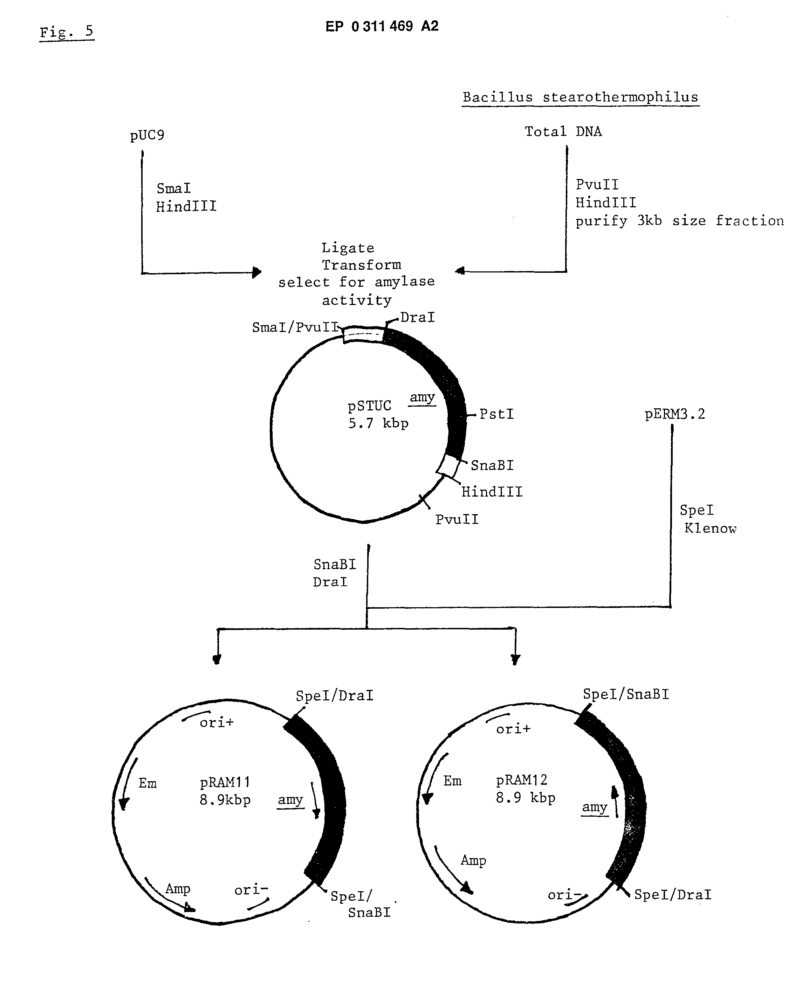

Figure 5 Construction of pRAM11 and pRAM12.

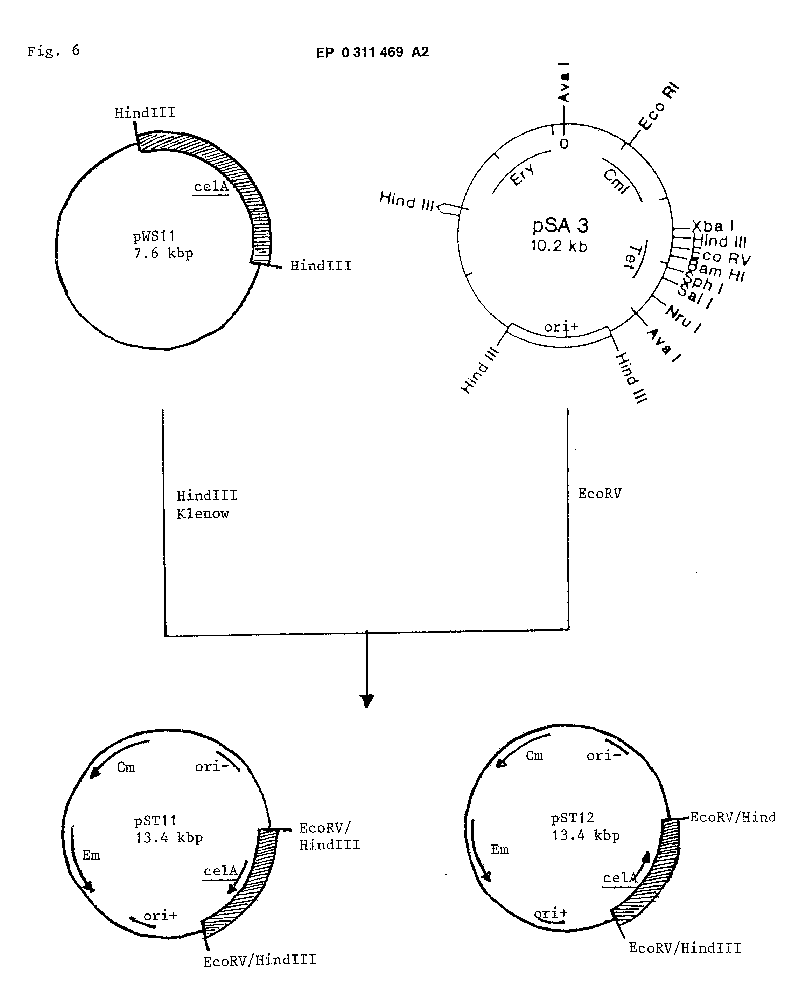

Figure 6 Construction of pST11 and pST12.

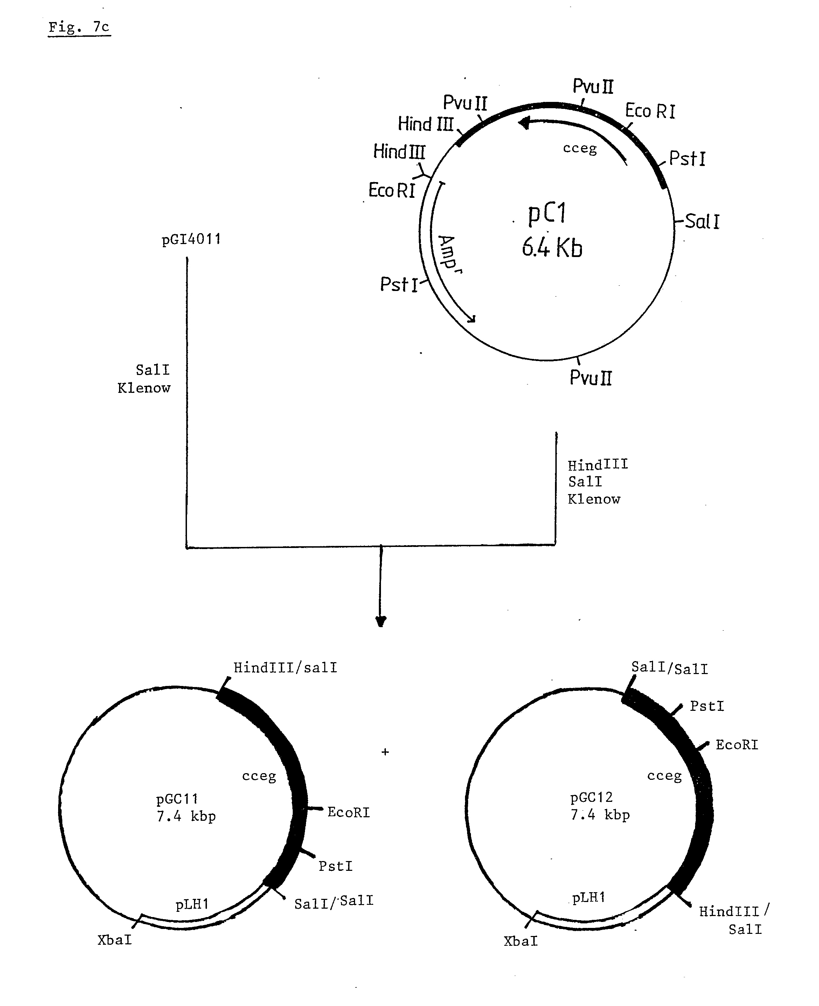

Figure 7 a. Sequence of PRLB promoter.

b. Construction of pPGV1LH.

c. Construction of pGC11 and pGC12.

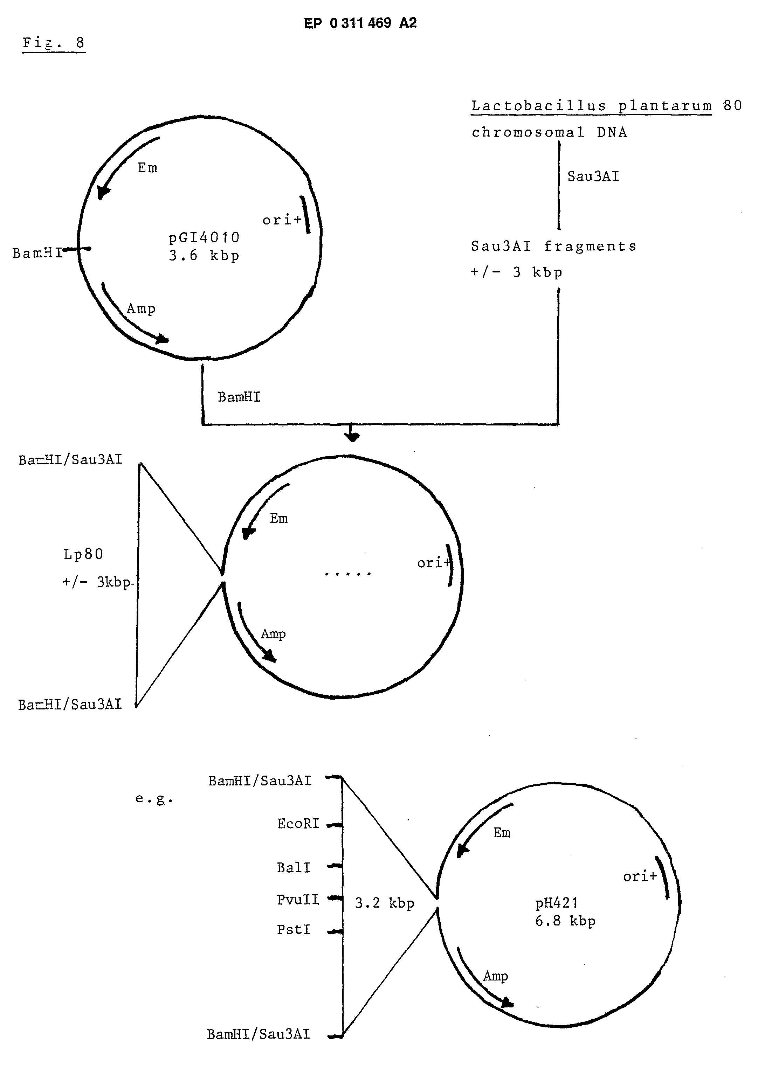

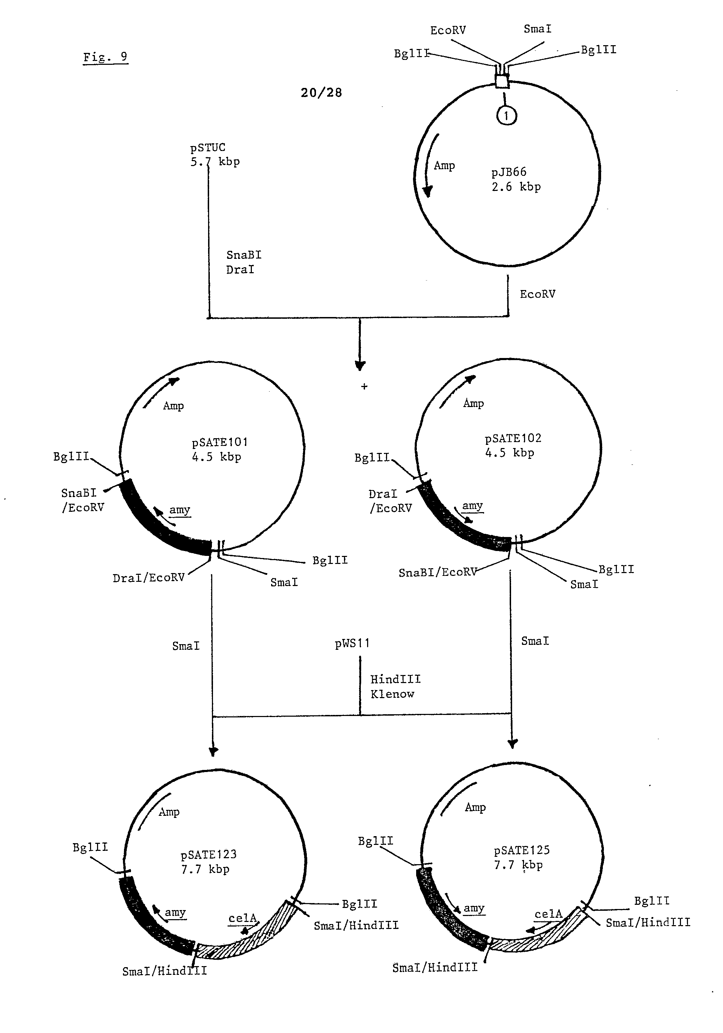

Figure 8 Construction of pH421.

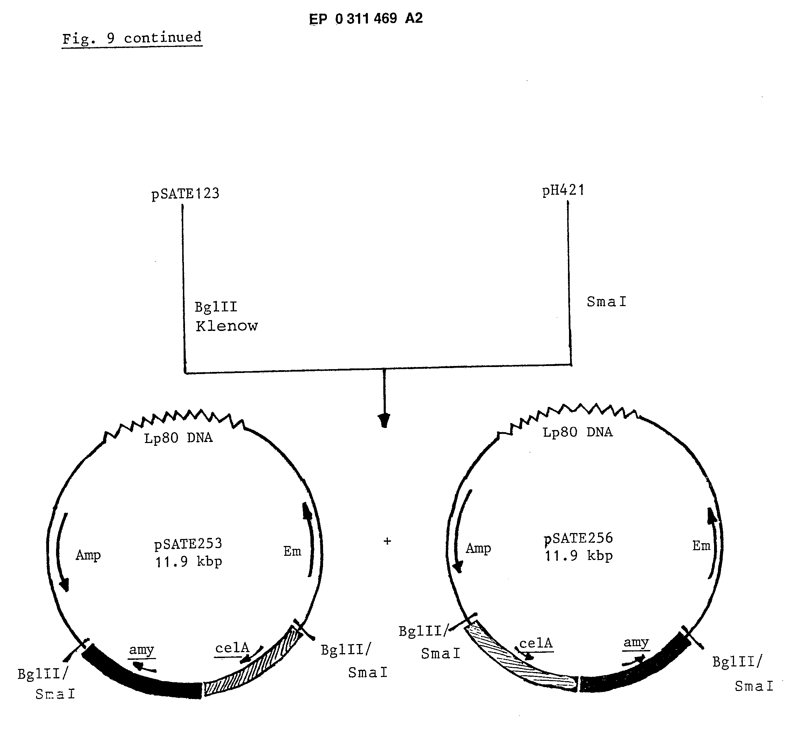

Figure 9 Construction of pSATE253 and pSATE256.

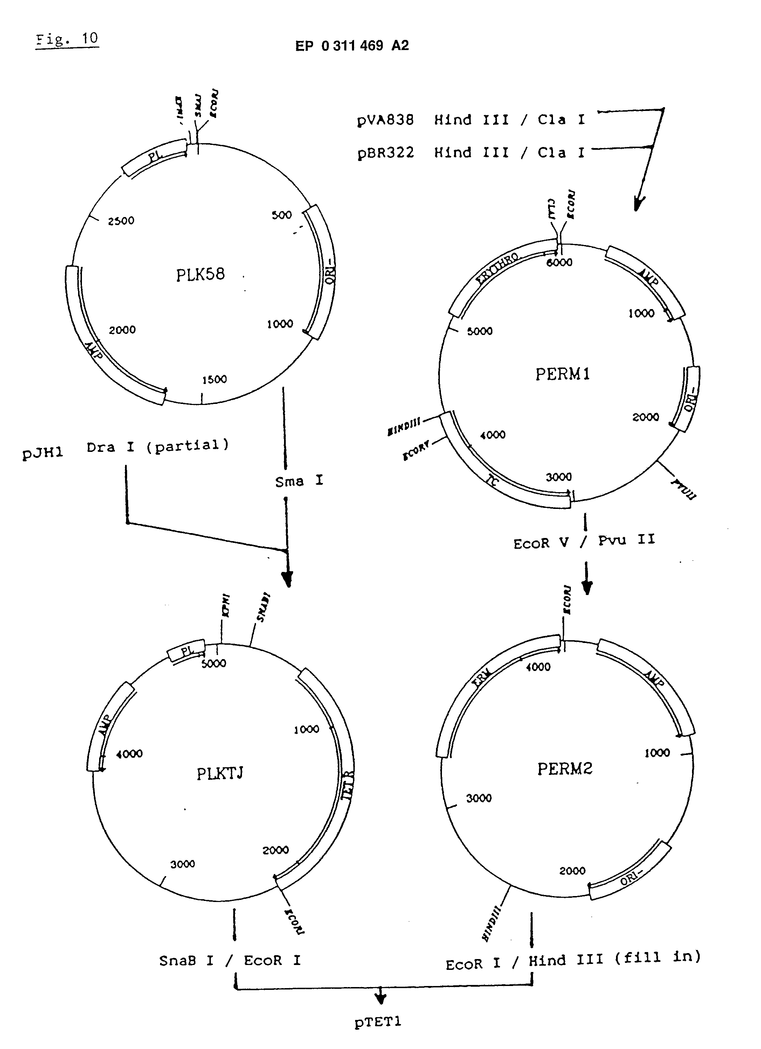

Figure 10 Construction of pTnAMYL.

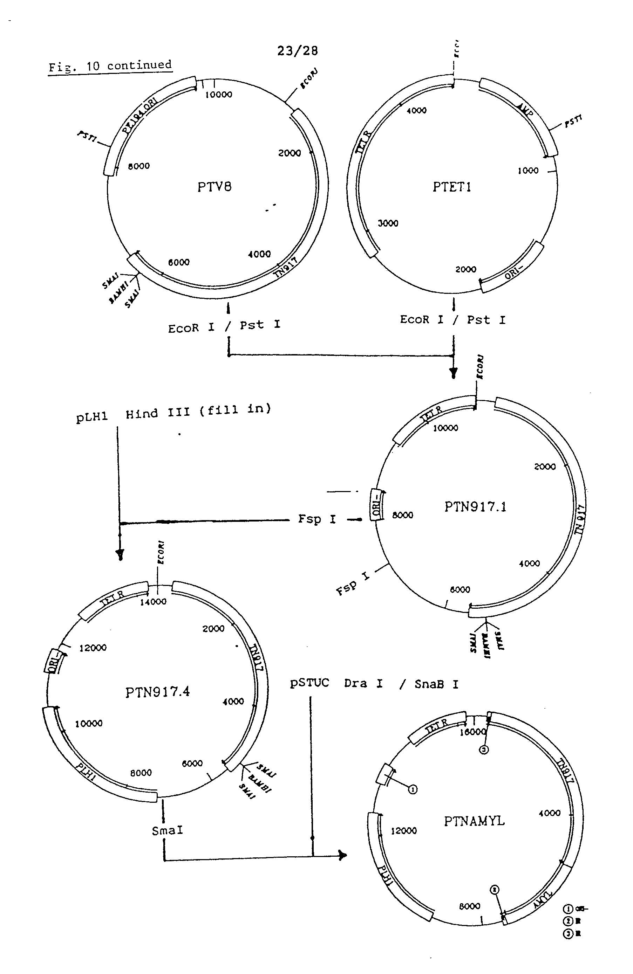

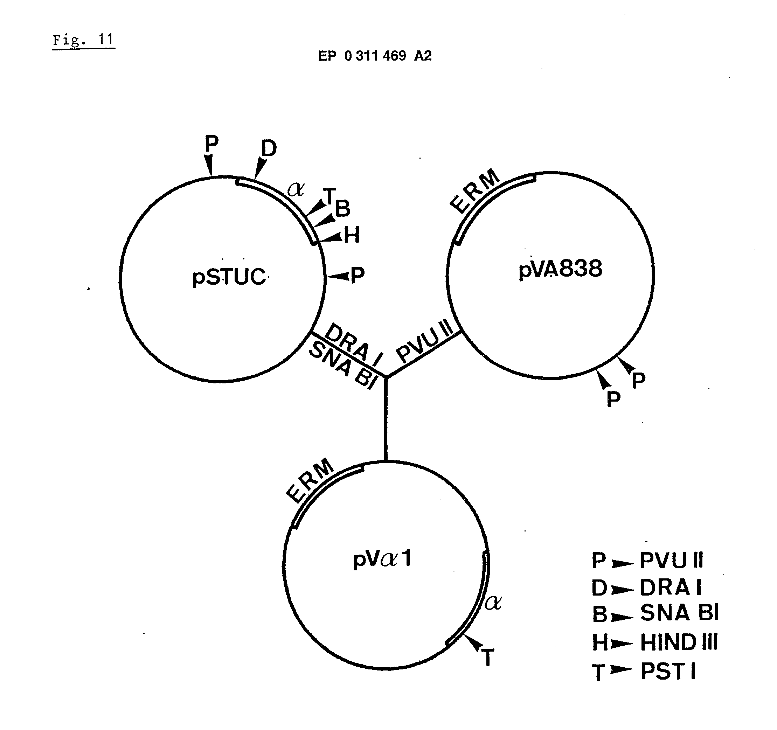

Figure 11 Construction of pVα.

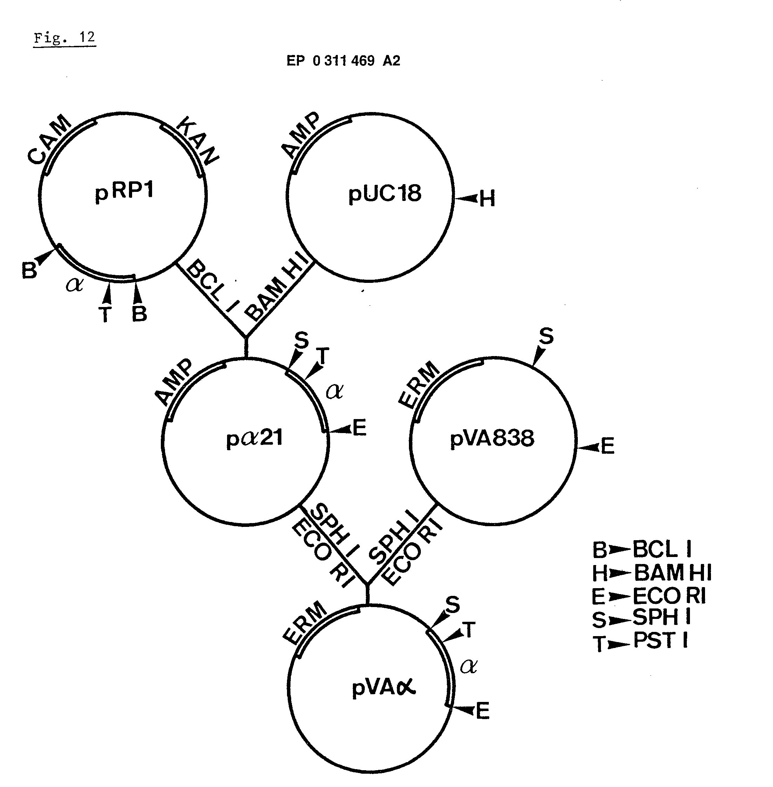

Figure 12 Construction of pVAα

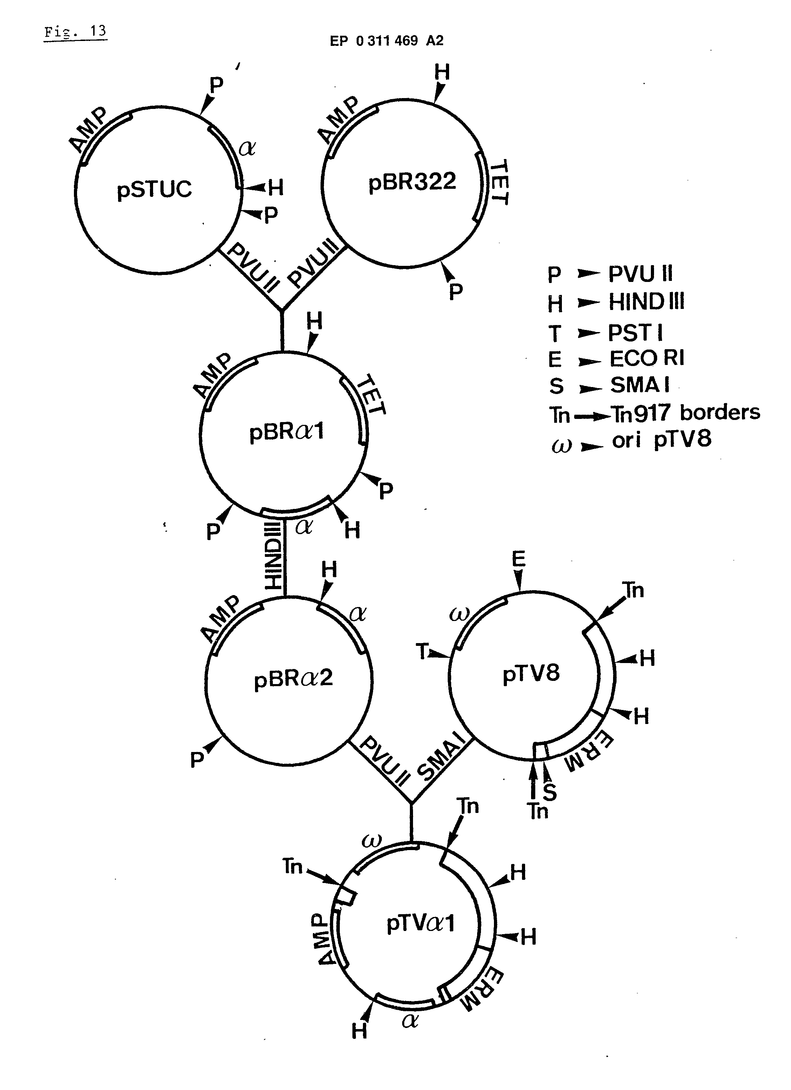

Figure 13 Construction of pTVα.

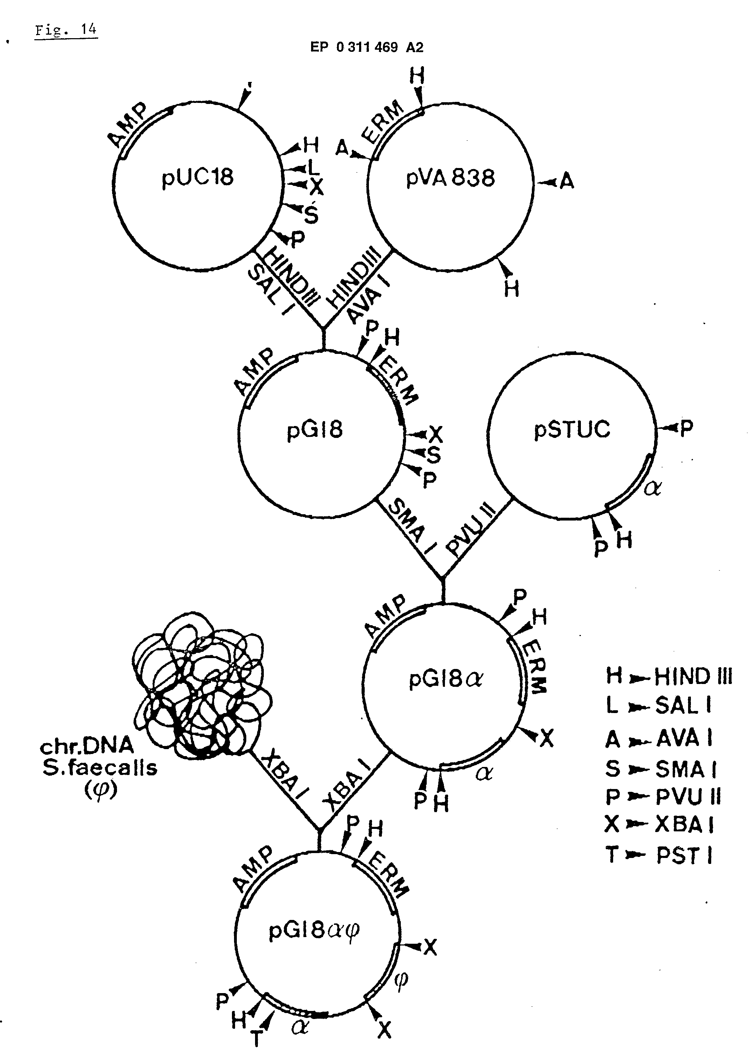

Figure 14 Construction of pG18αφ.

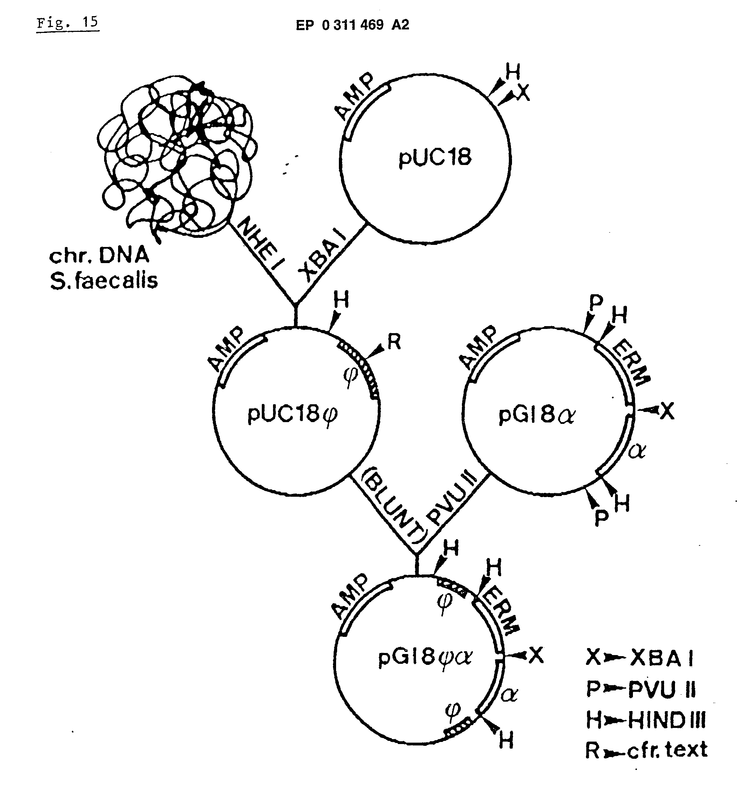

Figure 15 Construction of PG18φα. Abbreviations used in the figures and throughout the examples are:

- Em, $Em^R$, ERM, ERY, Erythro : erythromycin resistance gene
- Amp, $Amp^R$, BLA : Ampicillin resistance gene
- Cm, $Cm^R$, Cam, Cml : Chloramphenicol resistance gene
- $Km^R$, Kan : kanamycin resistance gene
- Tc, $tet^R$, Tet : tetracyclin resistance gene
- ① : polylinker
- ori : replication origin (ori- : gram negative bacteria; ori+ : gram positive bacteria)
- amy, α : α-amylase gene from Bacillus stearothermophilus
- celA : endoglucanase gene from Clostridium thermocellum
- lacpo : lac promoter
- cceg : DNA from Clostridium cellulolyticum containing an endoglucanase gene
- Lp80 DNA : chromosomal DNA from Lactobacillus plantarum 80
- PL : $P_L$ promoter from phage lambda
- LB : pLH1 sequence

Unless otherwise stated in the Examples, all procedures for making and manipulating recombinant DNA were carried out by the standardized procedures described in Maniatis et al, Molecular Cloning - A Laboratory Manual, Cold Spring Harbor Laboratory (1982). Unless otherwise stated, the Escherichia coli used for transformations was E. coli strain MC 1061 (Casadaban and Cohen, 1980).

The following Lactobacilli, used in the Examples, have been deposited in the Deutsche Sammlung Von Mikroorganismen (the "DSM"), Göttingen, Federal Republic of Germany, under the provisions of the Budapest Treaty:

| Microorganism | DSM Accession No. | Date |
|---|---|---|
| L. plantarum 80 | 4234 | 27 August 1987 |
| L. plantarum 50 | 4429 | 27 August 1987 |
| L. hilgardi 67 | 4233 | 27 August 1987 |
| LpGA39 | 4775 | 29 August 1987 |

7

The other microorganisms in the Examples are also readily available or can readily be reproduced from readily available microorganisms and genetic material without performing any inventive work.

Example 1: Cloning an α-amylase gene from Bacillus stearothermophilus

The α-amylase gene from B. stearothermophilus strain CU21 has been characterized and its DNA sequence determined (Aiba et al, 1983; Nakajima et al, 1985). Total DNA was prepared from B. stearothermophilus CU21 as described by Aiba et al. (1983), digested with PvuII and HindIII, and size fractionated on an agarose gel. The 3 kilobase ("kb") pair size fraction was recovered, and DNA was purified from this size fraction and ligated to plasmid pUC9 (Messing, 1980) which was digested with HindIII and SmaI. The ligation mixture was transformed into Escherichia coli strain JM83 (Yanish-Perron et al, 1985). Transformed cells were plated on LB agar with 100 mg/l ampicillin and 1% (by weight) soluble starch (Difco Laboratories, Detroit, Michigan, U.S.A.) and were incubated overnight at 37°C. The agar plates were then stained with iodine vapor. A halo of non-staining agar around certain colonies was indicative of amylase activity. These colonies were picked and colony purified, and plasmid DNA was prepared from them.

The DNA sequence of the insert in one of these plasmids named "pSTUC" (Fig. 5), was determined by the Maxam and Gilbert chemical degradation method (Maxam and Gilbert, 1980) and was found to be identical to the DNA sequence published previously for the α-amylase gene from this strain (Nakajima et al, 1985).

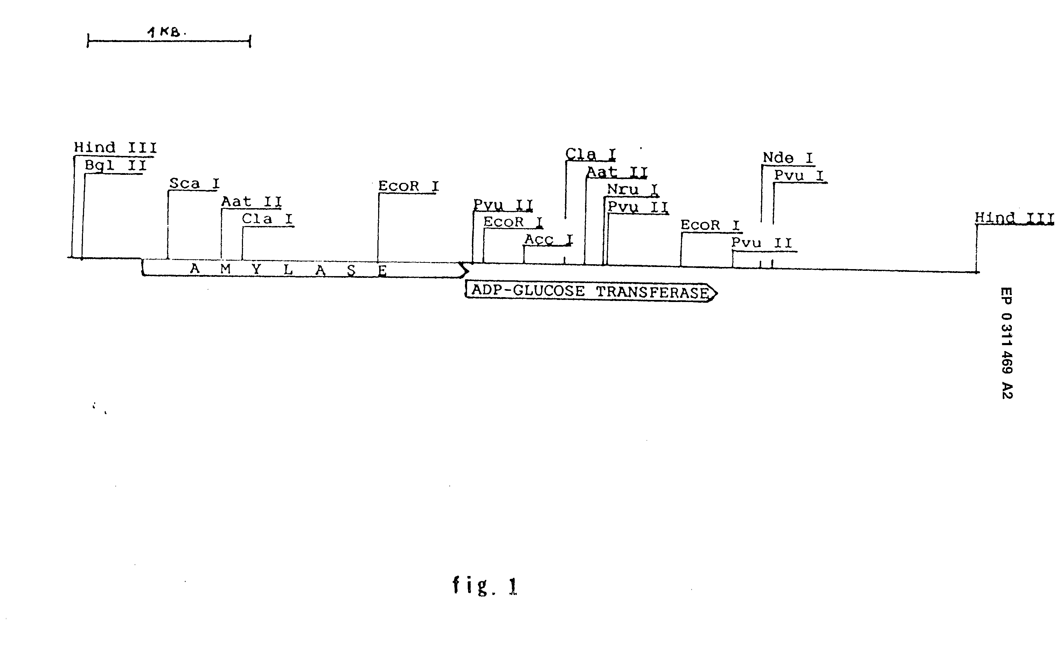

Example 2: Cloning an amylase gene from Bacillus thuringiensis (Figs. 1 and 2)

Total DNA was prepared (Aiba et al, 1983) from B. thuringiensis strain H1.1, serotype H1, var. thuringiensis, obtained from Dr. Huguette de Barjac, Institut Pasteur, Paris. It was digested with restriction enzyme HindIII an ligated to bacteriophage lambda cloning vector 590 (Murray et al, 1977) cleaved with HindIII. The ligation was packaged, infected on E. coli and plated on LB agar supplemented with 1% soluble starch. Phage containing the B. thuringiensis amylase gene were identified by the halo they form after staining in iodine vapor. The existence of these halos proved the expression of the amylase gene in E. coli.

From one such phage, having a 5.6 kb pair insert, a restriction map was determined, and the amylase gene was localized more precisely by subcloning restriction fragments of its insert in plasmid pBR322 (Bolivar and Backmann, 1979) and testing amylase expression in E. coli HB101 (Bolivar and Backmann, 1979). A 2.55 kilobase pair PvuII-HindIII restriction fragment of the 5.6 kb pair insert, cloned in pBR322, exhibited strong amylase activity on agar plates supplemented with 1% soluble starch. This pBR322 clone was named "pJAM23". The DNA sequence of this fragment insert in pJAM23 was shown by sodium dodecyl sulfate ("SDS") polyacrylamide electrophoresis of E. coli cell lysates to encode a 76,000 dalton protein.

A restriction map of this fragment, including the amylase gene, is given in Fig. 1. The DNA sequence of most of the fragment insert was defined by the Maxam and Gilbert chemical degradation method and is given in Fig. 2. There is only one big open reading frame ("ORF"), oriented from the HindIII site towards the PvuII site. This encodes a 76,059 dalton polypeptide consisting of 648 amino acids. No homology was found to other α- or β- amylases such as those described in Nakajima et al (1986), Nishizawa et al (1987) and Kawazu et al (1987).

Downstream of the B. thuringiensis amylase gene is a sequence that is highly homologous on the amino acid level to the E. coli ADP glucose transferase gene (Baecker et al, 1983).

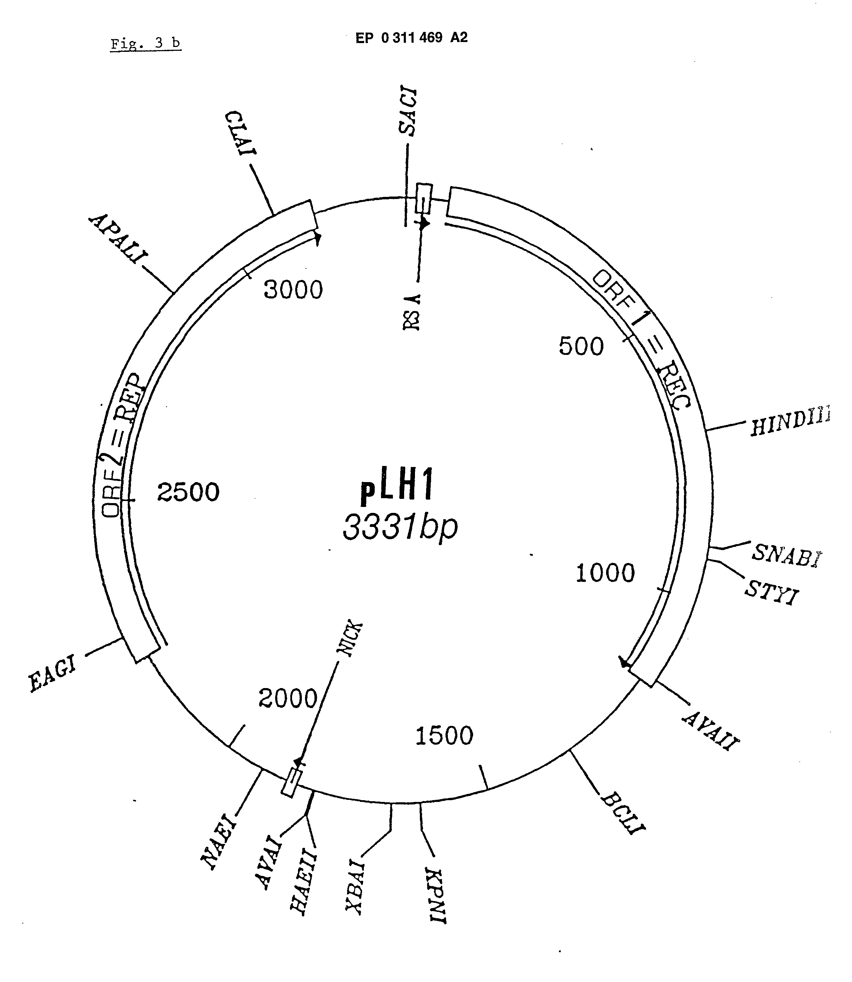

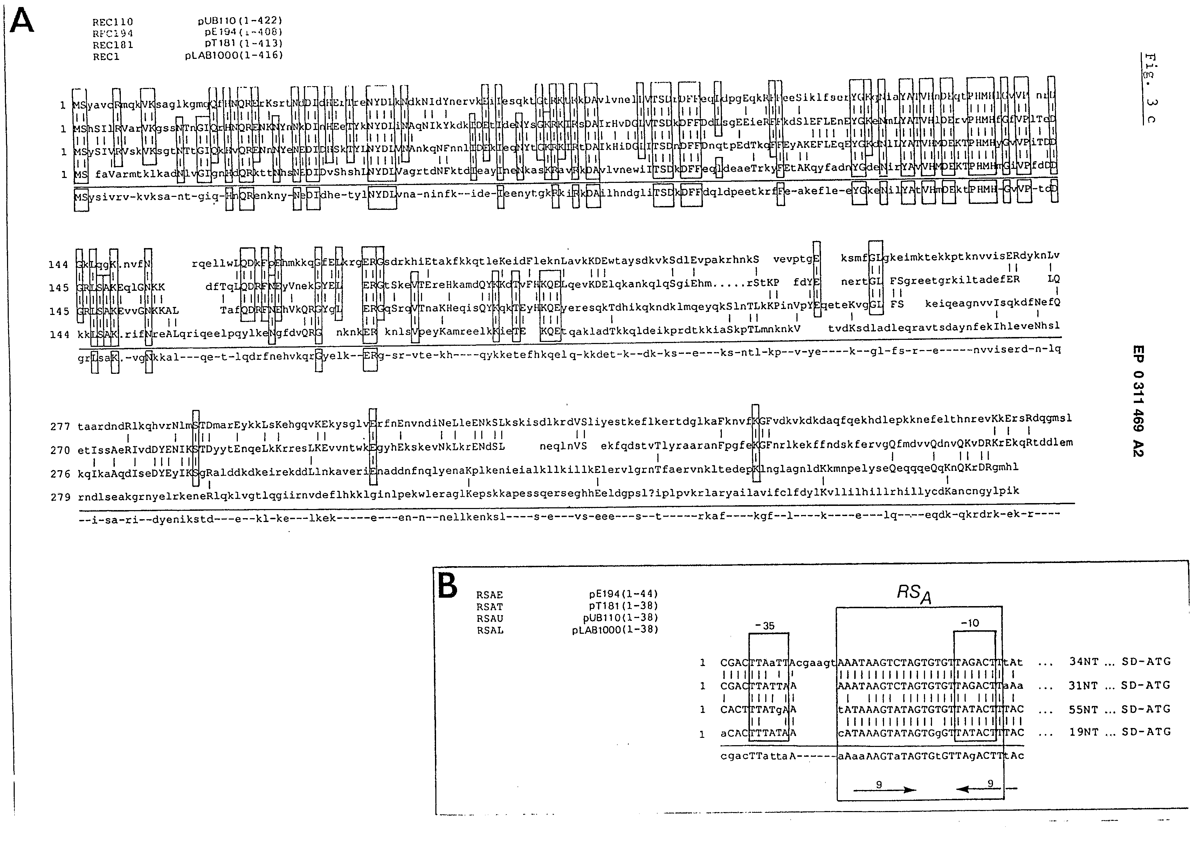

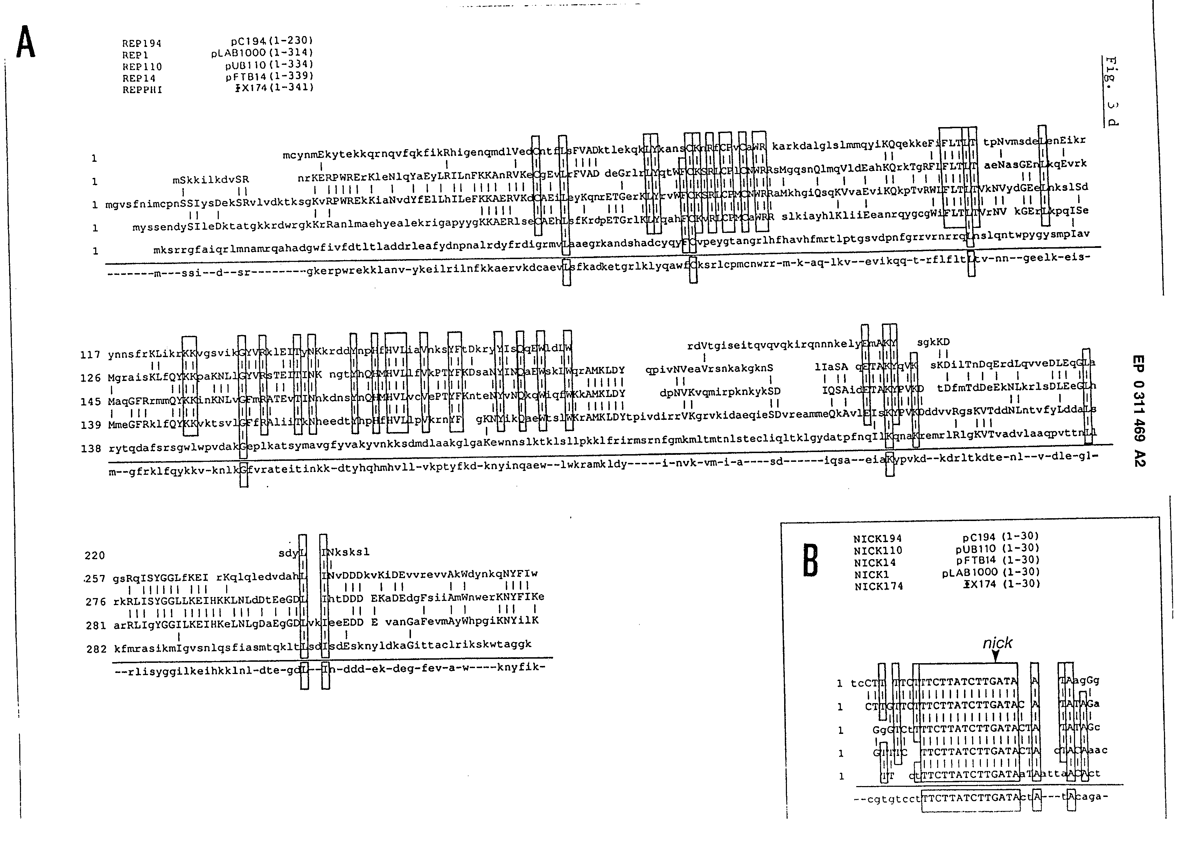

Example 3: Isolating and characterizing a Lactobacillus plasmid (Fig. 3)

Lactobacillus hilgardii 67 was isolated from grass silage by Radar N.V. (Deinze, Belgium). The strain was grown in static culture at 37°C in MRS medium (made by Oxoid Ltd., Basingstoke, Hampshire, England) supplemented with 20 mM D-L-threonine. Cells were pelleted from a late log phase culture, resuspended in 10 mM Tris-HCl pH 7.0, pelleted, resuspended in the same buffer and pelleted again. 0.2 gram of cells was resuspended in 2 ml of 20 mM Tris-HCl pH 7.0, 2ml of a 24% solution of polyethylene glycol in water were added, and 34 enzyme units of mutanolysine (Sigma Chemical Company, St. Louis, MO, USA) in water were added. The solution was incubated at 62°C for 1 hour. 0.4 mg of Proteinase K (Sigma) was added, and the cells were incubated 60 minutes at 37°C to form protoplasts.

The protoplasts were collected from the mixture by centrifugation and resuspended in 5 ml of 20 mM Tris-HCl and 10 mM ethylene diamine tetraacetate ("EDTA") at pH 8.0. SDS was added to 3% and mixed with the protoplasts which were then left at room temperature (25°C) for 30 minutes. The resulting cell lysate was centrifuged to remove unlysed cells, and the supernatant was extracted with water saturated phenol and then with chloroform-isoamyl alcohol (24/1 by volume). The nucleic acids were precipitated from the aequous phase by adding sodium acetate to 0.3 M and 1 volume of 2-propanol, followed by centrifugation. The pellet was resuspended in a mixture of 10 mM Tris-HCl/1 mM EDTA at pH 8.0. The DNA was tested for plasmid content by two dimensional gel electrophoresis (Hintermann et al, 1981).

One of the plasmids found was named "pLH1" -- later renamed "pLAB1000" -- and characterized as described below. The pLH1 plasmid was cleaved with XbaI (XbaI has 1 recognition site in pLH1) and ligated to E. coli vector pUC18 (Yanisch-Perron et al, 1985) cleaved with XbaI. The ligation mixture was transformed into E. coli strain K514 (Zabeau and Stanley, 1982), and the resulting fusion plasmid was named "pUH1.3" (Fig. 4). A restriction map of the pLH1 plasmid was determined, and the entire pLH1 plasmid was sequenced by the Maxam and Gilbert chemical degradation method. The sequence is 3331 basepairs long and has 3 ORFs reading in the same direction. A fourth ORF (87 amino acids), reading in the opposite direction, is found within ORF 1. The DNA sequence of the pLH1 plasmid is shown in Fig. 3a, and a physical map of the pLH1 plasmid, with restriction sites cleaving once or twice in pLH1, is shown in Fig. 3b.

ORF 1 in pLH1 encodes 361 amino acids. It exhibits amino acid homology with an ORF on the pE194 plasmid from Staphylococcus aureus (Horinouchi and Weisblum, 1982A), with an ORF from the pT181 plasmid of S. aureus (Khan and Novick, 1983), and with the prot B gene of pUB110 (McKenzie et al, 1987). Some of the ORFs that are homologous to the ORF 1 of pLH1 are known to encode site-specific recombinases (Gennaro et al, 1987) that act upon a target site located in the promotor of the recombinase gene. As shown in figure 3c-A, the promotor region of pLH1's ORF 1 contains significant homology with the target site of these site-specific recombinases. It is believed therefore that pLH1's ORF 1 is a site-specific recombinase, catalyzing low frequency recombination at the $RS_A$, site in the promotor region of ORF 1 (Figure 3c-B). ORF 1 and the $RS_A$ site are located on a 1819 basepair Aval-Sacl fragment of pLH1 in Fig. 3a.

ORF 2 in pLH1 shows amino acid homology with the ORF coding for protein A in plasmid pC194 from S. aureus (Horinouchi and Weisblum, 1982B; Alonso et al, 1987; McKenzie et al, 1987), with the prot A gene of plasmid pUB110 from S. aureus, and with an ORF of plasmid pFTB14 of Bacillus amyloliquefaciens (Murai et al, 1987) as shown in Figure 3d-A. These ORFs of other genes are known to encode replication proteins that initiate plasmid replication by nicking plasmids at specific sites. A nicking site was defined in pLH1 by the striking homology (Figure 3d-B) between a pLH1 DNA sequence and the nicking sites of the plasmids exhibiting homology with pLH1'S ORF 2. Moreover, as found in the other plasmids, this nicking site is located upstream of the gene encoding the replication protein in a cluster of inverted repeated sequences. ORF 2 and the nicking site are located on a 1512 basepair Aval-Sacl fragment of pLH1 between nucleotides 2221 and 710 in Fig. 3a. ORF 2 and the nicking site are both required for DNA replication and plasmid stability of pLH1 in a variety of Gram-positive bacteria (See Example 6). From this and from the direct demonstration of single stranded DNA in plasmid preparations of pLH1, it is believed that pLH1 replicates by a single strand intermediate as is the case for several other Gram-positive plasmids (Ter Riele et al, 1986).

In 66 Lactobacillus strains, sequences cross-hybridizing to pLH1 were searched by colony hybridization. Lactobacillus plantarum strains 183, 195 and 30 (isolated from grass silage by Radar N.V.) cross-hybridized to pLH1 in Southern blots of restriction-digested DNA, but the cross-hybridizing plasmids had a different restriction pattern and different size from pLH1.

## Example 4: Constructing Lactobacillus vectors based on the pLH1 replicon (Fig. 4)

Plasmid pVA838 (Macrina et al, 1982) carries the macrolide lincosamide streptogrammin B ("MLS") resistance gene, encoding resistance to erythromycin, of pAMB1 (Macrina et al, 1982). The DNA sequence of the pVA838 erythromycin resistance gene was published by Martin et al (1987) and found to be very homologous to the MLS resistance gene from Tn917 (Shaw and Clewell, 1985).

pVA838 was cleaved with HindIII and ClaI and ligated to pBR322 (Bolivar and Backmann, 1979) which was cleaved with HindIII and ClaI. The ligation mixture was transformed into E. coli, which was plated on LB agar supplemented with 100 ug/ml ampicillin. A plasmid was recovered carrying the β-lactamase and tetracycline resistance genes and the origin of replication from pBR322 joined to the erythromycin resistance gene from pVA838, and the plasmid was named "pERM1".

pERM1 was cleaved with EcoRV and PvuII and was religated in low DNA concentration. The ligation mixture was transformed in E. coli and was plated on LB agar supplemented with 100 mg/l ampicillin and 200 mg/l erythromycin. In this way, a pERM1 derived plasmid was obtained in which the pBR322 tetracycline resistance gene was deleted, and the plasmid was named "pERM2".

pERM2 was cleaved with HindIII, and ligated to pLH1 from Example 3 cleaved with HindIII. This produced plasmids "pERM3.1" and "pERM3.2" which differ in the orientation of the pLH1 fragment.

pERM3.2 was cleaved with EcoRI and DdeI, the cohesive ends were made blunt by filling in with Klenow DNA polymerase, and ligated to plasmid pLK58 (Botterman and Zabeau, 1987), cleaved with EcoRV. A plasmid was selected containing the erythromycin resistance gene of pERM3.2 inserted in pLK58, and this was named "pGI401". The $P_L$ promotor from pLK58 was removed from pGI401 by cleaving pGI401 with SalI and StuI, filling in the sticky ends with Klenow polymerase, and religating the mixture in dilute conditions, yielding plasmid "pGI4010".

pGI4010 was cleaved with BamHI, filled in with Klenow polymerase, then cleaved with XbaI, and ligated to plasmid pUH1.3 from Example 3, cleaved with SacI, rendered blunt by the exonuclease activity of Klenow polymerase, and then cleaved with XbaI. A plasmid containing pGI4010 as well as the pLH1 XbaI - SacI fragment was selected after transformation and plating on LB plates containing 100 mg/l ampicillin and 200 mg/l erythromycin, and was named "pGI4011". The BamHI site was regenerated at the BamHI-SacI fusion.

## Example 5: Introducing plasmids from Example 4 into strains of Lactobacillus and other Gram-positive bacteria

Lactobacillus plantarum 80, isolated from grass silage by Radar N.V., is noted for its rapid acid production, high acid resistance, high growth rate, homofermentative growth in silage and survival after lyophilization. Several methods can be used to transform this strain with pLH1 derived plasmids. Electroporation, as described below, is particularly efficient.

An overnight saturated culture of L. plantarum 80 was diluted 100 fold in MRS medium prewarmed at 37°C and grown in an aerobic shaking culture in MRS medium at 37°C. When the exponential culture reached an optical density ("O.D.") of 0.2-0.8 at 600 nm, its cells were centrifuged for 10 minutes at 5,000 g, resuspended in 1/20 of the original culture volume of sterile distilled water and centrifuged for 10 minutes at 10,000g. They were then resuspended in 1/100 culture volume of an autoclaved solution of 30% (weight by volume) polyethylene glycol 100 (Merck Schuchardt, Munich, Federal Republic of Germany) in distilled water. All centrifugations and washings were performed at room temperature (25°C). After these manipulations were completed, the cells were put on ice.

For each transformation of L. plantarum 80 cells by electroporation with a plasmid from Example 4, 0.8 ml of cell suspension was mixed with 4.0 µg of the vector as a 0.3 µg/ml solution in 10 mM Tris-HCl/l mM EDTA at pH 8.0, and placed in a 4 mm cuvette in a BioRad Gene Pulser (Bio Rad Chemical Division, 1414 Harbour Way, South Richmond, California 94804, U.S.A.) Plasmid DNA was introduced in L. plantarum 80 cells by discharging a single pulse of 1700 volts, capacity of 25 µF, over the cuvette. Typically, time constants of 15 - 20 milliseconds were observed (Time constant is the decay time from peak voltage to 1/e X peak voltage).

After the pulse, the cells were left on ice for 30 minutes in the original electroporation cuvette. Then, 10 ml of MRS were added, and the cells were incubated 1 to 2 hours at 28°C. 100 µl of cells (approximately $10^8$ to $10^9$ cells having received 0.05 µg of DNA) were plated on MRS medium supplemented with the appropriate antibiotic: either 10 mg/l chloramphenicol or 10 mg/l of erythromycin. The cells were incubated 48 hours at 37°C. 100 mg/l of lincomycin (Sigma) was also added to the MRS medium before incubation because most erythromycin resistance genes used in this Example, especially the erythromycin resistance gene from pVA838, also induce resistance to lincomycin. Moreover, the addition of lincomycin reduced the frequency of spontaneous erythromycin-resistant mutants of L. plantarum 80 from $10^{-7}$ down to less than $10^{-9}$. By this procedure, $10^6$ transformant colonies of L. plantarum 80 per microgram of input DNA were obtained.

Using this procedure, high efficiency transformation of L. plantarum 50 (another bacterium isolated from silage by RADAR N.V., and noted for its qualities as a silage starter strain), Lactobacillus casei strain DSM20020 (on deposit at the DSM under accession no. 20020), Enterococcus faecalis strain OG1X (Ike et al, 1983), Bacillus subtilis strains 1A40 and 1A42 (from Bacillus Genetic Stock Center, Columbus, Ohio, USA), B thuringiensis strains kurstaki DIPEL (from Abbott Laboratories, Abbott Park, North Chicago, Ill, U.S.A.), entomicides (received from Dr. D. Lereclus, Inst. Pasteur, Paris France) and an isolate of berliner 1715 (received from Prof. A. Klier, Inst. Pasteur, Paris, France), Bacillus cereus BCT4 (received from B. Carlton, Ecogen, Langhorne, PA, U.S.A.), Lactococcus lactis NIZO 22186 (from Nederlands Instituut voor Zuivelonderzoek, Ede, The Nederlands), could be obtained. Optimizations of the electroporation procedure for specific strains were done as described in Chassy and Flickinger (1987) and in the BioRad manual accompanying the Pulse Controller.

Example 6: Studying replication properties of pLH1 in several Gram-positive bacteria

It was found that plasmids (e.g., pGI4011 from Example 4), containing the pLH1's ORF 2 and nicking site (see Example 3) in a 1652 basepair XbaI - SacI fragment of pLH1, can replicate autonomously in Gram-positive bacteria. This was demonstrated for L. plantarum 50 and 80, L. casei DSM20020, E. faecalis strain OG1X, and B. subtilis strain 1A42. There was no evidence of autonomous replication of pLH1 in various strains of E. coli. Several pLH1-based replicons, which did not contain the intact 1652 basepair XbaI - SacI fragment of pLH1 (with the ORF 2 and the nicking site), were unable to replicate in Gram-positive bacteria.

Several plasmids originating from different Gram-positive bacteria were introduced in L. plantarum 80 by electroporation following the procedures of Example 5. The comparison plasmids were: pVA838 (Macrina et al, 1982), pSA3 (Dao and Feretti, 1985), pTG402 (Zukowski et al, 1983), pTV8 (Youngman, 1987), pDB64 (Gryczan et al, 1980) and pGKV2 (Kok et al, 1985). L. plantarum 80 cells transformed with the comparison plasmids and with pERM3.1 ( from Example 4) were grown for 13 generations on MRS medium (Oxoid Manual, 1982) without antibiotic pressure. The persistence of the transformed plasmid DNA in such non-selective growth conditions was verified by plating cultures on MRS agar (Oxoid Manual, 1982) supplemented with 10 mg/l erythromycin or chloramphenicol (as appropriate) before and after the non-selective growth for 13 generations. Only pLH1 was maintained in more than 30% of the cells after the nonselective growth for 13 generations. The other plasmids were lost in more than 90% of the cells after non-selective growth for 13 generations. This demonstrated the superiority of pLH1 as a cloning vehicle in Lactobacillus.

Plasmids replicating via single strand intermediates like pLH1 (from Example 3) are prone to reorganizations and deletions when used in recombinant DNA constructions (Noirot et al, 1987). No instability was observed in any of the cloning experiments with pLH1. In this regard, during the plasmid transformations in Examples 3, 4, 7 and 8, it was verified by restriction digests of DNA from plasmid minipreparations of transformed bacteria (Anderson & McKay, 1983) that the native configuration of the pLH1 plasmid did not change.

Example 7: Expressing an amylase gene and an endoglucanase gene in L. plantarum 80 using vectors based on pLH1 (Figs. 5 and 6)

α-amylase and endoglucanase genes were inserted in separate vectors replicating in L. plantarum 80 and assayed for expression as follows.

Plasmid pSTUC (Example 1) was cleaved with SnaBI and DraI, and ligated to pERM3.2 cleaved with SpeI and made blunt end by filling with Klenow polymerase. Two recombinant plasmids carrying the pSTUC fragment with the amylase gene inserted in both orientations in the pERM3.2 replicon were selected by their amylase production (as evidenced by the assay described in Example 1) and erythromycin 200 mg/l resistance in E. coli. They were named "pRAM11", and "pRAM12", respectively (Figure 5). Upon introduction in L. plantarum 80, both pRAM11 and pRAM12 replicate stably under erythromycin selective pressure, express amylase and secrete the enzyme into the medium (as evidenced by staining the MRS agar plates containing 1% soluble starch with iodine vapor). Since both pRAM11 and pRAM12 produce similar amounts of amylase activity, the expression of the amylase gene must have been directed from the native (B. stearothermophilus) promotor. The amylase expression appears not to have been repressed by presence of glucose in the medium, and not to have been dependent on the growth phase of the strain.

Plasmid pWS11, described by Schwarz et al (1986) and obtained from these authors, carries the Clostridium thermocellum endoglucanase cel A gene and surrounding sequences including the promotor on a 3.2 Kb HindIII fragment. The sequence of this gene is described in Beguin et al (1985). This gene has an endoglucanase activity towards carboxymethyl cellulose ("CMC") and towards lichenan which is a β-1-3-glucan (Hazlewood et al, 1988).

pWS11 was cleaved with HindIII, the cohesive ends were filled in by Klenow polymerase, and ligated to plasmid pSA3 (Dao and Feretti, 1985) cleaved with EcoRV. Plasmids having the cel A gene inserted in the pSA3 EcoRV site of pSA3 were identified among transformants in E. coli, plated on LB agar supplemented with chloramphenicol 25 mg/l, by: replica plating several colonies on LB agar containing chloramphenicol 25 mg/l and 0.5 g/l CMC (Sigma, low viscosity); allowing these replica plates to grow overnight at 37°C; and then flooding them with a solution of 10 g/l Congo Red (Merck, Darmstadt, Federal Republic of Germany) in water for 15 min at room temperature. The Congo red solution was then poured off, and the plates were washed with 1 M NaCl. Colonies producing endoglucanase were surrounded by a yellow halo on a red background (Cornet et al, 1983A). Two plasmids carrying the endoglucanase in a different orientation were identified, and named "pST11" and "pST12" (Fig. 6). Upon introduction in L. plantarum 80, both pST11 and pST12 replicated stably under erythromycin (10 mg/l) selective pressure, and secreted endoglucanase enzyme into the medium. Expression was not repressed by glucose in the culture medium. Endoglucanase activity could also be demonstrated by: incubating supernatants of liquid cultures of the transformed L. plantarum 80 containing pST11 or pST12 in wells of agar plates containing 1g/l CMC; measuring the viscosity decrease of aqueous CMC solutions upon incubation with said culture supernatants as described by Cornet et al (1983B); or growing the L. plantarum 80 containing pST11 or pST12 in MRS medium from which the normal glucose constituent was omitted, incubating the culture supernatant with a solution of CMC, and measuring the reducing sugars by the method of Somogyi-Nelson (Nelson, 1952). Since both pST11 and pST12 produced similar amounts of endoglucanase activity, the expression of the endoglucanase gene must have been directed from the native C. thermocellum promotor.

The stability of the plasmids pST11, pST12, pRAM11, pRAM12 was good with selective antibiotic (10 mg/l erythromycin) pressure. Without such selective pressure, the plasmids were gradually lost in growing cultures as described in Example 26.

## Example 8: Constructing a pLH1 derived expression vector (Fig. 7)

A promotor sequence was isolated from L. hilgardii 67 by digesting L. hilgardii 67 total cellular DNA (prepared according to Example 3) with Sau3AI and ligating the fragments in vector pPGV5 cleaved with BamHI (Fig. 7b). pPGV5 was derived from pPGV2 (D'Haese et al, 1984) by modifying the polylinker sequence in front of its promotorless CAT86 gene (Cm$^R$) (Fig. 7b). The ligation mixture was transformed in Bacillus subtilis strain 1A40, and the recombinants were selected for chloramphenicol resistance. The DNA sequence of one particular Sau3AI fragment, inducing very high levels of chloramphenicol acetyl transferase, is shown in Figure 7a. The fragment was named the "PRLB promoter". The pPGV5 vector with this Sau3AI fragment was named pPGV1LH (Platteeuw, 1986) and could also transform L. plantarum 80 to provide chloramphenicol resistance.

Plasmid pC1, obtained from Dr. Julio Polaina (Instituto de Agroquimica y Technologica de Alimento, Jaime Roig 11, Valencia, Spain) is described in Polaina et al (1988) and its restriction map is shown in Figure 7c. E coli, transformed with this plasmid, express the Clostridium cellulolyticum endoglucanase as an intracellular enzyme. This endoglucanase can be demonstrated on LB agar plates containing 1g/l CMC after the colonies have been lysed with chloroform vapor. The endoglucanase gene from Clostridium cellulolyticum was excised as a HindIII - SalI restriction fragment, made blunt end with Klenow, and was ligated to plasmid pGl4011 (from Example 4) which was cleaved with SalI and made blunt end with Klenow. The resulting plasmids were named "pGC11" and "pGC12". The structure of pGC11 and pGC12 was identical except for the orientation of the endoglucanase gene (Figure 7c). pGC11 and pGC12 produced small amounts of non-secreted endoglucanase in E. coli but no detectable intracellular or extracellular endoglucanase activity when introduced in L. plantarum 80 by the electroporation procedure of Example 5.

By inserting the PRLB promoter in pGC11 and pGC12, upstream from the C. cellulolyticum endoglucanase gene, the expression of the gene can be induced in L. plantarum 80. In this regard, endoglucanase activity can be detected by the methods described in Example 7.

## Example 9: Transforming L. plantarum 80 by integration of DNA sequences in the chromosome (Fig. 8)

Using the procedure described for Bacillus subtilis by O'Kane et al (1986) and the references cited therein, heterologous DNA was integrated in the chromosome of L. plantarum 80 by a single recombination event in a region of homology between a plasmid and the chromosome.

Total cellular DNA was prepared from L. plantarum 80 as described for L. hilgardii in Example 3. This DNA was cleaved partially with Sau3AI, so as to produce restriction fragments with an average size of 3 kilobase pairs (verified on agarose gel). These fragments were ligated in plasmid vector pGl4010 (from Example 4),

which was cleaved with BamHI, and the ligation mixture was transformed in E. coli. Minipreparation plasmid DNA was prepared from 48 transformants, and the presence of the chromosomal DNA insert was verified by restriction digests. The plasmid DNAs were combined in 8 pools of 6 plasmids each and were introduced in L. plantarum 80 by electroporation as in Example 5. About 10 transformants per μg of DNA were obtained with each of the pools. pERM3.2 was introduced in the same batch of L. plantarum 80 cells at a frequency of 5x10$^5$ transformants per μg of plasmid DNA. Electroporation with pure pGI4010 yielded no transformants. Since 8 independent pools gave rise to transformants, it appears that at least 8 (and probably more than 1 out of each 6) random 3 Kbp Sau3AI fragments of L. plantarum 80 DNA can be used as homology regions mediating chromosomal insertion of pGI4010 derived vector sequences.

From 20 L. plantarum 80 colonies, transformed with pGI4010 carrying a chromosomal Sau3AI insertion and named "LpGI1" to "LpGI20", plasmid DNA was prepared as described by Anderson and McKay (1983), and total cellular DNA was prepared as described in Example 3. Southern hybrydization showed the absence of pGI4010 related sequences in the plasmid fraction except in one transformant. This DNA was digested with restriction enzyme EcoRI, separated on an agarose gel, transferred to a nylon membrane (HyBond N, Amersham), and hybridized with radioactively labeled pGI4010 plasmid. The hybridization showed the absence of pGI4010 in the plasmid fraction of the LpGI transformants and in L. plantarum 80 and the presence of pGI4010-related material in the digested chromosomal DNA in 19 out of 20 transformants. In one transformant, the LpGI4010 sequences had integrated in the indigeneous plasmid of L. plantarum 80. The intensity of hybridization to the chromosomal DNA was higher than expected from a single copy insertion and varied in a way unlinked to the amount of chromosomal DNA loaded on the gel. Moreover, the pattern was more complex than expected for a single copy insertion. Therefore, the LpGI strains may carry multiple insertions of pGI4010 in the chromosome.

An EcoRI digest was made from total cellular DNA of LpGI2, LpGI4 and LpGI7 and from wild type L. plantarum 80. This DNA was diluted to low concentration and ligated, and transformed in E coli. No ampicillin-resistant transformants were obtained with the ligation mixture from L. plantarum 80. Ligations of LpGI1 to LpGI20, however, gave rise to ampicillin-resistant E. coli colonies. Plasmid DNA from these colonies showed the restriction pattern of the large EcoRI - BamHI fragment of pGI4010, linked at the BamHI site to a variable fragment of chromosomal L. plantarum 80 DNA. This shows that at least part of the integrated copies of pGI4010 show no major rearrangements.

From a comparison of restriction patterns of the pGI4010 plasmids, carrying random Sau3AI fragments of L. plantarum 80 DNA, with the plasmids obtained from selecting ampicillin resistance among circularized fragments of LpGI1 to LpGI20, it was possible to identify the plasmid that became inserted in LPGI4. This plasmid was named "pH421", and a concise restriction map of it is given in Figure 8. pH421 has a 3.2Kbp homology region with the L.plantarum 80 chromosome.

It was investigated whether the integrated DNA in LpGI strains is stably inherited and whether it influences the phenotype of the host strain. LpGI1 to LpGI20 were mixed with wild type L. plantarum 80 cells at a ratio of 1 LpGI cell per 9 L. plantarum 80 cells, and the mixture was allowed to grow for 13 generations in MRS liquid medium without any selective pressure. Appropriate dilutions of the resulting culture were plated on MRS agar and on MRS agar supplemented with 10mg/l erythromycin and 100mg/l lincomycin. It was found that 17 out of 20 LpGI strains have the same growth rate as the wild type L. plantarum 80 in this assay, and three strains, LpGI5, LpGI10, and LpGI20, do not grow as fast as L. plantarum 80.

LpGI2, LpGI4, and LpGI5 were mixed with L. plantarum 80 (wild type) at a ratio 1:10, and the mixture was inoculated at 10$^6$ CFU per gram in autoclaved Italian Rye grass. As in the above experiment, LpGI2 and LpGI4 had the same growth rate as L. plantarum 80, whereas LpGI5 grew slower. The ratio of 1/10 was maintained for over 5 days, indicating that the survival of LpGI2 and LpGI4 in grass was not affected.

LpGI2, LpGI4, and LpGI5 were grown for 78 generations in MRS liquid medium without any selective pressure for maintenance of the pGI4010 sequences. After growth for 13, 26, 39, 52, 65 and 78 generations, aliquots of the cultures were plated on MRS agar and on MRS agar supplemented with 10 mg/l erythromycin and 100 mg/l lincomycin. 100 colonies, that appeared on the MRS agar plate, were replicated on MRS agar supplemented with 10mg/l erythromycin and 100mg/l lincomycin. In this assay, more than 95% of the cells of LpGI2 and LpGI4 maintained their erythromycin resistance over 78 generations. However, in LpGI5, erythromycin sensitive cells appeared after 39 generations, and hardly any erythromycin resistant cells (less than 5%) could be found after 65 generations. Thus, integrated sequences are stable in many transformants (e.g., LpGI2 and LpGI4) but not in all transformants (e.g., LpGI5).

The fermentative abilities of LpGI1 to LpGI20 for 49 carbohydrates were tested by means of an API CH50L test) (API System S.A., La Balme les Grottes, 38390 Montalieu Vercieu, France). No differences were found between the fermentative abilities of any LpGI strain andL. plantarum 80.

The pattern of total cellular soluble proteins of the LpGI1 to LpGI20 strains and the L. plantarum 80 strain was compared by electrophoresis on SDS-PAGE (SDS - Polyacrylamide gel electrophoresis) (Lambert et al, 1987). No significant differences were found between the strains.

This experiment demonstrated the feasability of stably integrating exogenous DNA in the chromosome of L. plantarum 80 without detectably modifying the phenotype of the strain. Particularly, this experiment showed that strain LpGI4 is generated by integration of plasmid pH421 in the chromosome of L. plantarum 80 and that LpGI4 has the same phenotype as L. plantarum 80 and has a stable integration of pGI4010 sequences. pH421 is therefore used in the following examples as a vehicle for inserting an amylase gene and an endoglucanase gene in the chromosome of L. plantarum 80.

Example 10: Transforming L. plantarum 80 by integrating amylase and endoglucanase genes in the chromosome (Fig. 9)

Plasmid pSTUC (from Example 1) was cleaved with SnaBI and DraI and ligated with plasmid pJB66 (Botterman and Zabeau, 1987) cleaved with EcoRV. Two hybrid plasmids carrying the 1.9 Kbp pSTUC fragment with the amylase gene as an insertion in opposed orientations in pJB66 were selected by screening for amylase activity and restriction mapping of plasmid minipreps from candidate transformants. The plasmids were called "pSATE101" and "pSATE102" (Fig. 9).

Plasmid pWS11 (Schwarz et al, 1986) was cleaved with HindIII, the cohesive ends were made blunt by filling in with Klenow polymerase, and the digest was ligated to pSATE101 cleaved with SmaI. A plasmid was selected that had the 3.2 Kbp HindIII fragment with the cel A gene of pWS11 inserted in the SmaI site of pSATE101. This plasmid was named "pSATE123". An analogous construction was made for pSATE102 and was named "pSATE125". The direction of transcription of the cel A gene and the amylase gene was parallel in pSATE123 and antiparallel in pSATE125 (Figure 9). Both plasmids expressed amylase and endoglucanase activity in E. coli as detected by the assays described in Example 7. Moreover, both the amylase and the endoglucanase genes could be excised as a single BglII fragment.

pSATE123 was cleaved with BglII, and the cohesive ends were made blunt ended by filling in with Klenow polymerase. The resulting fragments were ligated to plasmid pH421 (from Example 9) which was cleaved with SmaI. Two plasmids were selected which had the amylase - endoglucanase cassette integrated in the SmaI site of pH421. They were named "pSATE253" and "pSATE256", and the orientations of the respective genes are shown in Figure 9.

Plasmid DNA of pSATE253 and pSATE256 was introduced in L. plantarum 80 by electroporation as described in Example 5, and selected on MRS agar containing 10mg/l erythromycin and 100mg/l lincomycin. About 3 transformants per µg were obtained with each plasmid. In the same electroporation experiment, pERM3.2 was introduced in L. plantarum 80 at a frequency of $5 \times 10^6$ transformants per µg plasmid DNA.

8 transformants of L. plantarum 80 with pSATE253 were named "LpGA3.1" - "LpGA3.10" (LpGA3.7 and LpGA3.8 were not allocated) and selected for further study. 11 transformants of L. plantarum 80 with pSATE256 were named "LpGA6.1" - "LpGA6.11" and selected for further study. All of these LpGA strains (except LpGA6.2) expressed both amylase and endoglucanase activity as determined by the methods described in Examples 1 and 7. One of the LpGA strains, LpGA 3.9, which expressed such activity, was deposited with the DSM.

Undigested chromosomal DNA of each LpGA strain was run on an agarose gel, blotted on Hybond N paper and hybridized with pSATE253. In all strains, except LpGA6.2, hybridization occurred with chromosomal DNA. LpGA6.2 hybridized with an episomal factor, and this clone probably resulted from a non-homologous insertion of pSATE256 in the indigeneous L. plantarum 80 plasmid. As for the LpGl strains of Example 9, the fermentation patterns of LpGA 6.3, 3.9 and 6.6 (as determined by the API-CH50L test is identical to L. plantarum 80, except for the positive reaction on starch of the LpGA strains. Also, the SDS-PAGE of soluble cell proteins reveals no difference between the wild-type and the transformed strains.

Example 11: Constructing a Lactobacillus transposon vector ("pTnAMYL") carrying an α-amylase gene (Fig. 10)

Plasmid pJH1 from E. faecalis (Banai and Le Blanc,1983) was cleaved partially with restriction enzyme DraI and ligated to plasmid pLK58 (Botterman, 1986) cleaved with SmaI. The ligation mixture was transformed into E. coli strain K12ΔHΔ1 trp (Bernard et al, 1979), and the transformed cells were plated on LB agar supplemented with 10 µg/ml tetracycline. A transformant was recovered that had the pJH1 tetracycline resistance gene inserted in the pLK58 SmaI site, and the transformant was named "pLKTJ". The DNA sequence of the pJH1 tetracycline resistance gene was determined and found to be very homologous to the sequence of the tetracycline resistance gene of plasmid pTHT15 from Bacillus stearothermophilus (Hoshino et al, 1985). pLKTJ was cleaved with SnaBI and EcoRI and was ligated to pERM2 (from Example 4) cleaved with HindIII filled in with Klenow polymerase and EcoRI. The ligation mixture was transformed in E. coli strain K514 (Zabeau and Stanley, 1982), and cells were plated on LB agar supplemented with 10µg/ml tetracycline. A plasmid was recovered that carries the pJH1 tetracycline resistance gene linked to the pERM2 replication origin and β-lactamase gene, and the plasmid was named "pTET1".

pTET1 is cleaved with restriction enzymes EcoRI and PstI, is ligated to plasmid pTV8 (Youngman, 1987) cleaved with EcoRI and PstI, and is transformed into E. coli strain HB101. Cells are plated on LB agar supplemented with 10 µg/ml tetracycline. A plasmid carrying the Tn917 element from PTV8 and the tetracycline resistance gene and gram negative origin of replication from pTET1 is recovered, and the plasmid is named "pTn917.1".

Plasmid pLH1 (from Example 3) is linearized by cleavage with HindIII, the cohesive HindIII ends are filled in with the Klenow fragment of E. coli DNA polymerase I, and the linearized plasmid is then ligated to pTn917.1 cleaved with FspI.

The ligation mixture is transformed into E. coli and is plated on LB agar supplemented with 10 µg/ml tetracycline. A plasmid is recovered in which both parental plasmids are joined, and the plasmid is named "pTn917.4".

pTn917.4 is cleaved with restriction endonuclease SmaI and is ligated to pSTUC (from Example 1) cleaved with SnaBI and DraI.

The ligation mixture is transformed into E. coli and plated on LB agar supplemented with 10 µg/ml tetracycline. Transformants are replicated on LB plates, on which 10 µg/ml tetracycline and 1% soluble starch were added, and were tested for secreted amylase activity by incubating them briefly with iodine vapor. A plasmid is recovered carrying the α-amylase gene inserted into the SmaI site of the Tn917 counterpart of pTn917.4 and exhibiting amylase activity. The α-amylase gene is oriented with its direction of transcription pointing from the inverted repeat of Tn917 towards the erythromycin gene. This plasmid is named "pTnAMYL".

Example 12: Introducing the pTnAMYL Transposon Vector carrying an α-amylase gene into two strains of Lactobacillus plantarum

L. plantarum 50 and 80 are transformed with pTnAMYL using the procedure described in Example 5.

100 colonies, exhibiting resistance to erythromycin are replicated onto MRS agar supplemented with 1% soluble starch, 50 µg/ml tetracycline and 10 µg/ml erythromycin. Colonies producing amylase are identified by staining the agar plates with iodine vapor. The native configuration of pTnAMYL in these cells is verified by restriction digests on DNA from plasmid minipreparations (Anderson and McKay,1983).

Example 13: Transforming L. plantarum with an α-amylase gene by integrating the pTnAMYL Transposon Vector in the chromosome of L. plantarum

20 colonies of cells of each strain from Example 12, exhibiting amylase activity, are purified and are inoculated in 1 ml of MRS liquid medium with 10 µg/ml erythromycin.

After overnight incubation at 37°C, each culture is diluted to 20 ml with MRS, nalidixic acid is added to 1.0 µg/ml, novobiocin is added to 0.1 µg/ml and erythromycin is added to 10 µg/ml (Novobiocin and nalidixic acid in the above concentrations cure lactobacilli from pLH1).

After 6 hours of growth as a static culture at 37°C, 0.1 ml of each culture is taken, and 10 ml of MRS (supplemented with erythromycin, novobiocin and nalidixic acid as above) is added. When each culture reaches saturation, 10 µg/ml aliquots are plated on MRS agar with 10 µg/ml erythromycin. The derivatives that are cured of pTnAMYL are identified among the growing colonies by colony hybridization with $^{32}$P radioactively labeled pLH1 DNA as a probe. 20 colonies which show no hybridization to pLH1 are tested and are found to be sensitive to tetracycline and resistant to erythromycin. These colonies are purified, and DNA is prepared from liquid cultures as in Example 3.

An AvaI restriction digest of this DNA is size separated on agarose gels and is then transferred to nylon membranes by Southern blotting. The membranes are hybridized to $^{32}$P radioactively labeled Tn917 DNA to verify insertion of the unmodified pTnAMYL construct in multiple sites of the L. plantarum genome. The erythromycin resistant, tetracycline insertion mutants exhibit α-amylase activity on starch plates.

Example 14: Transforming L. plantarum with the amylase gene from B. thuringiensis

Following the procedure set forth in Example 11, pJAM23 (from Example 2) is cleaved with restriction endonucleases PvuII and HindIII, and the cohesive ends are filled in with DNA polymerase I Klenow fragment. The resulting fragments are ligated to pTn917.4 cleaved with SmaI. After transformation into E. coli strain HB101, a tetracycline resistant colony exhibiting amylase activity is selected, and its plasmid is named "pTnAMYL2". As with pTnAMYL of Example 11, the direction of transcription of pTnAMYL2 points from the inverted repeat of Tn917 towards the erythromycin gene.

pTnAMYL2 is introduced into L. plantarum strains 50 and 80 following the procedure set forth in Examples 5 and 12 and is integrated into their chromosomes as described in Example 13.

Example 15: Constructing a shuttle plasmid ("pVα") containing an α-amylase gene from B. stearothermophilus (Fig. 11)

The vector pVA838 (ATCC catalog number 37160) is able to replicate in E. coli and Streptococcus sanguis (Macrina et al, 1982) and in E. faecalis (Wirth et al, 1986). pVA838 was prepared from the E. coli strain DB11 obtained from Dr. Clewell (Clewell, USA) using the rapid LiCl "mini prep" procedure (Wilimzig, 1985). pVA838 was digested with PvuII, and pSTUC (from Example 1) was digested with SnabI and DraI. The restriction digests were phenol extracted and ligated. The ligation mixture was used to transform E. coli (strain tgl, Amersham International) which were spread on LB plates containing 80 µg/ml erythromycin plus 0.2% soluble starch. This permited the selection and visualization of transformants harboring recombinant plasmids in which the small region between the two PvuII sites of pVA838 was substituted by the SnabI-DraI fragment from pSTUC containing the α-amylase gene (Fig. 11). Single transformant colonies were checked for the presence of the expected recombinant plasmid by restriction analysis of minipreparation plasmid DNA (Wilimzig, 1985). One such transformant, named "pVα", was retained for use as a shuttle plasmid in the transformation of E. faecalis as described below in Example 16.

Example 16: Transforming E. faecalis protoplasts with the pVα shuttle plasmid containing an α-amylase gene

The E. faecalis OG1X strain (Ike et al, 1983), obtained from Dr. Clewell, was used to prepare protoplasts according to the method of Wirth et al (1986). A 600 ml culture of the OG1X strain in THB (Todd-Hewith broth, made by Oxoid Ltd. Basingstroke, Hampshire, England), supplemented with 2% glycine, was brought to an O.D. of 0.15 (at 650 nm). Cells were collected by centrifugation and resuspended in 10 ml of THB containing 6% glycerol. Aliquots (1 ml) were frozen at 70°C until they were treated with lysozyme (1 mg/ml) and 10 mg/ml bovine serum albumin ("BSA") in TSCM buffer (TriS-HCl 100 mM, pH 8.0; Na Succinate 500mM; CaCl$_2$ 40mM; MgCl$_2$, 10mM) during 120 min. at 37°C to prepare protoplasts. Under these conditions, the efficiency of protoplasting was > 99,99 %, and protoplasts could be regenerated at a frequency > 90%.

The protoplasts were collected by centrifugation (10 min. at 3,000 g), were washed once in 10 ml of TSCM buffer containing 100 μg/ml BSA, and were resuspended by gentle shaking in 1 ml of the same buffer. A 100 μl sample of the protoplasts (approximately 10$^8$ cells) was mixed with about 100 μl (about 1 μg) of the pVα shuttle plasmid (from Example 15) in 2 X SSC (1 X SSC is 0.15 M NaCl plus 0.015 M Na-citrate). The mixture was added to 1.8 ml of filter sterilized 40% polyethyleneglycol with a molecular weight of 1000 ("PEG"; Sigma) in TSCM buffer. Mixing of the protoplasts, pVα and PEG was done by inverting five times a tube containing these materials. Then, this tube was incubated for 3 min. at 0°C and then for 5 min. at 37°C. The mixture in the tube was then added to a centrifuge tube containing 10 ml of TSCM buffer, supplemented with BSA (100 μg/ml). Protoplasts, recovered after a 10 minute centrifugation at 12,000 g, were washed once in 10 ml of THB medium containing Na Succinate (135 mg/ml) and BSA (100 mg/ml). The protoplasts were resuspended in 1 ml of the same medium and incubated for 120 min. at 37°C for phenotypic expression of transformed DNA. Finally, 20 to 100 μl samples were plated onto THB agar plates containing (per liter) 8 g of agar, 135 g of Na Succinate, 100 mg BSA and 5 mg erythromycin.

After 2 to 3 days incubation at 37°C, erythromycin resistant colonies were obtained at a frequency of about 10$^3$/μg DNA. They were replicated onto agar THB plates containing 20 μg/ml erythromycin plus 0.2% soluble starch. Erythromycin resistant colonies displayed a clear halo upon exposure to iodine vapor. Their plasmids were further identified by restriction analysis of mini preparation plasmid DNA (Ish-Horowicz and Burke and 1981). As expected, erythromycin-resistant, α-amylase positive colonies harbored the pVα recombinant plasmid. The halo surrounding the colonies on starch plates exposed to iodine vapor indicate that the B. stearothermophilus α-amylase gene was actively expressed in E. faecalis and that the α-amylase was secreted into the medium.

### Example 17: Constructing another shuttle plasmid ("pVAα") from B. licheniformis (Fig. 12)

The B. licheniformis α-amylase had been cloned from different strains, and its sequence had been determined by several groups (Ortlepp et al, 1983; Sibakov and Palva, 1984; Piggott et al, 1984; Joyet et al, 1984; Stephens et al, 1984; Yuuki et al, 1985; Gray et al, 1986). Plasmid pRPI bearing the α-amylase gene from the B. licheniformis RP01 as a BclI fragment (Piggott et al, 1984) was received from Dr. D.J. McConnell, Department of Genetics, Trinity College, Univ. Dublin, Republic of Ireland. This plasmid was restricted with BclI, and the 2.5 kb BclI fragment was recovered from 0.8% agarose gel in TBE buffer after electrophoresis for 2 hours at 75V in a horizontal submarine apparatus filled with TBE buffer. Recovery was performed according to Maxam and Gilbert (1980). This 2.5 kb BclI fragment containing the complete transcription unit for the α-amylase (Piggott et al, 1984) was ligated into pUC18 (Yanisch-Perron et al, 1985) previously cut with BamHI. The ligation mixture was transformed into E. coli strain JM101 (Messing et al, 1981). Colonies harboring recombinant plasmids comprising the pUC18 backbone plus the 2.5 kb α-amylase insert were selected on agar plates (LB supplemented with 50μg ampicillin/ml and 0.2% starch). Candidates were identified by the presence of a clear halo surrounding the colony after a brief exposure to iodine vapor. The identity of the recombinant plasmids present in such transformants was checked by restriction analysis performed on plasmid DNA prepared according to the LiCl rapid procedure (Wilimzig, 1985). One transformant colony harboring the expected recombinant plasmid was retained. After single colony purification, it was used for large scale purification of the recombinant plasmid by the alkaline lysis-CsCl method. This plasmid was named "pα21".

Plasmid pVA838 was prepared as before and digested with EcoRI and SphI. In parallel, the plasmid pα21 was digested with the same enzymes. The restriction digests were phenol-extracted and ligated. The ligation mixture was used to transform E. coli strain tgl (Amersham). Spreading on LB plates containing 80 μg/ml of erythromycin plus 0.2% starch allowed the selection and visualization of transformants harboring recombinant PVA838 plasmids whose EcoRI-SphI insert coming from pα21 and containing the α-amylase gene. Single transformant colonies were checked for the presence of the expected recombinant plasmid by restriction analysis of rapid DNA preparations as described before. One transformant colony harboring the expected recombinant plasmid was retained. After single colony purification, it was used for small scale purification of the recombinant plasmid according to Wilimzig (1985). This plasmid was named "pVAα" and used for the transformation of E. faecalis OG1X protoplasts strain as described in Example 16. After 2 or 3 days of incubation at 37°C, erythromycin-resistant colonies were obtained at a frequency of about 10$^3$/μg DNA. They were replicated onto agar THB plates containing 50 μg erythromycin/ml plus 0.2% soluble starch. All colonies displayed a clear halo upon exposure to iodine vapor. Their plasmids were further identified by restriction analysis of mini-preparation plasmid DNA prepared according to Ish-Horowicz and Burke (1981). As expected, all transformants harbored the pVAα recombinant plasmid. The large halo surrounding the colonies clearly indicated that B. licheniformis α-amylase gene was actively expressed in E. faecalis, and that the α-amylase protein was released into the medium.

Example 18: Constructing other shuttle plasmids ("pTVα") containing the α-amylase gene from B. stearothermophilus (Fig. 13)

The vector pTV1 can be propagated in B. subtilis (Youngman, 1987) or E. faecalis (Weaver and Clewell, 1987) as an autoreplicative plasmid but only at low temperatures (e.g., 32°C). At non-permissive temperatures (e.g., 48°C), its origin of replication is no longer functional, and the plasmid is lost in a few generations. However, the erythromycin resistance phenotype can be retained in some of the cells exposed to non-permissive conditions due to the transposition of Tn917 into the chromosome. Thus, pTV1 and its derivatives, such as PTV8 (Youngman, 1987), can be used either as an autoreplicative vector or as an integration vector as used below.

A shuttle plasmid was made in E. coli. First, pSTUC (from Example 1) and pBR322 were each digested with PvuII, phenol extracted and were ligated to yield a "pBRα1" plasmid consisting of a pBR322 backbone plus an α-amylase gene inserted at the PvuII site in the orientation shown in Fig. 13. The size of the pBRα1 plasmid was reduced by deletion of the HindIII fragment bearing the tetracycline resistance gene, yielding a "pBRα2" plasmid. The pBRα2 plasmid was restricted with PvuII, and the plasmid pTV8 was cut with SmaI. These digests were phenol extracted and ligated to yield pBRα2-pTV8 concatemers which were named "pTVα1" and "pTVα2" according to the orientation of the insert as shown in Fig. 13.

Example 19: Transforming E. faecalis protoplasts with the pTVα autoreplicative shuttle plasmids containing an α-amylase gene

The two shuttle plasmids pTVα1 and pTVα2 (from Example 18) were purified from E. coli by the LiCl rapid minipreparation method (Wilimzig, 1985) and were used to transform E. faecalis strain OGIX protoplasts as described in Example 16. Control experiments with intact pTV8 showed that the origin of replication of this plasmid was functional in the E faecalis strain at the permissive temperature (32°C). Transformation with the pTVα plasmids yielded erythromycin-resistant colonies upon selection at 32°C on THB plates supplemented with 5 μg/ml erythromycin. These transformants were replicated onto agar THB plates at 32°C supplemented with 20 μg/ml erythromycin plus 0.2% soluble starch. The erythromycin-resistant colonies displayed a clear halo upon exposure to iodine vapor. Their plasmids were further identified by restriction analysis of minipreparation plasmid DNA. Erythromycin resistant, α-amylase positive colonies harbored the pTVα plasmid. This shows that, under permissive conditions, the pTVα plasmids can be maintained in E. faecalis as autoreplicative plasmids with their amylase passenger genes being actively expressed.

Example 20: Integrating the pTVα borne α-amylase gene into E.faecalis chromosome by Tn917 transposition

Clones of E. faecalis strain OG1X with the autoreplicative pTVα1 and pTVα2 plasmids (from Example 19) were subjected to the procedure of Youngman (1987). In each case, a culture was prepared in liquid THB medium supplemented with 50μg/ml erythromycin. This culture was grown at 32°C with moderate aeration up to stationary phase. The culture was then diluted 1:50 with THB broth containing 0.5μg/ml erythromycin. This culture was grown overnight at 45°C with moderate aeration. Then, the culture was diluted again 1:50 with the same medium and grown at 45°C with moderate aeration. Two further cycles of dilution/growth at 45°C were performed. Cells were collected by centrifugation and were resuspended in the same medium to 1/25th of the original volume. Serial dilutions were prepared, plated onto THB plates containing 10μg/ml erythromycin and incubated at 28°C. Individual colonies were then replicated using sterile toothpicks onto THB plates containing 50μg/ml of erythromycin plus 300μg/ml of lincomycin, and 0.2% starch. 14 individual clones were retained in the pTVα1 and pTVα2 series, respectively, on the basis of their erythromycin/lincomycin resistance and α-amylase positive phenotype at 28°C. To demonstrate the absence of autoreplicative plasmids, each clone was subjected to the plasmid minipreparation procedure of Ish-Horowicz and Burke (1981). Each preparation Was analyzed by agarose gel electrophoresis, on the one hand, and used to back-transform E. coli, on the other hand. 13 clones from the pTVαA1 series, and 12 clones from the pTVα2 series were negative by both criteria. These were further characterized as follows.

First, the stability of the phenotypic traits resulting from the expected transposition event was demonstrated by culturing each clone under non-selective conditions, i.e., in liquid THB medium at 37°C, for 2 growth cycles. Dilutions were then made and plated onto THB plates and incubated at 37°C.

200 individual colonies were then toothpicked onto THB plates containing 50μg/ml of erythromycin, 300 μg/ml of linomycin and 0.1% starch. Upon incubation at 37°C, 12 out of 13 clones from the pTVα1 series and 12 out of 12 clones from the pTVα2 series turned out to be 100% stable. These experiments strongly suggested that the genes responsible for the stable phenotypic traits were chromosome-borne, since the same phenotypic traits turned out in control experiments, to be highly unstable under identical experimental conditions when directed by plasmid-borne genes (about 10% phenotypic reversion per generation).

In order to formally demonstrate the chromosomal location of both the erythromycin-resistance and the α-amylase marker genes as a result of transpositional integration, hybridization experiments were performed on several individual clones from each series according to the method of Southern (1975). These experiments clearly demonstrated that these genes were indeed chromosome-borne, the site of transposition being different in several individual clones.

Altogether, it was found that the α-amylase gene had been stably incorporated into the chromosome of E. faecalis and expressed as an active enzyme released into the medium, as evidenced by the presence of a halo surrounding the colonies.

By the method of Example 9, the growth rate of E. faecalis having pTVα and pVAα inserts was found to be

EP 0 311 469 A2

comparable to the growth rate of wild-type E. faecalis OGIX.

Example 21: Constructing a set of single recombination prone integration vectors ("PG18αφ") containing the α-amylase gene from B. stearothermophilus (Fig. 14)

A plasmid bearing a segment of chromosomal DNA from E. faecalis but lacking an origin of replication for that host behaves as an integration vector as a result of a single homologous recombination event taking place between duplicated chromosomal sequences. To make such a vector useful for purposes of this invention, α-amylase and a phenotypic marker, which will be expressed in E. faecalis, are inserted in the vector. A similar construction has recently been shown to function as an integration vector in B. subtilis (O'Kane et al, 1986).

The erythromycin marker from pVA838 was excised as a HindIII-AvaI fragment and inserted into pUC18 (Yanisch-Perron et al, 1985) cleaved with HindIII and SalI. Transformation into E. coli allows the recovery of clones harboring plasmids consisting of the pUC18 backbone plus the erythromycin resistance marker. These clones are selected on LB agar plates containing 50 µg ampicillin and 80 µg erythromycin per ml. After single colony purification, they are checked for the presence of the desired recombinant plasmid as described above by restriction analysis of minipreparation plasmid DNA (Wilimzig, 1985). One such clone is retained for further work and is named "pG18".

The α-amylase gene is then inserted into pG18. pG18 is restricted with SmaI, and pSTUC (from Example 1) is restricted with PvuII. Restriction digests are phenol extracted and ligated as described above. This ligation mixture is used to transform E. coli which allows the recovery of clones harboring the plasmid named "pG18α". In addition to being erythromycin and ampicillin resistant, these clones display a clear halo on starch plates exposed to iodine vapor.

In the next step, random fragments of the E. faecalis OG1X chromosome are inserted into pG18α. Chromosomal DNA is prepared according to Dale and Greenaway (Dale and Greenaway, 1984) and is digested with XbaI. In parallel, pG18α is cut with XbaI, and the digests are treated with calf intestine alkaline phosphatase ("CIP"). CIP treated, XbaI digested pG18α is then ligated to XbaI chromosomal fragments. The ligation mixture is used to transform E. coli, yielding a set of clones harboring recombinant plasmids, named generally "pG18αφ". These clones are selected on LB plates supplemented with 80 µg erythromycin/ml plus 50 µg ampicillin/ml and 0.2% starch. Halo positive colonies are individually analyzed by restriction analysis of minipreparation plasmid DNA as described above to establish the presence of recombinant plasmids of the expected structure. Among these, a subset of 24 pG18αφ clones with no PstI site in the φ region is retained for the integration experiments described below. Their plasmids are numbered pG18αφ 1 to 24.

Example 22: Integrating the pG18αφ borne α-amylase gene into an E. faecalis chromosome by single homologous recombination

Minipreparation plasmid is prepared from individual cultures of clones harboring plasmids pG18αφ1 to 24 (from Example 21). DNA from each preparation is used as before to transform E. faecalis OG1X protoplasts. In some cases, transformants are obtained which have integrated a given pG18αφ plasmid into their chromosome as a result of a single recombination event between the region of the plasmid and its chromosomal counterpart. These are identified among erythromycin-resistant halo-positive colonies by Southern blotting experiments as described below. PstI digested total DNA is hybridized with ($^{32}$P)-labeled pG18α as a probe. Control experiments are performed in parallel with total DNA from the corresponding pG18αφ transformed E. coli. Each control displays only one band upon hybridization with the probe, corresponding in size to the linear form of the pG18αφ plasmid-harbored by that clone (Fig. 14). Each integrative transformant displays two bands when hybridized with ($^{32}$P)-labeled PG18α. The sizes of those two bands are identical for all transformants of a given homology region, but different among different homology regions. The same band hybridizes with pUC18 as a probe. These results demonstrate that each pG18αφ plasmid has recombined into a different site on the E. faecalis chromosome, depending on the nature of the region present in each clone. Again, the presence of a halo surrounding the colonies on starch plates exposed to iodine vapor indicates that the α-amylase gene is expressed.

Example 23: Constructing a set of double recombination prone integration vectors ("pG18φα") containing the α-amylase gene from B. stearothermphilus (Fig. 15)

A plasmid, bearing a segment of chromosomal DNA from E. faecalis, into which the α-amylase gene has been inserted, and lacking an origin of replication for that host, behaves as an integration vector. In such a case, as opposed to the mode of integration of the pG18αφ vector described in Example 22, a double recombination event can occur, one on each side of the α-amylase gene. In order to allow for the selection of such double recombination prone integrative transformants, a marker gene should be present next to the α-amylase gene inside the chromosomal fragment. Constructions of this kind have been shown to function as integration vectors in B. subtilis (Youngman, 1987).

Random fragments of the E. faecalis chromosome are inserted into the pUC18 vector as follows. Chromosomal DNA from E. faecalis is prepared (Dale and Greenaway, 1984) and digested with NheI. In parallel, pUC18 is digested with XbaI. Restriction digests are phenol extracted and are ligated. Transformation into E. coli allows the recovery of plasmids consisting of the pUC18 backbone plus a fragment of E. faecalis chromosomal DNA. After single colony purification, they are checked for the presence of the desired recombinant plasmid by restriction analysis of minipreparation plasmid DNA (Wilimzig, 1985). The plasmids

17

present in these clones are given the generic name "pUC18φ". Among these plasmids, a subset of 24 pUC18φ plasmids are retained having no Xbal site while comprising at least one site for the following blunt-end restriction enzymes: Ball, EcoRV, Hpal, Nael, Nrul, and Stul (labeled "R" in Fig. 15). These plasmids are numbered pUC18φ 1 to 24.

To introduce the α-amylase gene coupled to the erythromycin resistance marker into the pUC18φ 1 to 24 plasmids, the following steps are taken. Each of the plasmids is cut with its cognate blunt-end restriction enzyme as listed above and ligated to Pvull cut pG18α (from Example 21). Transformation into E. coli yields a set of recombinant plasmids, each consisting of a given pUC18φ backbone plus an insert comprising the α-amylase/erythromycin resistance region of pG18α. The corresponding clones are selected individually on LB plates supplemented with 50 µg ampicillin/ml plus 80 µg erythromycin/ml and 0.2% soluble starch. Halo-positive colonies are analyzed by restriction analysis of minipreparation plasmid DNA to establish the presence of recombinant plasmids of the expected structure. These plasmids are given the generic name "pG18φα" and numbered pG18φα 1 to 24.

Example 24: Integrating the pG18φα-borne α-amylase gene into an E. faecalis chromosome by double homologous recombination

Minipreparation plasmid DNA is prepared as described above from individual cultures of clones harboring plasmids pG18φα 1 to 24 (from Example 23). DNA from each preparation is used as before to transform E. faecalis protoplasts.

Plating on THB plates containing 5µg erythromycin/ml allows, in some cases, the recovery of transformants which have integrated the erythromycin resistance gene and the α-amylase gene into their chromosomes as a result of double recombination events which have occured between each of the φ regions of pG18φα and their chromosomal counterparts. These transformants are erythromycin-resistant and halo-positive and display a specific pattern in Southern blotting experiments as described below. Total DNA is extracted from candidate colonies, is digested with Xbal and is analyzed by Southern blotting using ($^{32}$P)-labeled pG18α as a probe. Control experiments are performed in parallel with total DNA from the corresponding pG18φα transformed E. coli. Each control displays one band upon hybridization with the probe, corresponding in size to the linear form of the pG18φα plasmid harbored by that clone (Fig. 15). The expected E. faecalis integrative transformants display two bands with the probe with band sizes that vary from one transformant to another. The transformants showing two bands with the probe display an identical pattern in Southern blotting experiments using ($^{32}$P)-labeled pVα1 as a probe, but they do not show any hybridizable band with ($^{32}$P)-labeled pUC18 as a probe, unlike the integrative transformants resulting from the single recombination event described above.

When digested with HindIII and analyzed by Southern blotting using pG18α as probe, the total DNA of transformants, showing two bands with the probe, display a single major band on the autoradiogram. This band corresponds in size with the HindIII fragment from pG18α spanning the erm and α regions (See Fig. 15). These results demonstrate that the erythromycin resistance gene plus the α-amylase gene from pG18φα plasmids have recombined by double cross over into different sites of the E. faecalis chromosome, depending on the nature of the region present in a given clone. Again, the halo in the presence of iodine vapor indicates that the α-amylase gene is expressed.

Example 25: Evaluation of the performance of transformed L. plantarum 80 and E. faecalis OG1X in grass silage

Comparisons have been made between wild type and transformed L. plantarum 80 and E. faecalis OG1X strains as to the ability to extract fermentable sugars from a variety of commonly used silage materials and silage additives and the rate of acidification in a number of liquid media.

25.1. Production of reducing sugars

The ability to convert poly- or oligosaccharides into lower molecular weight mono-or oligosaccharides upon incubation was measured as an increase in reducing sugar content. The Somogyi-Nelson method (Nelson, 1952) is the most reliable for this purpose (Breuil and Saddler, 1985). A panel of strains of lactic acid bacteria was incubated with a collection of test substrates and additives commonly used in silage.

The strains consisted of:
L. plantarum 80 (hereinafter also referred to as Lp 80)
L. plantarum 80 containing pRAM11 (Example 7) mixed in a 1:1 ratio with L. plantarum containing pST11 (Example 7),
LpGA6.6 (Example 10)
E. faecalis OG1X (hereinafter also referred to as EfOG1X)
E. faecalis OG1X with plasmid pVAα (Example 17) A blanco (water) was included

The substrates were:
- soluble starch 3% (DIFCO solubilized by boiling and dialyzed).
- carboxy methyl cellulose (CMC) low viscosity 1% (solubilized by boiling and dialyzed).
- corn starch 3% in dialysis buffer and dialyzed.
- milled corn grains 3 % in dialysis buffer.

- blanco: dialysis buffer

The substrates were dialyzed (cutoff 12,000 dalton) overnight for the first time then twice dialyzed for 2 hours against 5 mM $CaCl_2$, 50 mM Na-Succinate buffer pH 5.8 at 4°C. This removes most of the indigeneous low molecular weight carbohydrates in e.g. corn starch.

The strains were cultured overnight in MRS (for Lp80) or TH (for E. faecalis). For transformed strain, 10 μg/ml erythromycin and 100 μg/ml lincomycin were added.

2 ml of Lp80 cultures and 4 ml of E. faecalis cultures were inoculated in 100 ml MRS from which the normal glucose constituent was omitted. Again erythromycin and lincomycin were added for the transformed strain. The bacteria were grown for 15 h at 37°C. The OD and pH of each culture was determined. The OD's of all cultures were equalized to an $OD_{600}$ = 0.3. while the pH was brought to 5.8. To kill off the bacteria each culture was incubated for 15 minutes with shaking after addition of 0.1 ml chloroform per 100 ml. Killing the bacteria excludes the consumption of carbohydrates by fermentation. The cultures and substrates were preheated to 60°C and seven ml of each culture was mixed with 10 ml of each substrate. From each mixture a 4 ml sample was frozen at -20°C (this constitutes time = 0). Two more 4 ml samples were incubated at 60°C. For each mixture one of these 4 ml samples was frozen (-20°C) after 3 hours and the other one after 38 hours of incubation.

For analysis the frozen samples were thawed and subsequently centrifuged for 2 minutes in an Eppendorf centrifuge. Reducing sugars were determined on the supernatans by the Somogyi-Nelson method (Nelson, 1952).

The measurement of the OD was roughly linear to the concentration of glucose in a series of controls and 1 OD equalled approximately 0.14 g/l.

The results are presented in Table 1. The figures given in Table 1 are the net increases in glucose equivalents in the medium (in g/l) after 38 hours of incubation.

Table 1

|  | soluble starch | CMC | Corn starch | Corn | Blanco |
|---|---|---|---|---|---|
| Lp80 | 0.016 | 0.003 | 0.001 | 0 | 0 |
| Lp80 pRAM11 . Lp80 pST11 | 1.472 | 0.161 | 0.252 | 0.784 | 0 |
| LpGA6.6 | 0.375 | 0.265 | 0.033 | 0.250 | 0 |
| EfOG1X | 0.196 | 0.012 | 0 | 0.083 | 0 |
| EfOG1X pVAα | 0.452 | 0.009 | 0.001 | 0.177 | 0 |
| Blanco | 0.123 | 0.001 | 0 | 0.107 | 0 |

Table 1 shows that the mixture of Lp80 transformed with pRAM11 and Lp80 transformed with pST11 and LpGA 6.6 performed better than the controls for all subtrates while EfOG1X transformed with pVAα performed better than its control for soluble starch and milled corn grains.

25.2. Rate of acidification
The following strains:
- L. plantarum 80 (Lp80)
- LpGl4 (Example 9)
- Lp80 pRAM11 (Example 7)
- Lp80 pST11 (Example 7)
- a mixture of LpGA3.2, 3.1, 6.3, and 6.4 (1:1:1:1) (Example 10)
were grown in MRS, inoculated at a 2% concentration in MRS without glucose, and grown overnight at 37°C. The pH and the $O.D._{600}$ of the cultures were uniformized to approx. pH = 6.0 and $OD_{600}$ = 0.6 ($10^8$ cells/ml).
In a 100 ml bottle were mixed:
- 10 ml of MRS without glucose
- 1 ml of one of the above strains
- 1 mg erythromycin for all strains except L. plantarum 80
- 5 mg kanamycin
- 2 grams of one substrate from the list below
- sterile water up to 100 ml.
The final concentration of the cells was $10^6$ cells per ml.
The following substrate solutions were used:
- soluble starch: see 24.1
- milled corn: see 24.1
- CMC: see 24.1
- dextrose (positive control)
- no additives as blanco (negative control)

The substrates were not sterilized to avoid modification of the physicochemical structure. However, the probability of secondary infection was reduced by the addition of kanamycin which does not affect Lp80 and erythromycin (10 µg/ml) which does not affect the transformed strains.

The 100 ml bottles as prepared above were incubated at 37°C with shaking. The pH was measured after 0, 2 and 5 days.

The results are presented in Table 2.

Table 2

|  | starch | corn | CMC | dextr | H$_2$O |
|---|---|---|---|---|---|
| Lp80 | 6.09 | 4.98 | 6.42 | 6.25 | 5.86 |
|  | 5.92 | 5.50 | 6.41 | 4.18 | 5.62 |
|  | 4.95 | 5.00 | 6.10 | 3.36 | 5.86 |
| LpGI | 6.08 | 4.87 | 6.37 | 4.20 | 5.70 |
|  | 6.01 | 4.85 | 6.48 | 3.83 | 5.70 |
|  | 5.70 | 4.89 | 6.46 | 3.35 | 5.70 |
| pRAM11 | 6.12 | 4.10 | 6.32 | 6.18 | 5.98 |
|  | 5.63 | 3.68 | 6.30 | 3.12 | 5.30 |
|  | 4.29 | 3.42 | 6.22 | 3.61 | 5.30 |
| pST11 | 6.04 | 4.88 | 6.23 | 6.30 | 5.72 |
|  | 5.89 | 4.85 | 6.10 | 5.12 | 5.60 |
|  | 5.92 | 4.90 | 5.60 | 3.42 | 5.93 |
| LpGA 3.1-4 | 6.10 | 4.88 | 5.91 | 6.22 | 5.14 |
|  | 6.06 | 4.71 | 5.29 | 3.92 | 5.07 |
|  | 4.20 | 3.75 | 5.39 | 3.55 | 5.30 |

Table 2 shows that pRAM11 and the LPGA mixture induced a more pronounced pH decrease than the other strains on soluble starch and milled corn. Table 2 also shows that pST11 and the LpGA mixture induced a more pronounced pH decrease than the other strains on CMC. Some variability of the results is probably due to growth of contaminating bacteria.

Example 26: Stability and competitive abilities of engineered Lactobacillus plantarum strains in various conditions

26.1. To test the stabilities of the engineered Lactobacillus plantarum 80 in the absence of selective pressures on the foreign DNA fragments, bacteria were fermented overnight in MRSE (MRS supplemented with 10 µg/ml erythromycin), pelleted and resuspended in the original volume of MRS. 10 µl of this suspension was used to inoculate 10 ml MRS, and was grown to saturation (overnight, 37°C). 10 µl of this DAY 1 culture was used to inoculate 10 ml MRS and subsequently cultured overnight to obtain a DAY 2 culture. The above procedure was repeated once more to obtain a DAY 3 culture. Aliquots of DAY 1, DAY2 and DAY 3 cultures were taken and the number of viable cell was determined by plating sequential dilutions on MRS and MRSE agar.

The number of colony forming units (CFU) of transformed Lp80 on both MRS and MRSE after 1, 2, and 3 days (respectively 13, 26 and 39 generations) are presented in Table 3. The numbers in Table 3 are in 10$^7$ CFU per ml. A comparison of the figures in Table 3 for MRS and MRSE shows the stability of the fermented transformed strains under non-selective conditions.

Table 3

Segregation of engineered DNA, monitored as erythromycin resistance, upon non-selective growth of transformed L. plantarum 80

|  | DAG1 | | DAG 2 | | DAG 3 | |
|---|---|---|---|---|---|---|
|  | MRS | MRSE | MRS | MRSE | MRS | MRSE |
| pST11 | 152 | 26 | 242 | 10 | 232 | - |
| pRAM11 | 34 | 26 | 198 | 2 | 186 | - |
| 1PGA3.1 | 142 | 115 | 236 | 236 | 260 | 179 |
| 1PGA3.2 | 152 | 179 | 192 | 284 | 305 | 186 |
| 1PGA3.3 | 206 | 181 | 209 | 234 | 176 | 130 |
| 1PGA3.4 | 162 | 153 | 256 | 315 | 183 | 161 |
| 1PGA3.6 | 239 | 186 | 238 | 278 | 185 | 156 |
| 1PGA3.9 | 184 | 167 | 264 | 276 | 227 | 216 |

Table 3 (continued

|  | DAG1 | | DAG 2 | | DAG 3 | |
|---|---|---|---|---|---|---|
|  | MRS | MRSE | MRS | MRSE | MRS | MRSE |
| 10GA6.1 | 119 | 128 | 191 | 226 | 127 | 80 |
| 1PGA6.2 | 83 | 60 | 273 | 47 | 186 | 8 |
| 1PGA6.3 | 84 | 94 | 249 | 231 | 132 | 151 |
| 1PGA6.4 | 195 | 139 | 209 | 291 | 162 | 129 |
| 1PGA6.5 | 180 | 149 | 271 | 242 | 182 | 169 |
| 1PGA6.6 | 103 | 121 | 210 | 182 | 177 | 46 |
| 1PGA6.7 | 155 | 129 | 289 | 260 | 149 | 114 |
| 1PGA6.8 | 158 | 131 | 287 | 228 | 213 | 136 |
| 1PGA6.9 | 137 | 120 | 221 | 206 | 127 | 162 |
| 1PGA6.11 | 124 | 98 | 240 | 260 | 143 | 98 |

It is clear that under these non-selective conditions the erythomycin resistance, when contained on plasmids (see also Example 6), is rapidly lost, while the same characteristic is very stable when present in the bacteria as a chromosomal insert. Only in 1 case (LpGA 6.2) the resistance is lost after 3 days possibly caused by the presence of the erythromycin resistance gene on an indigenous plasmid of Lp80 (see Example 10).

26.2. Experiments to test the abilities of the engineered strains to compete with the wild type strains were performed as follows.

About 250 g of fresh grass was autoclaved and inoculated with $10^8$ cells of wild type L. plantarum and $10^7$ cells of LpGI2 (or analogously LPGI4 or LpGI5) and stored under conditions mimicking silage (anaerobic, 28°C). Cells are taken from overnight cultures (37°C). After respectively 1, 2, 3, 4, 5 and 11 days, 25g of grass from each culture was suspended in 225 ml 0.1 % proteose pepton, serially diluted to a factor of $10^{-6}$ and plated on both MRS and MRSE. The ratio of the number of CFU's observed on MRSE to the number observed on MRS is indicative of the competitive abilities of the strain containing the erythromycin resistance on a chromosomal insert as compared to the wild type strain.

The results are presented in Table 4. The numbers in Table 4 refer to $10^6$ CFU per gram of ensiled grass. It is obvious from Table 4 that LPGI2 and LPGI4 have the same growth rate and survive equally well in grass silage as wild type L. plantarum 80. As in liquid culture (Example 9), LpGI5 has a lower growth rate although its survival is comparable to wild type L. plantarum 80. This is probably due to the fact that insertion of the Em gene occurred in a vital place of the bacterial chromosome, proving the importance of an adequate choice of a chromosomal segment as a recombination target.

Table 4

Comparison between wild-type and transformed
L. plantarum 80 as to growth rate and subsequent
survival in ensiled grass

Lp80 + LpG12 (1:9)

| Day | MRS | MRSE | Ratio MRS/ MRSE |
|---|---|---|---|
| 1 | 288 | 45 | 1/6 |
| 2 | 313 | 78 | 1/4 |
| 3 | 220 | 56 | 1/4 |
| 4 | 65 | 19 | 1/3 |
| 5 | 85 | 15 | 1/6 |
| 11 | 3 | 1 | 1/3 |

Lp80 + LpG14 (1:9)

| Day | MRS | MRSE | Ratio MRS/ MRSE |
|---|---|---|---|
| 1 | 136 | 40 | 1/3 |
| 2 | 800 | 86 | 1/9 |
| 3 | 275 | 44 | 1/6 |
| 4 | 274 | 29 | 1/9 |
| 5 | 53 | 14 | 1/4 |
| 11 | 7 | 1 | 1/7 |

Lp80 + LpG15 (1:9)

| Day | MRS | MRSE | Ratio MRS/ MRSE |
|---|---|---|---|
| 1 | 326 | 4 | 1/81 |
| 2 | 132 | 2 | 1/66 |
| 3 | 245 | 3 | 1/61 |
| 4 | 28 | 0.1 | 1/282 |
| 5 | 36 | 0.6 | 1/61 |
| 11 | 5 | .02 | 1/250 |

In an analogous experiment, insertion of Lp80 with respectively LPGA6.3, LPGA6.6 and LPGA1 3.9 (all strains containing the amylase- endoglucanase-erythromycin resistance cassette as a chromosomal insert), in a ratio of 1:9, were fermented in 10 ml MRS during 1 day.

For each culture a sample of 100 $\mu$l was taken, serially diluted to a factor $10^{-5}$ and plated on both MRS and MRSE. Colonies were counted as in the above. The results are presented in Table 5. Numbers in Table 5 refer to $10^6$ CFU per ml.

Table 5

Comparison between wild-type and transformed
L. plantarum 80 as to growth rate

|  | MRS | MRSE | Ratio MRS/ MRSE |
|---|---|---|---|
| LPGA 6.3 | 1078 | 277 | 1/4 |
| LPGA 6.6 | 1700 | 280 | 1/6 |
| LPGA 3.9 | 1480 | 110 | 1/13 |

Table 5 shows that LpGA 3.9, 6.3 and 6.6 have growth rates comparable to L. plantarum 80 as was expected since strain LpGl4 has the same growth rate as wild type L.plantarum 80. Again the engineered strains perform very well.

26.3. 220 g of non-sterile fresh grass was inoculated with respectively Lp80, LpGl4, LpGA 6.6. In each case $10^{-6}$ bacteria per gram of grass were added. After respectively 1, 4 and 7 days two samples of 25 g of grass were suspended in 225 ml in MRS, serially diluted and plated on both Rogosa medium (R) (Oxoid Manual, 1982) and Rogosa supplemented with 10 µg/ml erythromycin (RE). The number of colonies were counted on both plates and converted to the number of bacteria per gram of grass. The results are presented in Table 6. Numbers in Table 6 are in $10^6$ bacteria per gram of grass.

The figures for days 4 and 8 are the average of two samples.

Table 6

| No inoculum | | | L. plantarum 80 | | |
|---|---|---|---|---|---|
| day | R | RE | day | R | RE |
| 0 | 0.0006 | - | 0 | (1) | - |
| 1 | 10 | - | 1 | 180 | - |
| 4 | 89 | - | 4 | 380 | - |
| 8 | 130 | - | 8 | 165 | - |
| LpG14 | | | LpGA 6.6 | | |
| day | R | RE | day | R | RE |
| 0 | (1) | - | 0 | 0 | (1) |
| 1 | 76 | 56 | 1 | 110 | 100 |
| 4 | 355 | 266 | 4 | 280 | 140 |
| 8 | 160 | 100 | 8 | 200 | 107 |

It is clear from Table 6 that the engineered strains constitute a considerable fraction (more than 50%) of the total population of lactobacilli, and that inoculation with transformed strains does not alter the proliferation profile of lactic acid bacteria. This proves that the Lp80 engineered to contain foreign genes as a chromosomal insert remains usable as a silage starter organism.

It will readily be appreciated that the invention thus provides a breakthrough in the art of genetically engineered, lactic acid bacteria useful as inocula in silage and as probiotics. It also goes without saying that this invention is not limited to the above examples. In this regard, other lactic acid bacteria, transformed in accordance with this invention, can be used as inocula in silage and as probiotics.

Also, DNA sequences other than those shown in Figs. 2 and 3 for the novel amylase gene and plasmid, respectively, of this invention can be used for transforming lactic acid bacteria. In this regard, such DNA sequences can be modified: 1) by replacing some codons with others that code either for the same amino acids or for other amino acids; and/or 2) by deleting or adding some codons; provided that such modifications do not substantially alter the properties of such DNA sequences. Furthermore, other DNA recombinants containing the aforementioned DNA sequences in association with other foreign DNA, particularly the DNA of vectors suitable for transforming microorganisms other than lactic acid bacteria, such as E. coli, B. subtilis and even eucaryotic organisms (e.g., yeasts), are encompassed by this invention.

Furthermore, other exogenous DNAs encoding enzymes can be used for transforming lactic acid bacteria in accordance with this invention. In this regard, exogenous DNAs encoding other proteins, which promote the degradation of polysaccharides and/or oligosaccharides to fermentable carbohydrates, can be used.

There follows a list of references which have been referred to above in describing the invention and its examples with regard to known methods for achieving some of the process steps referred to herein and with regard to general knowledge which has formed the basis for this invention.

REFERENCES

Aiba S., Kitai, K., and Imanaka, T. : Cloning and expression of thermostable α-amylase gene from Bacillus stearothermophilus in Bacillus stearothermophilus and Bacillus subtilis. Appl. Envir. Microbiol. 46, 1059 (1983).

Alonso, J., and Tailor, H. : Initiation of plasmid pC194 replication and its control in Bacillus subtilis. Mol. Gen. Genet. 210, 476-484 (1987).

Anderson, D.G. and Mc Kay, L.L. : Simple and rapid method for isolating large plasmid DNA from lactic streptococci. Appl. Envir. Microbiol., 46 , p. 549 (1983).

Aumaitre : Additifs alimentaires et efficacité zootechnique chez le porc Biofutur 69, 91-93 (1988) Baecker P.A., C.E. Furlong & J. Preiss: Biosynthesis of bacterial glycogen. J. Biol. Chem. 258, 5084 (1983).

Banai, B., and Leblanc, D.J. : Genetic, molecular, and functional analysis of Streptococcus faecalis R plasmid pJH1. J. Bacteriol. 155, 1094 (1983).

Beck, Th. : The Microbiology of Silage Fermentation. In : Fermentation of silage - A review, (M.E. Mc. Cullough, ed.), National Feed Ingredients Assoc., Iowa, 63-102 (1978).

Beguin, P., Cornet, P., Aubert, J.P. : Sequence of a cellulase gene of the thermophilic bacterium Clostridium thermocellum. J. Bacteriol. 162, 102-105 (1985).

Bernard, H.U., Remaut, E., Hershfield, M.V., Das, H.K., Helinski, D.R., Yanofsky, C., and Franklin, N. : Construction of plasmid cloning vehicles that promote gene expression from the bacteriophage lambda pL promotor. Gene 5, 59 (1979).

Bolivar, F., and Backmann, K. : Plasmids of Escherichia coli as cloning vectors. Methods Enzymol. 68, 245 (1979).

Bolson, K.K. : Silage and silage additives in the U.S.A. In : Silage New Biological Aspects, 153-158, ed. Sanofi Animal Health, Paris (1985).

Botterman, J. : Ph. D. Thesis, State University of Ghent, (1986).

Botterman, J., and Zabeau, M. : A standarized vector system for manipulation and enhanced expression of genes in Escherichia coli. DNA, 6, 583 (1987).

Breuil C. and J.N.Saddler : Comparison of the 3,5-dinitrosalicylic acid and Nelson-SDomogyi methods of assaying for reducing sugars and determining cellulase activity. Enzyme and Microb.Techn. 7:327 (1985).

Broz. J. Effects of supplemented enzymes on the nutritional value of various cereals for growing chicks, Int. J. Vitamin Nutrition Res. 57, 346 (1987).

Casadaban, M., and Cohen, S.N. : Analysis of gene control signals by DNA fusion and cloning in Escherichia coli. J. Mol. Biol. 138, 179-207 (1980).

Chassy and Flickinger : Transformation of Lactobacillus casei by electroporation FEMS. Microbiol. Letts. 44, 173-177 (1987).

Chesson, A. : Supplementary enzymes to improve the utilization of pig and poultry diets in : HARESIGN, W. and COLE, D.J.A. (eds) Recent Advances in animal nutrition, Butterworths, London UK, pp 71-89 (1987).

Clewell. D. Department of Oral Biology and Microbiology / Immunology School of Dentistry and Medicine and the Dental Research Institute, The University of Michigan, Ann Harbor, Michigan USA).

Cornet. P., Millet, J., Beguin, P., Aubert, J.P. : Characterization of two cel (cellulose degradation) genes of Clostridium thermocellum coding for endoglucanases. Bio/Technology 1, 589-594 (1983A).

Cornet. P., Tronik, D., Millet, J., and Aubert, J.P. : Cloning and expression in Escherichia coli of Clostridium thermocellum genes coding for amino acid synthesis and cellulose hydrolysis. FEMS Microbiol. Lett. 16, 137-141 (1983B).

Coughlan M.P. : The properties of fungal and bacterial cellulases with comment on their production and application. Biotechn. Genet. Eng. Rev. 3, 39-109 (1985).

D'Haese, P., Hussey, C., Van Montagu, M. : Thermo-inducible gene expression in Bacillus subtilis using transcriptional regulatory elements from temperate phage phi105, GENE, 32, 181-194 (1984).

Dale. J.W. and Greenaway, P.J. : Preparation of chromosomal DNA from E coli. In : Methods in molecular biology, Vol. 2 Nucleic Acids, J.M. Walker (ed.), Humana, Clifton, N.J. USA. (1984).

Dao. L.M., and Feretti, J.J. : Streptococcus-Escherichia coli shuttle vector pSA3 and its use in the cloning of streptococcal genes. Appl. Envir. Microbiol. 49, 115-119 (1985).

Fengler, A.I. and Marquardt , R.R. : An antinutritional factor in rye : Isolation and activity of the water-soluble pentosans. Can. J. Anim. Sci. 66, 330 (1986).

Fernandes C.F., Shahani K.M. and Amer M.A.: Therapeutic role of dietary lactobacilli and lactobaccillic fermented dairy products. FEMS Microbiol. Rev. 46, 343-356 (1987).

Gray. G.L., Mainzer, S.E., Rey, M.W., Lamsa, M.H., Kindle, K.L., Carmona, C. and Requadt, C. Structural genes encoding the thermophilic α-amylases of Bacillus Stearothermophilus and Bacillus licheniformis. J. Bact. 166, 635-643 (1986).

Gennaro, M., Kornblum, J., Novick, R.P. A site-specific recombination function in Staphylococcus aureus plasmids. J. Bacteriol. 169, 2601-2610 (1987).

Gilliland, S.E. : Probiotics, fact or fancy. Speech at the 8 the International Biotechnology Symposium, Paris July 1988.

Gryczan T.J., Shivakumar A.G., Dubnau D. : Characterization of chimeric plasmid cloning vehicles in Bacillus subtilis. J. Bacteriol. 141, 246-253 (1980).

Hazlewood, G.P. et al. : A catalogue of Clostridium thermocellum endoglucanase, B-glucosidase, and xylanase genes cloned in Escherichia coli. FEMS Microbiol. Lett. 51, 231-236 (1988).

Hintermann, G., Fischer, H-M., Crameri, R., and Htter, R. : Simple Procedure for distinguishing CCC, OC, and L forms of plasmid DNA by agarose electrophoresis. Plasmid, 5, 371 (1981).

Horinouchi, S. and Weisblum, B. : Nucleotide sequence and functional map of pE194, a plasmid that specifies inducible resistance to macrolide, lincosamide, and streptogramin type B antibiotics. J. Bacteriol. 150, 804 (1982A).

Horinouchi, S. and Weisblum, B. Nucleotide sequence and functional map of pC194, a plasmid that specifies inducible chloramphenicol resistance. J. Bacteriol. 150, 851 (1982B).

Hoshino, T., Ikeda, T., Tomizuka, N., and Furukawa, K. : Nucleotide sequence of the tetracycline resistance gene of pTHT15, a thermophilic Bacillus plasmid comparison with staphylococcal TcR controls. GENE 37, 131 (1985).

Ike, Y., Craig, R.A., White, B.A., Yagi, Y. and Clewell, D. : Modification of Streptococcus faecalis sex pheromones after acquisition of plasmid DNA. Proc. Natl. Acad. Sci. USA 80, 5369-5373 (1983).

Ish-Horowicz D. and J.F.Burke, Rapid and efficient plasmid cloning. Nucleic Acids Research 9, 2989-2998 (1981).

Joyet, P., Guerineau, M. and Heslot, H. : Cloning of a thermostable α-amylase gene from Bacillus licheniformis and its expression in Escherichia coli and Bacillus subtilis. FEMS Microbiology letters 21, 353-358 (1984).

Kawazu, T., Nakanishi, Y., Uozumi, N., Sasak, T., Yamagati, H., Tsukagoshi, N., Udaka, S. : Cloning and nucleotide sequence of the gene coding for enzymatically active fragments of the Bacillus polymyxa β-amylase. J. Bacteriol. 169, 1564 (1987).

Khan, S.A., and Novick, R.P. : Complete nucleotide sequence of pT181, a tetracycline-resistance plasmid from Staphylococcus aureus. Plasmid 10, 251 (1983).

Klaenhammer, T.R. : Microbiological considerations in selection and preparation of Lactobacillus strains for use as dietary adjuncts. J. Dairy Sci. 65, 1339-1349 (1982).

Knowles, S., Lehtovaara, P., and Teeri, T. : Cellulase families and their genes, TIBTECH 5, 255-261 (1987).

Kok, J., Van Dijl, J.M., Van Der Vossen, J.M.B.M., Hugenholtz, J., and Venema, G. : Cloning and expression of a Streptococcus cremoris protein in Bacillus subtilis and Streptococcus lactis. Appl. Envir. Microbiol. 50, 94 (1985).

Lambert B., F.Leyns, L.Van Rooyen, F.Gosselé, Y.Rapon & J.Swings, Rhizobacteria of maize and their antifungal activities. Appl.Envir.Microbiol. 53, 1866-1871 (1987)

Lyons, T.P. : The role of biological tools in the feed industry. In : LYONS, T.P. (ed) Biotechnology in the feed industry, Alltech Technical Publications, Nicholasville, Kentucky, USA, pp. 1-49 (1987).

Macrina, F.L., Tobian, J.A., Jones, K.R., Evans, R.P., Clewell, D.B. : A cloning vector able to replicate in E. coli and Streptococcus sanguis. GENE 19, 345 (1982).

Maniatis, T., Fritsch, E.F., and Sambrook, J. : Molecular cloning, a laboratory manual. Cold Spring Harbor Laboratory, New York (1982).

Marquardt, P.R., Fengler, A.I. and Pawlik, J. ; Improvement of the nutritional value of rye and wheat grains through the use of crude enzymes of microbial origin. In LYONS, T.P. ed) Biotechnology in the feed industry, Alltech Technical Publications, Nicholasville, Kentucky, USA, pp. 241-250 (1987).

Martin, B., Alloing, G., Mejean, V., Claverys, J-P. : Constitutive expression of erythromycin resistance mediated by the ermAM determinant plasmid pAMB1 results from deletion of 5′ leader peptide sequences. Plasmid 18, 250-253 (1987).

Maxam, A.M., and Gilbert, W. : Sequencing end-labeled DNA with base-specific cleavages. Methods Enzymol. 65, 499 (1980).

McDonald, P. : The Biochemistry of silage. Ed. : John Wiley & Sons, Chichester-New York-Brisbane-Toronto (1981).

McKenzie, T., Hoshino, T., Tanaka, T., and Sueoka, N. : A revision of the nucleotide sequence and functional map of pUB110. Plasmid 17, nr. 1 (1987).

Messing, J., Crea, R. and Seeburg, P.H. : A system for shotgun DNA sequencing. Nucleic Acid Res. 9, 309-321 (1981).

Messing, J. : New M13 vectors for cloning. Methods enzymol 101, 20 (1983).

Montelarote R.C., Lohrey N., Parekh B, Blakeney E.W. and Essel C.J. : Isolation and comparative biochemical properties of the major internal polypeptides of equine infections anemia virus. J. Virol. 42, 1029-1038 (1982).

Murai M., Miyashiba H., Araki H., Seki T. ad Oshima Y. : Molecular structure of replication origin of Bacillus amybliquefaciens plasmid pFTB14. Mol. Gen. Genet. 210, 92-100 (1987).

Murray, N.E., Brammar, W.J., and Murray, K. : Lamboid phages that simplify the recovery of in vitro recombinants. Mol. Gen. Genet. 150, 53 (1977).

Nakajima, R., Imanaka, T., Aiba, S. : Nucleotide sequence of the Bacillus stearothermophilus α-amylase gene. J. Bacteriol. 163, 401 (1985).

Nakajima, R., Imanaka, T., and Aiba, S. : Comparison of amino acid sequences of eleven different α-amylases. Appl. Microbiol. Biotech. 23, 355 (1986).

Nelson, N. : A photometric adaptation of the Somogyi method for the determination of glucose. J. Biol. Chem. 153, 376-380 (1952).

Nishizawa, M., Ozawa, F. Hishinuma, F. : Molecular cloning of an amylase gene from Bacillus circulans. DNA, 6, 255 (1987).

Noirot, Ph., Petit, M.A., Ehrlich, S.D. : Plasmid replication stimulates DNA recombination in Bacillus subtilis. J. Mol. Biol. 196, 39-48 (1987).

O'Kane, C., Stephens, M.A. and McConnell, D. : Integrable α-amylase plasmid for generating random transcriptional fusions in Bacillus subtilus. J. Bacteriol. 168, 973-981 (1986).

Ortlepp, S.A., Ollington, J.F. and McConnell, D.J. : Molecular cloning in Bacillus subtilis of a Bacillus licheniformis gene encoding a thermostable α-amylase. Gene 23, 267-276 (1983).

Oxoid Manual, 5th Ed (1982), Oxoid Ltd, Basingstoke, Hampshire, England.

Piggott, R.P., Rossiter, A., Ortlepp, S.A., Pembroke, J.T. and Ollington, J.F. : Cloning in Bacillus subtilis of an extremely thermostabele α-amylase comparison with other cloned heat stable α-amylase. Biochem. Biophys. Res. Commun. 122, 175-183 (1984).

Platteeuw, C. : Licenciate's thesis at the State University of Ghent (Belgium) (1986).

Polaina, J., Perez-Martinez, G., Gonzalez-Candelas, L., Flors, A. : High expression of an endoglucanase gene from Clostridium cellulolyticum in Escherichia coli. In : Biochemistry and genetics of cellulose degradation (Aubert J, P. Beguin & J.millet, eds) Abstract book (1987).

Raibaud P. and Ducluzeau, R. : Les bacteries du tube digestif. La recherche, 151, 12-21 (1984).

Schleifer, K.H., and Kilpper-Bälz, R. : Molecular and chematoxonomic approaches to the classification of Streptococci, Enterococci and Lactococci : A review. System. Appl. Microbiol. 10, 1-19 (1987).

Schwarz, W.H., Grabnitz, F., Staudenbauer, W.L. : Properties of a Clostridium thermocellum endoglucanase produced in Escherichia coli. Appl. Envir. Microbiol. 51, 1293-1299 (1986).

Seale, D.R. : Bacteria and enzymes as products to improve silage preservation. In : Developments in silage. Ed. : J.M. Wilkinson and Bastark, Chalcombe publications, 47-62 (1987).

Seale, D.R. : Bacterial inoculants as silage additives. J. Appl. Bacteriol. Symp. Suppl., 9S - 26 S (1986).

Shaw, J.H., and Clewell, D.B. : Complet nucleotide sequence of macrolide-lincosamide-streptogramin B resistance transposon Tn917 in Streptococcus faecalis. J. Bacteriol. 164, 782 (1985).

Sibakov, M. and Palva, I. : Isolation and the 5′-end nucleotide sequence of Bacillus licheniformis α-amylase gene. Eur. J. Biochem 145, 567-572 (1984).

Simons G., and De Vos, W.M. : Gene expression in lactic Streptococci. In : Proc. 4th European Congress on Biotechnology, Vol. 1 ed. by O.M. Neyssel, R.R. Van Der Mee and K.C.A.M. Luyben. Elsevier Science Publishers B.V. Amsterdam (1987).

Sneath, P.H.A., Mair, N.S. and Sharpe, M.E. (1986) Bergey's Manual of Systematic Bacteriology, Vol. 2. Williams and Wilkins, Baltimore, MD.

Southern, E.M. : Detection of specific sequences among DNA fragments separated by gel electorphoresis. J. Mol. Biol. 98, 503-517 (1975).

Stephans, M.A., Ortlepp, S.A., Ollington, J.F. and McConnel, D.J. : Nucleotide sequence of the 5′region of the Bacillus licheniformis α-amylase gene comparison with the amyloliquefaciens gene ( 1984).

Ter Riele, H., Michel, B., Ehrlich, S.D. : Single-stranded plasmid DNA in Bacillus subtilis and Staphylococcus aureus. Proc. Natl. Acad. Sci. USA 83, 2541-2545 (1986).

Van Der Vossen, J.M.B.M., Van Der Lelie D., and Venema, G. : Isolation and characterization of Streptococcus cremorus Wg2 - specific promotors. Appl. Enviromental Microbiol. 53, 2452-2457 (1987).

Vrignaud, Y. : Les ferments lactiques dans les industries alimentaires. Importance dans la flore intestinale. Supplémentation des aliments d'allaitement en culture de ferments lactiques. Les ferments lactiques chez l'homme. Industries alimentaires et agricole, 147-160 (1982).

Weaver, K.E. and Clewell, D.B. : Transposon Tn 917 vectors for mutagenesis in Streptococcus faecalis. In : FERRETTI, J.J. and CURTIS, ROY III (eds) Streptococcal Genetics, American Society for Microbiology, Washington DC, USA, pp. 17-21 (1987).

Wilimzig, M. : LiCl boiling method for plasmid mini-preps. Trends in Genetics 1, 158 (1985).

Wirth, R., An, F.Y. and Clewell, D. : Highly efficient protoplast transformation system for Streptococcus faecalis and a new Escherichia coli - S. faecalis shuttle vector. J. Bacteriol. 165, 831-836 (1986).

Wolter, R. and Henry, N. Les probiotiques en alimentation animale. Rec. Med. Vet. 158, 283-290 (1982).

Wong, W.K.R., Curry, C., Parekh, R.S., Parekh, S.R., Wayman, M., Davies, R.M., Kilburn, D.G., and Skippes, N. : Wood hydrolysis by Celulomonas fimi endoglucanase and exoglucanase coexpressed as secreted enzymes in Saccharomyces cerevisiae.

Woolford M.K., and Wilkins, R.J. : Preliminary Experiments with Simulated Silage. J. Sci. Fd. Agric., 26, p. 141-148 (1974). Biotechnology, 6, 713-719.

Woolford, M.K., and Sawczyc, M.K. : An investigation into the effect of lactic acid bacteria on fermentation in silage. 1. Strain selection. Grass and Forage science 39, 39-148 (1984).

Woolford, M.K. The Silage Fermentation. In : Microbiology Series 14, Marcel Dekker, Inc., New York and Basel (1984).

Wu, J.H.D., Armo-Johnson, W.H., and Demain, A.L. : Two components of an extracellulase protein aggregate of Clostridium thermocellum together degrade crystalline cellulase. Biochemistry 27, 1703-1709 (1988).

Yanisch-Perron, C., Vieira, J., and Messing, J. : Improved M13 phage cloning vectors and host strains : nucleotide sequence of the M13mp18 and pUC19 vectors. GENE 33, 103 (1985).

Youngman, P. : Plasmid vectors for recovering and exploiting Tn917 transpositions in Bacillus and other Gram-positives. In : Plasmids, a practical approach. Ed. : K. Hardy, IRL press, OXFORD (1987).

Yuuki, T., Nomura, T., Tezuka, H., Tsuboi, A., Yamagata, H., Tsukagoshi, N. and Udaka, S. : Complete nucleotide sequence of a a gene coding for heat - and pH - stable α-amylase from <u>Bacillus licheniformis</u> comparison of the amino acid sequences of three bacterial liquefying α-amylases dedeuced from the DNA sequences. J. Biochem <u>98</u>, 1147-1156 (1985).

Zabeau, M., and Stanley, <u>K</u>. : Enhanced expression of cro-B-galactosidase fusion proteins under control of the $P_R$ promotor of bacteriophage lambda. EMBO J. <u>1</u>, 1217 (1982).

Zukowski, M.M. Gaffney, D.F., Speck, D., Kauffmann, M., Findell, A., Wisecup, A.Z., and Lecocq, J.P. : Chromogenic identification of genetic regulatory signals in <u>Bacillus subtilis</u> based on expression of a cloned <u>Pseudomonas</u> gene. Proc. Natl. Acad. Sci. USA <u>80</u>, 1101-1105 (1983).

**Claims**

1. An inoculum for silage characterized by lactic acid bacteria, preferably a <u>Lactococcus</u>, <u>Enterococcus</u>, <u>Lactobacillus</u>, <u>Leuconostoc</u> or <u>Pediococcus</u>, particularly <u>Lactobacillus plantarum</u> and <u>E. faecalis</u>, transformed with an exogenous DNA which codes for an enzyme that breaks down an oligosaccharide and/or a polysaccharide into a fermentable carbohydrate.

2. The inoculum of claim 1, in which the exogenous DNA codes for an amylase,pectinase, cellulase or hemicellulase, preferably α-amylase, β-amylase, gluccomylase, isoamylase, pullulanase, endoglucanase, exoglucanase, beta-glucosidase, pectic lyase, pectic glycosidase, pectic methyl esterase, xylanase, mannanase, arabinanase, inulinase, levanase or fructanase, especially an α-amylase encoded by the DNA sequence of Fig. 2, particularly from nucleotide 184 to nucleotide 2131.

3. The inoculum of anyone of claims 1 and 2, in which the lactic acid bacteria is transformed with two or more of the exogenous DNAs, at least one of which is preferably under the control of a promoter having a DNA sequence as shown in Fig. 7a.

4. The inoculum of anyone of claim 1-3, in which the lactic acid bacteria is transformed with at least one, preferably at least two, of the exogenous DNAs that code for a cellulase and with at least one of the exogenous DNAs that codes for an enzyme that breaks down a polysaccharide and/or an oligosaccharide but is not a cellulase.

5. A probiotic characterized by the transformed lactic acid bacteria of anyone of claims 1-4; provided the bacteria are not <u>Streptococcus</u> unless the bacteria are transformed with at least two of the exogenous DNAs.

6. DNA which codes for an amylase, characterized by the DNA sequence shown in Fig. 2, particularly from nucleotide 184 to nucleotide 2131.

7. A plasmid DNA containing the DNA sequence shown in Fig.3a.

8. An ensilage method characterized by: the use of the inoculum of anyone of claims 1 to 4 on a crop.

9. A method for establishing and maintaining optimal intestinal flora in humans or animals characterized by feeding the humans or animals the probiotic of claim 5.

10. An amylase having the amino acid sequence shown in Fig. 2, particularly from nucleotide 184 to nucleotide 2131.

11. The transformed lactic acid bacteria of anyone of claims 1-4; provided the bacteria are not <u>Streptococcus</u> unless the bacteria are transformed with at least two of the exogenous DNAs.

12. A method for transforming lactic acid bacteria with an exogenous DNA characterized by electroporation of the exogenous DNA, preferably carried out in a low ionic strength buffer, with rapidly growing exponential phase cells concentrated to $10^{10}$ to $10^{11}$ CFU/ml.

13. DNA which contains the ORF 2, the nicking site, or both of the 1512 basepair <u>Aval-Sacl</u> fragment of the plasmid of claim 7.

14. DNA which contains the ORF 1, the $RS_A$ site, or both of the 1819 basepair <u>Aval-Sacl</u> fragment of the plasmid of claim 7.

15. A method for detecting a microorganism, particularly lactic acid bacteria, transformed with two or more exogenous DNAs, one of which codes for an enzyme, particularly an amylase, characterized by detecting the enzyme produced by the microorganism.

fig. 1

EP 0 311 469 A2

fig. 2

EP 0 311 469 A2

```
AAA TAT AGA AGT AGA ATG AAG CGT TTA CAA TGA ATA TTC GTC AAA ATT CGA

TAT TTT ACA CGG AAA TGT GAA GAA AAA CTA ACA TTT TTA AGA AAA AAA CGA

ATG TTT TTT ATA AAT TAA CAA GGA TAT TGC AGT TTT TCG CAG AAA TAG TTA

                                    VAL SER ILE LEU ASN VAL ILE
AAG TAT AAA AAA TTC TGT CAT CTA AGT GAG GTG AGC ATA TTG AAT GTA ATA

ASN CYS GLU GLU VAL LYS ARG ASP GLU PHE HIS THR GLU LYS TYR TYR GLU
AAT TGT GAA GAA GTG AAA CGA GAT GAG TTT CAT ACA GAA AAG TAC TAT GAA

SER TYR ASN ILE PHE GLY ALA HIS VAL VAL THR GLU ASP GLU ILE GLN GLY
AGT TAT AAC ATC TTT GGC GCG CAT GTT GTG ACG GAA GAT GAG ATT CAA GGG

VAL ARG PHE THR VAL TRP ALA PRO HIS ALA LYS ALA MET SER VAL VAL GLY
GTA CGA TTT ACA GTG TGG GCT CCT CAT GCG AAA GCA ATG AGT GTT GTT GGG

ASP PHE ASN GLU TRP ASP TYR GLU GLN HIS LYS MET LEU GLN VAL THR GLU
GAT TTT AAT GAG TGG GAT TAT GAG CAA CAT AAG ATG CTA CAA GTG ACA GAA

GLU GLY ILE TRP SER LEU PHE ILE PRO HIS ILE GLU GLU GLY GLU ILE TYR
GAA GGC ATT TGG TCC TTA TTT ATA CCG CAT ATT GAA GAA GGA GAA ATA TAT

LYS TYR ALA ILE GLU THR LEU ALA GLY ASP VAL ILE LEU LYS ALA ASP PRO
AAA TAT GCG ATT GAA ACG TTG GCT GGT GAC GTC ATT TTA AAG GCA GAT CCG

TYR ALA VAL TYR ALA GLU VAL ARG PRO ASN THR ALA SER VAL VAL PHE ASP
TAT GCT GTA TAT GCA GAA GTA AGA CCG AAT ACG GCA TCT GTA GTT TTT GAT

ILE LYS GLY TYR GLU TRP ASN ASP LYS ASN TRP ASN ARG LYS LYS LYS LYS
ATA AAA GGA TAT GAA TGG AAT GAT AAA AAC TGG AAT CGT AAG AAA AAG AAA

LYS SER ILE TYR LYS GLU ALA MET THR VAL TYR GLU LEU HIS PHE GLY SER
AAA TCG ATT TAT AAA GAA GCG ATG ACA GTT TAT GAA TTA CAT TTT GGT TCT

TRP LYS LYS LYS GLU ASP GLY THR LEU TYR SER TYR ARG GLU MET VAL GLU
TGG AAA AAG AAA GAA GAT GGA ACG CTG TAC TCT TAC AGG GAA ATG GTT GAA

GLU LEU ILE PRO TYR VAL VAL GLU HIS GLN PHE THR HIS ILE GLU ILE MET
GAG CTC ATC CCG TAT GTG GTG GAA CAT CAA TTT ACA CAT ATT GAA ATT ATG

PRO LEU VAL GLU HIS PRO TYR ASP ARG SER TRP GLY TYR GLN GLY THR GLY
CCG CTT GTT GAG CAT CCA TAT GAT CGT TCT TGG GGA TAC CAA GGA ACG GGA

TYR TYR ALA ALA THR SER ARG PHE GLY THR PRO HIS ASP LEU MET HIS PHE
TAT TAT GCA GCG ACG AGT AGA TTT GGT ACG CCG CAT GAT TTA ATG CAT TTT

VAL ASP GLU CYS HIS LYS TYR GLY ILE GLY VAL ILE LEU ASP TRP VAL PRO
GTC GAC GAA TGT CAT AAA TAT GGA ATC GGT GTC ATT TTA GAT TGG GTG CCG

GLY HIS PHE CYS LYS ASP ALA HIS GLY LEU TYR LEU PHE ASP GLY THR PRO
GGG CAT TTT TGT AAA GAT GCT CAC GGT TTA TAT TTG TTT GAT GGT ACA CCG

THR TYR GLU TYR LYS ASP LYS ASP VAL GLN GLU ASN PRO VAL TRP GLY THR
ACC TAT GAA TAT AAA GAT AAA GAT GTA CAA GAA AAT CCA GTA TGG GGA ACT

VAL ASN PHE ASP LEU GLY LYS ARG GLU VAL ARG ASN PHE LEU ILE SER ASN
GTC AAT TTT GAT TTA GGA AAG AGA GAG GTA CGT AAT TTC TTA ATT TCA AAT

ALA LEU PHE TRP MET ARG TYR PHE HIS ILE ASP GLY PHE ARG VAL ASP ALA
GCG TTA TTT TGG ATG AGA TAT TTC CAT ATT GAT GGT TTC AGA GTT GAT GCA

VAL ALA ASN MET LEU TYR TRP ASN LYS GLU GLY GLN GLU GLN SER ASN GLU
GTT GCG AAC ATG TTG TAC TGG AAT AAA GAA GGA CAA GAG CAA AGT AAT GAG

HIS ALA VAL SER PHE LEU ARG GLU LEU ASN GLU ALA VAL PHE ALA GLU ASP
CAT GCT GTT TCA TTT TTA AGA GAG TTA AAT GAA GCA GTG TTT GCA GAG GAT
```

GLU ASP PHE LEU MET THR ALA GLU ASP SER THR ALA TRP PRO LEU VAL THR
GAA GAT TTT CTT ATG ACG GCA GAA GAT TCA ACA GCT TGG CCA CTT GTA ACA

THR PRO THR TYR GLU GLY GLY LEU GLY PHE ASN TYR LYS TRP ASN MET GLY
ACT CCA ACG TAT GAA GGT GGG CTT GGA TTC AAT TAC AAA TGG AAC ATG GGC

TRP MET ASN ASP VAL LEU LYS TYR MET GLU CYS ALA PRO GLU TYR ARG LYS
TGG ATG AAT GAC GTG CTG AAA TAT ATG GAG TGT GCG CCT GAA TAT AGG AAA

HIS ILE HIS GLU LYS MET THR PHE SER LEU LEU TYR ALA TYR SER GLU ASN
CAC ATT CAT GAG AAA ATG ACG TTT TCT TTA CTA TAT GCG TAC TCT GAA AAC

PHE ILE LEU PRO LEU SER HIS ASP GLU VAL VAL HIS GLY LYS LYS SER LEU
TTC ATA TTA CCG CTT TCT CAT GAT GAA GTC GTT CAT GGG AAA AAG TCG TTA

LEU ASN LYS MET PRO GLY ASP TYR TRP ASP LYS PHE ALA GLN LEU ARG LEU
TTA AAT AAA ATG CCA GGT GAT TAC TGG GAT AAG TTT GCT CAA CTT CGT TTA

LEU TYR GLY TYR PHE PHE THR HIS PRO GLY LYS LYS LEU LEU PHE MET GLY
TTA TAT GGA TAT TTC TTT ACT CAC CCA GGA AAG AAG TTA CTT TTC ATG GGA

GLY GLU PHE GLY GLN PHE ASP GLU TRP LYS ASP LEU GLU ASP LEU ASP TRP
GGA GAA TTC GGA CAG TTT GAT GAG TGG AAA GAC CTT GAA GAT TTA GAT TGG

ASN LEU HIS ASP PHE GLU MET HIS ARG TYR MET HIS ASP TYR PHE ASN GLU
AAT TTA CAT GAT TTT GAA ATG CAT CGT TAT ATG CAT GAT TAC TTT AAC GAG

LEU ILE ALA LEU TYR LYS ARG SER LYS PRO LEU TRP GLN LEU ASP HIS SER
CTC ATA GCA TTG TAT AAG CGC TCA AAA CCA CTT TGG CAA CTT GAC CAT TCA

PRO GLU GLY PHE GLN TRP ILE ASP ALA ASN ASN ASN GLU GLN SER ILE PHE
CCT GAA GGT TTT CAG TGG ATT GAT GCT AAT AAT AAT GAG CAA AGT ATT TTC

SER PHE ILE ARG GLN GLY ASP LYS GLN GLU ASP ALA LEU VAL VAL VAL CYS
TCT TTT ATC CGC CAA GGG GAT AAA CAA GAA GAT GCG TTA GTT GTC GTA TGT

ASN PHE THR LYS ALA THR TYR GLU ASN TYR LYS VAL GLY VAL PRO ASP PHE
AAT TTT ACG AAA GCT ACA TAT GAA AAC TAT AAA GTA GGT GTA CCA GAT TTC

GLU TYR TYR ASN GLU ILE LEU ASN SER ASP ALA GLU GLN TYR GLY GLY SER
GAG TAT TAT AAC GAG ATT TTA AAC AGT GAT GCT GAG CAA TAT GGC GGT TCG

GLY GLN VAL ASN LYS LYS ARG LEU LYS THR ILE LEU GLU PRO TYR HIS ASN
GGG CAA GTG AAT AAG AAA CGT CTT AAG ACG ATT CTA GAA CCG TAC CAT AAT

GLN ALA ALA HIS VAL GLU ILE THR ILE PRO PRO PHE GLY VAL SER ILE LEU
CAA GCA GCG CAT GTA GAG ATT ACA ATT CCA CCA TTT GGC GTA TCC ATA TTA

ARG PRO VAL LYS THR ARG LYS GLY SER LYS LYS GLN ASP GLY SER LYS THR
CGA CCA GTG AAG ACG AGA AAG GGG AGC AAA AAA CAA GAT GGC TCA AAA ACA

LYS VAL ARG SER ASN VAL THR SER ARG GLY LYS ARG
AAA GTG CGT AGC AAT GTT ACT AGC AGG GGG AAA AGG TAG TCG TTT AAG TGC

ATT AAC AAA AAA TTT AGC CAA GCC AGC TGT TCC ATT TGG TGG TAA ATA TCG

CAT TAT TGA TTT TAC GCT AAG TAA CTG TGC GAA TTC TGG TAT TGA AAC GGT

TGG GAT TTT AAC GCA ATA TCA ACC ACT TGA ACT TCA TAA TTA TAT CGG AAT

CGG AAA TGC GTG GGA TTT AGA CCG AGT AAA CGG TGG GGT CAC AGT ATT ACC

TCC TTA TGC GGA GTC TTC AGG GGT TAA GTG GTA TAC AGG TAC GGC AAG TGC

CAT TTA TCA AAA TTT AAA CTA TTT AAG TCA GTA TGA ACC GGA GTA TGT TCT

TAT TTT ATC AGG CGA CCA TAT TTA TAA AAT

Fig. 2 continuation

**Figure 3a:**

```
   K   L   C   L   C   L   L   F   F   G   L   Y   F   F   Q   F   F   P   H   S   F   V       66
AAGCTTTGCTTGTGTCTCTTGTTTTTCGGTCTCTATTTTTTTCAATTCTTCCCGCATAGCTTTGTA
   L   K   A   Q   T   E   Q   K   E   T   E   I   K   K   L   E   E   R   M   A   K   Y

   F   G   Y   *   STOP ORF 4                                              132
TTCGGGTACTGATAAATTCTTACGCTCTTTATTTTTGTTACCACGTTGAACATCAAAGCCATTTTC
   E   P   V   S   L   N   K   R   E   K   N   K   N   G   R   Q   V   D   F   G   N   E

                                                                        198
TTTGAGGTACTGTGGTAATTCCTCTTGAATGCGCTGTAAGGCTTCACGATTGAATATACGCTTAGC
   K   L   Y   Q   P   L   E   E   Q   I   R   Q   L   A   E   R   N   F   I   R   K   A

                                                                        264
TGAGAGCTTTTTTATCATCATCAAAGGGCACAATGCCCATATGCATGTGAGGGGTCTTCTCGTCCAT
   S   L   K   K   D   D   D   F   P   V   I   G   M   H   M   H   P   T   K   E   D   M

                          a                               a           b  330
ATGAACAACTGCATAGCGAATATTTTCGTCACCATAGTTATCTGCAAAATATTGTTTGGCTGTTTC
   H   V   V   A   Y   R   I   N   E   D   G   Y   N   D   A   F   Y   Q   K   A   T   E

      a                                                               396
AAAATATTTACGGGTTTCGGCTTCGTCTAATTGCTCAAAAAAATCTTTGTCACTGGTTATAATCCA
   F   Y   K   R   T   E   A   E   D   L   Q   E   F   F   D   K   D   S   T   I   I   W

                  b                                                   462
CTCATTGACTAAAACAGCGTCTTTGCGAACTGCCCGCTTACTCGCTTTGTTTTCGTTGATATAGGC
   E   N   V   L   V   A   D   K   R   V   A   R   K   S   A   K   N   E   N   I   Y   A

                                                                        528
TTCAATATCCGTTTTAAAGTTATCAGTGCGCCCAGCCACTAAATCATAATTCAGGTGAGAGTGGGA
   E   I   D   T   K   F   N   D   T   R   G   A   V   L   D   Y   N   L   H   S   .H  S

                                                                        594
AACATCAATATCTTCGTTGCTGTGATTAGTCGTTTTCCGTTGGTCATGATTCCCAATGCCGACTAA
   V   D   I   D   E   N   S   H   N   T   T   K   R   Q   D   H   N   G   I   G   V   L

                                                      SD              660
ATTATCAGCTTTTAATTTCGTCATTCTAGCCACTGCAAAACTCATTGTATGCACCTCATTTCGGAG
   N   D   A   K   L   K   T   M   R   A   V   A   F   S   M   START ORF 1

                      c                         c                     726
TTACCTTCCAAGTAAAGTATAACCCACTATACTTTATGTTATAAAGTGTGAGCTCTTTCAGAGGGA
                      -10                        -35 .

                                                                        792
CGCCTTGCCAGCGGGACAGACCCCACCGCTTATTTTTCTGACGAAAAATTCCGCTCTCGTTATCGA

                                                                   d 858
AAATTTTGGCTGTTTTTTTTACGCCAAAATAATCTCAACTCACGTTTGCTTTTTCAAGCAAAAAAGA

                      d                                               924
ACGGATACTAGCTCCGTTCTTTTCCCCTTTACCAGATAAAATAATTTTGCTTATTATAATCCCATT
                  STOP ORF 2   *   W   I   F   Y   N   Q   K   N   Y   D   W   K
```

```
                                                                          990
TCGCAACGACCTCGCGAACCACCTCATCGATTTTTACTTTATCATCATCGACATTAATTAAGTGAG
 A  V  V  E  R  V  V  E  D  I  K  V  K  D  D  D  V  N  I  L  H  A


                                                                         1056
CATCAACATCTTCAAGTTGTAACTGCTTGCGAATTTCTTTAAATAACCCACCATAACTAATTTGAC
 D  V  D  E  L  Q  L  Q  K  R  I  E  K  F  L  G  G  Y  S  I  Q  R

                                                                         1122
GTGACCCCGCCAATCCCTGTTCCAAATCCTCAACCACTTGCAGGTCACGTTCTTGGTCATTCGTCA
 S  G  A  L  G  Q  E  L  D  E  V  V  Q  L  D  R  E  Q  D  N  T  L

     e              g                                                    1188
AAATATCTTTGGACTTAACTTGATATTTAGCGGTTTCTTGTGCACTCGCAATTAACGAGTTTTTAC
 If D  K  S  K  V  Q  Yf K  A  T  E  Q  A  S  A  I  L  S  N  K  G

                                                                         1254
CTTTAGCCTTGTTCGAGCGAACAGCCTCAACATTAACAATTGGCTGATAATCTAACTTCATAGCCC
 K  A  K  N  S  R  V  A  E  V  N  V  I  P  Q  Y  D  L  K  M  A  R

            e                                                            1320
GTTGCCAAAGTTTTGACCACTCTGCCTGATTAATATAATTTGCTGAATCTTTAAAATACGTTGGTT
 Q  W  L  K  S  W  E  A  Q  N  I  Y  N  A  S  D  K  F  Y  T  P  K

                              g                                          1386
TAACGAAAAGCAGCACGTGCATGTGCTGATGATAAGTCCCATTTTTGTTGATCGTAATTTCGGTTG
 V  F  L  L  V  H  M  H  Q  H  Y  T  G  N  K  N  I  T  I  E  T  S

                                       h                                 1452
AACGCACATAACCCAACAAGTTTTTGGCCGGTTTCTTGTATTGAAACAGCTTAGAAATTGCCCGTC
 R  V  Y  G  L  L  N  K  A  P  K  K  Y  Q  F  L  K  S  I  A  R  G

                    h                                                 1 1518
CCATTTTTCTGACTTCCTGTTTCAAGTTTTCGCCAGATGCGTTTTCCGCCGTCAGCGTTAAAAACA
 M  K  R  V  E  Q  K  L  N  E  G  S  A  N  E  A  T  L  T  L  F  L

            i        j                                               k 1584
AAAAGCGACCCGTTTTTCGTTGCTTATGAGCTTCGTCTAAAACTTGCATTAACTGATTAGATTGTC
 F  R  G  T  K  R  Q  K  H  A  E  D  L  V  Q  M  L  Q  N  S  Q  G

  l             n      l      k                                         1650
CCATTGAGCGACGCCAATTGCACAATGGACACAATCGACTCTTACAAAACCATGTCTGATAAAGCC
 M  Sm R  R  W  nN C  L  P  C  L  R  S  K  C  F  W  T  Q  Y  L  R

              j                                                        1716
GCAGTCGGCCCTCATCATCAGCAACAAATCTCAATACTTCACCGCATTCTTTAACACGATTAGCTT
 L  R  G  E  D  D  A  V  F  mR L  V  E  G  C  E  K  V  R  N  A  K

                                                                       1782
TTTTAAAATTAAGTATTCTCAAATACTCCGCGTACTGTAAGTTTTCCAATTTACGTTCACGCCACG
 K  F  N  L  I  R  L  Y  E  A  Y  Q  L  N  E  L  K  R  E  R  W  P

                                                                SD 1848
GCCGTTCTTTTCTGTTACGACTAACATCTTTTTAAAATCTTTTTCGACATAAAAATAGCTCCAATCC
 R  E  K  R  N  R  S  V  D  K  L  I  K  K  S  M  START ORF 2
```

Fig. 3a cont. 1

```
                                              o
                           ┌─────────────────────────→                    1914
AGCAATGAATTTCTCACGTGGCTTGGAGCTAATCACATTCGTAAAGTATAAAAATAGACTTTAGAT
                                              ─────
                                               -10

     ←══r══                                                 ←      o    1980
     ┌───────────→
AGTCAACGTTGACAAATAACTCACTTGTCAGTAGACTTATCTCACGTATATTATTGATTAGCTCAC
─────     p                                                         ─────
-35                                                                  q

                                                                      2046
GTGTCCTGCTCGCCAAAGTTAGGATTTCCACGTGAGTTTTTTTATGCAGTTTTTTAAATAATCTAA
───→                          ─────────
                                  q

                                                                      2112
GACAACTGAGTCAATAAAAGTTCAAAACCCTCAGTCAACTTTAATAAGCCAAATTATGCACTATAA
                                                   STOP ORF 3   *   L

                 ←───────                                             2178
ACGCCGGCCTGTCAACGCTTTACAGCAAAACCAAAAAAATGACGTTGTTTGTAGTAGTATCAAGAT
    R   R   G   T   L   A   K   C   C   F   W   F   F   S   T   T   Q   L   L   I   L   I

            s                     s                                   2244
       ┌────────→           ←─────────
AAGAAGAAACTGGTCAAAATCGACCAGTTTCCAAGCCCTCGGGCGCTGACGCCCCGAACCCAGTCC
    L   L   F   Q   D   F   D   V   L   K   W   A   R   P   R   Q   R   G   S   L   G

                           t                   t                     2310
                      ┌──────→           ←────────
AAGCTGTACCAGCTTGACCCTATAATTGAGCGGGGTTCCCCGCTCAAACTCACTCCCACTCGCCGT
    L   Q   V   L   K   V   R   Y   N   L   P   T   G   R   E   F   E   S   G   S   A   T

                 u                     u                             2376
            ┌──────→           ←────────
GTGGCAGGCGGAACAAGTGGTAAATCTACTTGTTCCGGCAAGCCGGTTAGTGTCTAGATTCGAGTA
    H   C   A   S   C   T   T   F   R   S   T   G   A   L   R   N   T   D   L   N   S   Y

       v                SD                              v            2442
  ┌──────→                                       ←──────────
ATGCTGTCAATTCCTTATCCTCCCACGCTCCGCTCGTCCGGTACCATTGACAGCGGCAGCATTAGC
  H   Q   START ORF 3  ────────                                       ─────
                                                                      -10

            w                              y                        2508
       ┌────────→                     ┌──────→
CTCTCAACATTTTTATTGAGTACGCTTACTTTTAGCAATAAAATATAATTGTACTAAGTATTTTAA
─────       ───────                   ─────────
-35            x                          x

            w                                       z               2574
       ←─────────                               ┌──────→
GGAGGAATATACTCATAAAAATTATTAAATCAGTTACCTTTGCTATTGTTGGTACAACACTTTTAG

                                          z                         2640
                                     ←─────────
CTACTAGTTTTCCCGCTATTCAAGCACACGCAAACGATCAAACAATAGAAAGTAATACAGTCGTTG

                                                                      2706
AAAGTTTCGGTTGGCAATAAGGATTGGTTATATATGAAAAATAGCCTATGGATTCGAAGTATACTG

            y                                                         2772
       ←──────────
ATCATCGCCTTATTATTTATTTTATGGGCAAGTAACCATTACAATTGGCCTTATCACAATATAACA

                                                                      2838
GGATATGTCTTAATAGTATGTGGAGTATTAGTAGGACTTTTAGGTAATCAAATAGACAAAAAATAA

                                                                      2904
CAGCTAAAAATAGCATATCTAGCAAGCCTTTTAACCGGTAAAGGAATCTAAAGGCTTGGACCATCTA
                         STOP ORF 1   *   L   S   P   G   D   L
```

Fig. 3a cont. 2

```
                          0
                    ——————————→                                    2970
ATTCCTCATGGTGTCCCTCTGAACGCTCCTGTGAGCTTTCAGGGGCTTTTTTAGACGGTTCTTTTA
  E   E   H   H   G   E   S   R   E   Q   S   S   E   P   A   K   K   S   P   E   K   L

                                    -35                            3036
GTCCTGCACGCTCTAGCCACTTTTCAGGTAAATTAATACCTAGTTTTTTGTGTAGAAACTCATCAA
    G   A   R   E   L   W   K   E   P   L   N   I   G   L   K   K   H   L   F   E   D   V

       -10                        0                                3102
CATTTCGTATAATGCCTTGAAGCGTTCCTACTAGTTTTTGCAATCGCTCATTTTCTTTTCTCAGTT
  N   R   I   I   G   Q   L   T   G   V   L   K   Q   L   R   E   N   E   K   R   L   E

                                                                   3168
     SD                          START ORF 4  M   I   F   Y   F   Q   M   N
CATAGTTGCGGCCCCTTGGCTTCACTCAAATCATTACGTAAACTATGATTTTCTACTTCCAGATGAA
  Y   N   R   G   K   A   E   S   L   D   N   R   L   S   H   N   E   V   E   L   H   I

                                                                   3234
   F   F   K   V   I   S   V   A   S   H   R   P   L   F   Q   I   S   E   I   R   F   I
TTTTTTCAAAGTTATAAGCGTCGCTAGTCACCGCCCTTTGTTCCAAATCAGCGAGATCAGATTTAT
    K   E   F   N   Y   A   D   S   T   V   A   R   Q   E   L   D   A   L   D   S   K   D

                                                                   3300
   N   R   D   F   I   F   I   H   Q   G   G   F   T   S   N   F   F   G   I   P   W   F
CAACCGTGACTTTATTTTTATTCATCAAGGTGGGTTTACTAGCAATTTTTTTGGTATCCCGTGGTT
    V   T   V   K   N   K   N   M   L   T   P   K   S   A   I   K   K   T   D   R   P   K

              3331
   D   F   I   K   L   F   F   S   I   C
TGATTTCATCAAGCTGTTTTTTAGTATCTGC
    I   E   D   L   Q   K   K   T   D   A
```

Fig. 3a Cont. 3

Fig. 3 b

EP 0 311 469 A2

pLH1
*3331bp*

Fig. 3 c

EP 0 311 469 A2

**A**

```
REC110    pUB110(1-422)
REC194    pE194(1-408)
REC181    pT181(1-413)
REC1      pLAB1000(1-416)

  1 MSyavcRnqkVKsaglkgmcQfiNQPRlrKsrtNdDIdHEzIreNYDlkNdkNIdYnervkEhIfesqktqtqIkklukKkDkDAvlvnelIVTSDrDFFeqdIdpgEqkRHleeSikIfsurIYGkRPlalaYkNIVIInDEqtPHMIlCvVl nld
  1 MShSIlRvarVKgssNtngIQrHNQPRENKNYrnkDInHEeHYkNYYDlliNAqNIkykdkIDEtIdeNYsGNRKIIRsDAIrHvDGIVTSDkDFFdIJsgEEIeEFkdS1EFLEnEYGkeNnlYATHvHIDErvPHMHGfvPltedP
  1 MSySIVRVarVKsgtNTtgIQkfvvQRENnNYeNEDIDIIskTYIYNDIMNAnkqNFnnlIDEkIeqqNYtCQRKEKlrtKRtKDAIrHIDGIITSDnDFFDrqtpEdTkqPFEyAKEFLEqEYGkdKilYATvMOEKTPHMHydvVPltdD
  1 MSySIVRVsKVKsgtNTtgIQkfvvQRENnNYeNEDIDIIskTYIYNDIMNAnkqNFnnlIDEkIeqqNYtCQRKEKlrtKRtKDAIrHIDGIITSDnDFFDeaeTrkyFEtAKqyfadnYGdeNirYAvVHfQiVPfdDD
  1 faVarmtklkadNlvGIqnEdQRkttNhsNFDIvShshINYDIVagrtdNFKtdHeaylIneKkaskRavRkDAvlvnewiITSDkDFFeqdeaeTrkyFEtAKqyfadnYGdeNirYAvVHfQiVPfdDD
```

```
MSysivrv-kvksa-nt-giq-EnQRenkny-NpIdhe-tylNYDIvna-ninfk--ide-IIeenytgkRk.IrFkDAIlhndgliITSDkDFFdqldpeetkrfIIe-akefle-eYGkeNilYAktPHMH-GvVP-tcD
```

```
144 GkLqgkK.nvfN          rqellwIQDkFpEhmkkqGIEELkrgERGsdrkhiEtakfKqtleKeidFleknIavkKDEwtaysdkvkSdlEvpakrhnkS  vevptgE      ksmEGIgkeimktekkptknvvisERDyknLv
145 GRLSAKEqlGNKK         dfTqLQDRFNEyVneKgYEL       ERGtSkeVTEreHkamdQYRkdTvFHKQEIqevkDEIqkankqlqSgiEhm.....rStKP  fdyE      nertGI  FSgreetgrkiltadeFER      LQ
145 GRLSAKEvvGNKKAL       TafqDRFNEhVkQRGYgl         ERGqSrqVTnaKheqisQYYkqkTEyHkQEyeresqkTdhikqNdkImqeyqkSlnTLkKPinvpYEqetekVgGI  FS   keiqeagnvvisqkdfNefQ
149 kkLSAK.rlfNreAlqriqeelpqylkeNgfdvQRG   nknkER   knlsVpeyKamreelkKieTE  KQEtqakladrTkqldeikprdtkkiaSkpTlmknknV   tvdKsdIadleqravtsdaynfekIhleveNhsl
```

```
grfLsaK.-vgNkkal---qe-t-lqdrfnehvkqrGyelk--ERg-sr-vte-kh----qvyketefhkqeI-q--kkdet-k--dk--ks---e---ks-ntl-kp--v-ye----k--gl-fs--r----nvviserd-n-lq
```

```
277 taardndRlkqhvrNlmSTDmarEykKLsKehgqvKEkysglvFrfnEnvndilNelLeEENkSLkskisdlkrdVSliyestkefIkertdglkaFknvFKGFvdkvkdkdaqfqekhdlepknefelthnrevkKErsRdqgmsl
270 etIssAeRIvdDYENIKSTDyytEnqeLkKrresILKEvvntwKEgyhEkskevNkLkrENdSL     neqlnVS   ekfqdstvTlyraaranFpqfeKGFnrlkekffndskfarvgQfmdvvQdnvQKvDRKrEkqRtddlem
276 kgIkaAqdIseDYEyIRKSqRaldokdkeirekdDlnkaverIEnaddnfnqlyenaKplkenieialklkillkElervlgrnTfaervnkltedepKlnqlaqnldKkmnpelyseQeqgqeQqKnQkrDRgmhl
279 rndlseakgrnyelrkeneRlqklvgtlqgliirnvdeflhkklginlpekwleraglKepskkapessqersseghhEeldgpsl?iplpvkrlaryailavifclfdylKvliilhillrhillyccKancngylplk
```

```
--i-sa-ri--dyenikstd----e--kl-ke---lkek------en-n--nellkenksl----s-e---vs-eee---s--t-----kgf--l---k----e---lq--~-eqdk-qkrdrk-ek-r-----
```

**B**

```
         RSAE     pE194(1-44)
         RSAT     pT181(1-38)
         RSAU     pUB110(1-38)
         RSAL     pLAB1000(1-38)
```

```
                       -35                                      -10
        1 CGACTTAaTTAcgaagt AAATAAGTCTAGTGTGTTAGACTTtAt    ...  34NT ... SD-ATG
                          ||||||||||||||||||||| || |||
        1 CGACTTATTAA      AAATAAGTCTAGTGTGTTAGACTTaAa    ...  31NT ... SD-ATG
                          ||||||||||||||||||| | || |||
        1 CACTTTATgAA      tATAAAGTATAGTGTGTTATATACTTTAC  ...  55NT ... SD-ATG
                          ||||||||||||||||||| || || |||
        1 aCACTTTATAA      CATAAAGTATAGTGGTTATATACTTTAC   ...  19NT ... SD-ATG

          cgacTTattaA------aAaaAAGTaTAGTGtGTTAgACTTtAc
                                        9                     9
```

**Fig. 3 d**

A

REP194    pC194(1-230)
REP1      pLAB1000(1-314)
REP110    pUB110(1-334)
REP14     pFTB14(1-339)
REPPHI    ΦX174(1-341)

```
                    mcynmEkytekkqrnqvfqkfikRhigenqmdlveoCnt flsFVADktlekqkiYkansQKrRfCEFvCaWR  karkdalgl slmmqylKQqekkeFlFLTlIT  tpNvmsdeLLenEikr
    mSkkilkdvSR     nrKERPWRErKleNlqYaEyLRILnFKKAnRVKeCgEvLrFVAD deGrlILqtWFCKSRLCFlCnWRRsMgqsnQlmqVldEahKQrkTgrRFlFLTlIT  aeNasGEnLkqEvrk
1 mgvsfnimcpnSSIysDekSRvlvdktksgKvRPWRErKKiaNvdYfEllhILeFKKAERVKdCaAE lLeyKqnrETGerKJYrvWFCKSRLCPFxCnWRRamKhglQsqKVvaEvlKQkpTvRWIFLTlITVkNVydGEeLnkslSd
1 myssendySIleDktatgkkrdwrgkKrRanlmaehyealekrigapyygKKAERlseCAERlsfKrdpETGrlKLYqahFCKSRLCPtCaWRR  slkiayhlKliiEeanrqygcgWiFLTlITVrNV  kGErLxpqISe
1 mksrrgfaiqrlmnamrqahadgwfivfdtltladdrleafydnpnalrdyfrdigrmvLaaegrkandshadcyyFCvpeygtangrlhfavhfmrtlptgsvdpnfgrrvnrrqnslqntwpygysmpIav
    ---------m---ssi---d--sr------ gkerpwrekklanv-ykellrilnfkkaervkdcaevLsfkadketgrklklyqawfCksrslcpmcnwrr-m-k-aq-lkv--evikqq--t-rflflt ─kv-nn--geelk-els-
```

```
117 ynnsfrKLikrKKvgsvikGvVRbkleIllYyNkkrddMinpHfHVlliaVnksYFtDkryYlsQgEWldIlW                                  rdVtgiseltgvqvkIrqnnkelyEnAKQY  sgkKD
126 MgraisKLfQYKKpaKNLlGvVRsTEIIlINK   ngtMhQrMHVllfVkPTyFKDsaNYINQaEWsklIWqrAMKlDY    qplvNveaVrsnkakgknS      llaSA qETAKQqvKc                  sKDilTnDqErdIqvveDLEqGLa
145 MaqGFRrmmQYKKdinKNLvGFmRATEvTIINkdnsMnQrMHVlvcMePTYFKnteNYvnNQkqWLqfWKkAMKlDY    dpNVKvqmlrpknkykSD       IQSAidETAKbPVKD               tDfmTdDeEKNLkrlsDLEeGLh
139 MmeGFRklfQYKKvktsvlGFfRAliffKhheedtMnpHfHVlllpVkrnYF   gKNYllKbaeWlsdWKrAMKlDYtplvdirrVKgrvkldaeqleSDvreammeQkAvlEIskQPVKbddvvvRgskVTvddNLntvfyLddaLs
138 rytgdafsrsgwlwpvdakGeplkatsymavgfyvakyvnkksdmdlaakglqaKewnnslxktkisllpkklfirirmsrnfgmkmltmtnlstecliqltkIgydatpfnqIlkmakremrlRlgKVTvadvlaaqpvttnLt
    m--gfrklfqykkv-knlkGfvrateltinkk-dtyhqhmhvll-vkptyfkd-knyingaew--lwkramkldy-----i-nvk-vm-i-a----sd----lqsa--elaKypvkd--kdrltkdte-nl--v-dle-gl-
```

```
220               sdyL  INksksl
257 gsRqISYGGLfKEI rKqlqledvdahI  iNvDDDkvKlDEvvrevvAkWdynkqNYFIw
276 rkRLISYGGLLKEIHKKLNLdDTeEGDI  ihtDDD EKaDEdgFsllAmWnwerKNYFIKe
281 arRLIgYGGILKEIHKeLNLgDaEgGDtvkIeeEDD E vanGaFevmAyWhpglKNYilK
282 kfmrasikmIgvsnlqsfiasmtqkltlsdEsknyldkaGittaclrikskwtaggk
    --rlisyggllkeihkklnl-dte-gdI─ln-ddd-ek-deg-fev-a-w---knyflk-
```

B

NICK194   pC194 (1-30)
NICK110   pUB110 (1-30)
NICK14    pFTB14 (1-30)
NICK1     pLAB1000 (1-30)
NICK174   ΦX174 (1-30)

```
                          nick
                           ▼
1 tcCTll  llTcCTTaTCTTGATAT CTTATCTTGATA  A   TAAgGg
1 CTtCgltCtTTCTTATCTTGATAC CTTATCTTGATAC A   TAtTAGa
1 GgCtClltTTCTTATCTTGATACTA CTTATCTTGATACTA  TAtTyGc
1 GtTllC  llTTCTTATCTTGATACTA CTTATCTTGATACTA  cTtACAaaac
1 lIT  ctTTCTTATCTTGATAa TAhttaACtct
   --cgtgtcctTTCTTATCTTGATAaTAahttaACtct A  ─-tAcaga-
```

Fig. 4

pERM3.2
(+pERM3.1 which differs in orientation of pLH1 insert)

Fig. 4 - continued (1)

EP 0 311 469 A2

Fig. 4 - continued (2)

pGI401

**EP 0 311 469 A2**

Fig. 5

Fig. 6

Fig. 7 a

5' Sau3A
GATCAGCTGCGACACAACTAGTTTACTTACTCGCTTATTAAACCAGACCCACAATCTTT

-35          -10          TRANS-

TACACAGATACAATATTTTTAGTGGAAACTTCTTGACATTTCGGCCCATGACCTTTACTCTGTTATAAATTACTTTTAT
-35          -10          -35          -10
CRIPTIE   Sau3A
GGGGGACGATCACACTAGCAAAGGAGTTACCTAAGCCCCGA    ATG TTC AAT GGG AAG ACT TCC CCA ATC
RBP                                          Met Phe Asn Gly Lys Thr Ser Pro Ile

ATG ACC CAC ATT ACG GGA CCC CAA GTT GCG GAG AAG AAG GCG ATG TAAACTGTCAAAGCAATCA
Met Thr His Ile Thr Gly Pro Gln Val Ala Glu Lys Lys Ala Met stop

Sau3A
CAGAGATGATC 3'

EP 0 311 469 A2

Fig. 7 b

Lactobacillus hilgardi 67

chromosomal DNA

Sau3AI

ligate
transform in B.subtilis
select for chloramphenicol
resistance

BamHI

e.g.

pPGV1LH

Fig. 7c

Fig. 8

Lactobacillus plantarum 80
chromosomal DNA

pGI4010
3.6 kbp

Em

ori+

BamHI

Amp

Sau3AI

Sau3AI fragments
+/- 3 kbp

BamHI

BamHI/Sau3AI

Lp80
+/- 3kbp

Em

ori+

Amp

BamHI/Sau3AI

e.g.

BamHI/Sau3AI

EcoRI

BalI

PvuII

PstI

3.2 kbp

pH421
6.8 kbp

Em

ori+

Amp

BamHI/Sau3AI

Fig. 9

Fig. 9 continued

Fig. 10    EP 0 311 469 A2

Fig. 10

Fig. 10 continued

Fig. 11

P ► PVU II
D ► DRA I
B ► SNA BI
H ► HIND III
T ► PST I

Fig. 12

B►BCL I
H►BAM HI
E►ECO RI
S►SPH I
T►PST I

Fig. 13

EP 0 311 469 A2

Legend:

P ► PVU II
H ► HIND III
T ► PST I
E ► ECO RI
S ► SMA I
Tn → Tn917 borders
ω ► ori pTV8

Fig. 14

H ━ HIND III
L ━ SAL I
A ━ AVA I
S ━ SMA I
P ━ PVU II
X ━ XBA I
T ━ PST I

Fig. 15